(19) Europäisches Patentamt
European Patent Office
Office européen des brevets

(11) **EP 3 773 750 B1**

(12) **EUROPEAN PATENT SPECIFICATION**

(45) Date of publication and mention of the grant of the patent:
**20.04.2022 Bulletin 2022/16**

(21) Application number: **19713467.9**

(22) Date of filing: **26.03.2019**

(51) International Patent Classification (IPC):
***A61K 49/00*** *(2006.01)*

(52) Cooperative Patent Classification (CPC):
**A61K 49/0032; A61K 49/0056; A61K 49/0069**

(86) International application number:
**PCT/EP2019/057538**

(87) International publication number:
**WO 2019/185607 (03.10.2019 Gazette 2019/40)**

(54) **FORMULATION AND METHOD OF PREPARATION**

FORMULIERUNG UND VERFAHREN ZUR HERSTELLUNG

FORMULATION ET PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2018 GB 201804835**

(43) Date of publication of application:
**17.02.2021 Bulletin 2021/07**

(73) Proprietor: **GE Healthcare AS**
**0485 Oslo (NO)**

(72) Inventor: **KISERUD, Marit Swärd Nordmo**
**0495 Oslo (NO)**

(74) Representative: **Lawrie IP Limited**
**310 St. Vincent Street**
**Glasgow G2 5RG (GB)**

(56) References cited:
**WO-A1-95/29178**
**WO-A1-2009/027095**
**WO-A2-2004/078778**
**WO-A2-2007/124090**
**WO-A2-2008/139207**
**WO-A2-2015/071299**

- **CLAUDIA DI TOMMASO ET AL: "Investigations on the lyophilisation of MPEG-hexPLA micelle based pharmaceutical formulations", EUROPEAN JOURNAL OF PHARMACEUTICAL SCIENCES., vol. 40, no. 1, 23 February 2010 (2010-02-23), pages 38-47, XP055590079, NL ISSN: 0928-0987, DOI: 10.1016/j.ejps.2010.02.006**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 773 750 B1

SEQUENCE LISTING

<110> GE Healthcare AS

<120> Formulation and Method of Preparation

<130> P150950.WO.01

<150> GB1804835.5
<151> 2018-03-26

<160> 8

<170> PatentIn version 3.5

<210> 1
<211> 17
<212> PRT
<213> Homo sapiens

<220>
<221> MISC_FEATURE
<222> (2)..(2)
<223> Xaa at position 2 may be Asn, His or Tyr

<220>
<221> MISC_FEATURE
<222> (4)..(4)
<223> Xaa at position 4 may be Gly, Ser, Thr or Asn

<220>
<221> MISC_FEATURE
<222> (8)..(8)
<223> Xaa at position 8 may be Thr or Arg

<220>
<221> MISC_FEATURE
<222> (15)..(15)
<223> Xaa at position 15 may be Ala, Asp, Glu, Gly or Ser

<220>
<221> MISC_FEATURE
<222> (16)..(16)
<223> Xaa at position 16 may be Ser or Thr

<220>
<221> MISC_FEATURE
<222> (17)..(17)
<223> Xaa at position 17 may be Asp or Glu

<400> 1

```
Cys Xaa Cys Xaa Gly Pro Pro Xaa Phe Glu Cys Trp Cys Tyr Xaa Xaa
1               5                   10                  15


Xaa
```

<210> 2
<211> 18
<212> PRT
<213> Homo sapiens

<220>
<221> MISC_FEATURE
<222> (3)..(3)
<223> Xaa at position 3 may be Asn, His or Tyr

<220>
<221> MISC_FEATURE
<222> (5)..(5)
<223> Xaa at position 5 may be Gly, Ser, Thr or Asn

<220>
<221> MISC_FEATURE
<222> (9)..(9)
<223> Xaa at position 9 may be Thr or Arg

<220>
<221> MISC_FEATURE
<222> (16)..(16)
<223> Xaa at position 16 may be Ala, Asp, Glu, Gly or Ser

<220>
<221> MISC_FEATURE
<222> (17)..(17)
<223> Xaa at position 17 may be Ser or Thr

<220>
<221> MISC_FEATURE
<222> (18)..(18)
<223> Xaa at position 18 may be Asp or Glu

<400> 2

```
Ser Cys Xaa Cys Xaa Gly Pro Pro Xaa Phe Glu Cys Trp Cys Tyr Xaa
1               5                   10                  15

Xaa Xaa
```

<210> 3
<211> 22
<212> PRT
<213> Homo sapiens

<220>
<221> MISC_FEATURE
<222> (5)..(5)
<223> Xaa at position 5 may be Asn, His or Tyr

<220>
<221> MISC_FEATURE
<222> (7)..(7)
<223> Xaa at position 7 may be Gly, Ser, Thr or Asn

<220>
<221> MISC_FEATURE
<222> (11)..(11)
<223> Xaa at position 11 may be Thr or Arg

<220>
<221> MISC_FEATURE
<222> (18)..(18)
<223> Xaa at position 18 may be Ala, Asp, Glu, Gly or Ser

<220>
<221> MISC_FEATURE
<222> (19)..(19)
<223> Xaa at position 19 may be Ser or Thr

<220>
<221> MISC_FEATURE
<222> (20)..(20)
<223> Xaa at position 20 may be Asp or Glu

<400> 3

```
Ala Gly Ser Cys Xaa Cys Xaa Gly Pro Pro Xaa Phe Glu Cys Trp Cys
1               5                   10                  15

Tyr Xaa Xaa Xaa Gly Thr
            20
```

<210> 4
<211> 4
<212> PRT
<213> Homo sapiens

<400> 4

```
Gly Gly Gly Lys
1
```

<210> 5
<211> 4
<212> PRT
<213> Homo sapiens

<400> 5

```
Gly Ser Gly Lys
1
```

<210> 6
<211> 5
<212> PRT
<213> Homo sapiens

<400> 6

```
Gly Ser Gly Ser Lys
1               5
```

<210> 7
<211> 26
<212> PRT
<213> Homo sapiens

<400> 7

```
Ala Gly Ser Cys Tyr Cys Ser Gly Pro Pro Arg Phe Glu Cys Trp Cys
1               5                   10                  15

Tyr Glu Thr Glu Gly Thr Gly Gly Gly Lys
            20                  25
```

<210> 8
<211> 26
<212> PRT
<213> Artificial Sequence

<220>
<223> 26-mer scrambled cMET binding peptide (negative control)

<400> 8

```
Thr Gly Glu Cys Thr Cys Pro Tyr Trp Glu Phe Arg Pro Cys Glu Cys
1               5                   10                  15

Gly Ser Tyr Ser Gly Ala Gly Gly Gly Lys
            20                  25
```

## Description

### Field of Invention

The present invention relates to a formulation for human or animal administration and to a method of preparation thereof. In particular, the present invention relates to more stable pharmaceutical formulations, such as those for intravenous administration, and to a method of preparation thereof.

### Background

WO2008/139207 describes labelled cMet binding peptides suitable for optical imaging *in vivo.* The peptides are labelled with an optical reporter group suitable for imaging in the red to near-infrared region. Also described are intravenously administered optical molecular imaging cMet binding peptides, such as those that can be used for the early diagnosis of colorectal cancer (CRC).

In more detail, WO2008/139207 describes a water soluble 26-amino acid cyclic peptide with a high affinity towards human cMet, which comprises a fluorescent optical reporter imaging moiety suitable for imaging the mammalian body *in vivo.* It exhibits strong fluorescence signals with peak excitation at 653 nm and peak emission at 675 nm, which is considered the optimal wavelength region for imaging of superficial lesions.

However, the present inventors have observed that the imaging agents described have a poor shelf life in solution, and therefore storage and degradation of the imaging agents prior to intravenous injection can be a problem. Furthermore, for intravenous use, imaging agents are beneficially at an appropriate pH and tonicity for intravenous administration.

Therefore, an object of the invention is to provide a more stable formulation for imaging agents, in particular optical imaging agents, more particularly optical imaging agents comprising peptides such as those described in WO2008/139207. A further object of the invention is to provide a formulation for imaging agents, in particular optical imaging agents, more particularly optical imaging agents comprising peptides such as those described in WO2008/139207, and that illustrate better reconstitution, in particular at an appropriate pH and/or tonicity for intravenous use.

WO 2004/078778 discloses a lyophilised c-Met binding polypeptide.

### Disclosure of Invention

According to a first aspect of the invention, there is provided a lyophilised formulation comprising:

(i) an active pharmaceutical ingredient (API);
(ii) a buffering agent; and
(iii) a lyoprotectant;

wherein the API is an imaging agent comprising at least one cMet binding peptide, suitable for optically imaging the mammalian body, the imaging agent comprising a compound of Formula I:

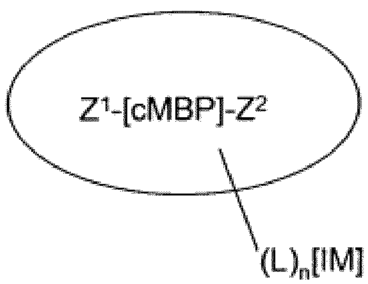

(Formula I)

wherein:

$Z^1$ is attached to the N-terminus of cMBP, and is H or $M^{IG}$;
$Z^2$ is attached to the C-terminus of cMBP, and is OH, $NH_2$, $OB^c$ or $M^{IG}$;
wherein $B^c$ is a biocompatible cation;

cMBP is a cMet binding cyclic peptide of 17 to 30 amino acids, which comprises the amino acid sequence (SEQ-1):

$$Cys^{a}\text{-}Xaa^{1}\text{-}Cys^{c}\text{-}Xaa^{2}\text{-}Gly\text{-}Pro\text{-}Pro\text{-}Xaa^{3}\text{-}Phe\text{-}Glu\text{-}Cys^{d}\text{-}Trp\text{-}Cys^{b}\text{-}Tyr\text{-}Xaa^{4}\text{-}Xaa^{5}\text{-}Xaa^{6};$$

wherein: $Xaa^1$ is Asn, His or Tyr;
$Xaa^2$ is Gly, Ser, Thr or Asn;
$Xaa^3$ is Thr or Arg;
$Xaa^4$ is Ala, Asp, Glu, Gly or Ser;
$Xaa^5$ is Ser or Thr;
$Xaa^6$ is Asp or Glu;
and $Cys^{a-d}$ are each cysteine residues such that residues a and b as well as c and d are cyclised to form two separate disulphide bonds;

$M^{IG}$ is a metabolism inhibiting group, which is a biocompatible group that inhibits or suppresses *in vivo* metabolism of the peptide;
L is a synthetic linker group of formula -(A)m- wherein each A is independently $-CR_2-$, -CR=CR-, -C=C-, $-CR_2CO_2-$, $-CO_2CR_2-$,-NRCO-, -CONR-, -NR(C=O)NR-, -NR(C=S)NR-, $-SO_2NR-$, $-NRSO_2-$, $CR_2OCR_2-$, $-CR_2SCR_2-$, $CR_2NRCR_2-$, a $C_{4-8}$ cycloheteroalkylene group, a $C_{4-8}$ cycloalkylene group, a $C_{5-12}$ arylene group, a $C_{3-12}$ heteroarylene group, an amino acid, a sugar or a monodisperse polyethyleneglycol (PEG) building block;
each R is independently chosen from H, $C_{1-4}$ alkyl, $C_{2-4}$ alkenyl, $C_{2-4}$ alkynyl, $C_{1-4}$ alkoxyalkyl or $C_{1-4}$ hydroxyalkyl;
m is an integer of value 1 to 20;
n is an integer of value 0 or 1;
IM is an optical reporter imaging moiety suitable for imaging the mammalian body using light of violet to near-infrared wavelength (400 to 1,200 nm);

wherein the formulation comprises from approximately 4 to approximately 12 % by weight API, from approximately 3 to approximately 35 % by weight buffering agent, and from approximately 56 to approximately 91 % by weight lyoprotectant; and wherein the formulation, when reconstituted, has a pH of between approximately pH 6.8 and approximately pH 9.

The optical imaging of the mammalian body may be *in vivo.*

By the term "buffering agent" it is meant a pH-adjusting agent. The buffering agent may be a compound or composition (e.g., a salt) that forms a buffer on dissolution. The buffering agent may be a dehydrated buffer salt. The buffering agent may be at least one of: an acid (typically a weak acid) and one of its salts, and a base (typically a weak base) and one of its salts.

Any reference to a "lyoprotectant" includes a "cryoprotectant" and *vice* versa, given that the terms are often used interchangeably in the field of the invention.

By the term "imaging agent" is meant a compound suitable for imaging the mammalian body *in vivo* or ex *vivo. Ex vivo* imaging still requires administration to the mammalian body, but may also involve removal of a tissue sample or the like and imaging that sample external to the body. In such an example, the formulation is used *in vivo* insofar as it is administered to the mammalian body, and is used *ex vivo* insofar as the imaging takes place external to the mammalian body. The mammal may be a human subject. The imaging may be invasive (e.g., intra-operative or endoscopic) or non-invasive. The imaging method may be endoscopy. Whilst the compound of Formula I is suitable for *in vivo* imaging, it may also have *in vitro* applications (e.g., assays quantifying cMet in biological samples or visualisation of cMet in tissue samples). The imaging agent may be used for *in vivo* imaging.

The $Z^1$ group substitutes the amine group of the last amino acid residue. Thus, when $Z^1$ is H, the amino terminus of the cMBP terminates in a free $NH_2$ group of the last amino acid residue. The $Z^2$ group substitutes the carbonyl group of the last amino acid residue. Thus, when $Z^2$ is OH, the carboxy terminus of the cMBP terminates in the free $CO_2H$ group of the last amino acid residue, and when $Z^2$ is $OB^c$ that terminal carboxy group is ionised as a $CO_2B^c$ group.

By the term "metabolism inhibiting group" ($M^{IG}$) is meant a biocompatible group which inhibits or suppresses *in vivo* metabolism of the cMBP peptide at either the amino terminus ($Z^1$) or carboxy terminus ($Z^2$). Such groups are well known to those skilled in the art and are suitably chosen from, for the peptide amine terminus: N-acylated groups $-NH(C=O)R^G$ where the acyl group $-(C=O)R^G$ has $R^G$ chosen from: $C_{1-6}$ alkyl, $C_{3-10}$ aryl groups or comprises a polyethyleneglycol (PEG) building block. Suitable PEG groups are described for the linker group (L), below. Such PEG groups may be the biomodifiers of Formula IA or IB. Such amino terminus $M^{IG}$ groups may be acetyl, benzyloxycarbonyl or trifluoroacetyl, typically acetyl.

Suitable metabolism inhibiting groups for the peptide carboxyl terminus include: carboxamide, tert-butyl ester,

benzyl ester, cyclohexyl ester, amino alcohol or a polyethyleneglycol (PEG) building block. A suitable M group for the carboxy terminal amino acid residue of the cMBP peptide is where the terminal amine of the amino acid residue is N-alkylated with a $C_{1-4}$ alkyl group, optionally a methyl group. Such $M^{IG}$ groups may be carboxamide or PEG, and may be carboxamide.

Formula I denotes that the $-(L)_n[IM]$ moiety can be attached at any suitable position of $Z^1$, $Z^2$ or cMBP. For $Z^1$ or $Z^2$, the $-(L)_n[IM]$ moiety may either be attached to the $M^{IG}$ group when either of $Z^1/Z^2$ is a $M^{IG}$. When $Z^1$ is H or $Z^2$ is OH, attachment of the $-(L)_n[IM]$ moiety at the $Z^1$ or $Z^2$ position gives compounds of formulae $[IM]-(L)_n-[cMBP]-Z^2$ or $Z^1-[cMBP]-(L)_n-[IM]$ respectively. Inhibition of metabolism of the cMBP at either peptide terminus may also be achieved by attachment of the $-(L)_n[IM]$ moiety in this way, but $-(L)_n[IM]$ is outside the definition of $M^{IG}$ herein.

The $-(L)_n-$ moiety of Formula I may be attached at any suitable position of the IM. The $-(L)_n-$ moiety either takes the place of an existing substituent of the IM, or is covalently attached to the existing substituent of the IM. The $-(L)_n-$ moiety is optionally attached *via* a carboxyalkyl substituent of the IM.

By the term "cMet binding cyclic peptide" (cMBP) is meant a peptide which binds to the hepatocyte growth factor (HGF) high affinity receptor, also known as cMet (c-Met or hepatocyte growth factor receptor). Suitable cMBP peptides have an apparent Kd for cMet of cMet/HGF complex of less than about 2 $\mu$M. The cMBP peptides comprise proline residues, and it is known that such residues can exhibit cis/trans isomerisation of the backbone amide bond. The cMBP peptides described herein include any such isomers.

By the term "biocompatible cation" ($B^c$) is meant a positively charged counterion which forms a salt with an ionised, negatively charged group, where said positively charged counterion is also non-toxic and hence suitable for administration to the mammalian body, especially the human body. Examples of suitable biocompatible cations include: the alkaline metals sodium or potassium; the alkaline earth metals calcium and magnesium; and the ammonium ion. Typical biocompatible cations are sodium and potassium, typically sodium.

By the term "amino acid" is meant an L- or D-amino acid, amino acid analogue (e.g., naphthylalanine) or amino acid mimetic which may be naturally occurring or of purely synthetic origin, and may be optically pure, i.e. a single enantiomer and hence chiral, or a mixture of enantiomers. Conventional 3-letter or single letter abbreviations for amino acids are used herein. The amino acids used may be optically pure. By the term "amino acid mimetic" is meant synthetic analogues of naturally occurring amino acids which are isosteres, i.e. have been designed to mimic the steric and electronic structure of the natural compound. Such isosteres are well known to those skilled in the art and include but are not limited to depsipeptides, retro-inverso peptides, thioamides, cycloalkanes or 1,5-disubstituted tetrazoles [see M. Goodman, Biopolymers, 24, 137, (1985)].

By the term "peptide" is meant a compound comprising two or more amino acids, as defined above, linked by a peptide bond (i.e. an amide bond linking the amine of one amino acid to the carboxyl of another). The term "peptide mimetic" or "mimetic" refers to biologically active compounds that mimic the biological activity of a peptide or a protein but are no longer peptidic in chemical nature, that is, they no longer contain any peptide bonds (that is, amide bonds between amino acids). Here, the term peptide mimetic is used in a broader sense to include molecules that are no longer completely peptidic in nature, such as pseudo-peptides, semi-peptides and peptoids.

By the term "optical reporter imaging moiety" (IM) is meant a fluorescent dye or chromophore which is capable of detection either directly or indirectly in an optical imaging procedure using light of green to near-infrared wavelength (400-1,200 nm, optionally 600-1,000 nm). The IM may have fluorescent properties.

One of the roles of the linker group $-(A)_m$,- of Formula I may be to distance the IM from the active site of the cMBP peptide. This is particularly important when the imaging moiety is relatively bulky, so that interaction with the enzyme is not impaired. This can be achieved by a combination of flexibility (e.g. simple alkyl chains), so that the bulky group has the freedom to position itself away from the active site and/or rigidity such as a cycloalkyl or aryl spacer which orientate the IM away from the active site. The nature of the linker group can also be used to modify the biodistribution of the imaging agent. Thus, e.g., the introduction of ether groups in the linker will help to modify plasma protein binding. When - $(A)_m$- comprises a polyethyleneglycol (PEG) building block or a peptide chain of 1 to 10 amino acid residues, the linker group may function to modify the pharmacokinetics and blood clearance rates of the imaging agent *in vivo.* Such "biomodifier" linker groups may accelerate the clearance of the imaging agent from background tissue, such as muscle or liver, and/or from the blood, thus giving a better diagnostic image due to less background interference. A biomodifier linker group may also be used to favour a particular route of excretion, e.g., *via* the kidneys as opposed to *via* the liver.

By the term "sugar" is meant a mono-, di- or tri- saccharide. Suitable sugars include: glucose, galactose, maltose, mannose, and lactose. Optionally, the sugar may be functionalised to permit facile coupling to amino acids. Thus, e.g., a glucosamine derivative of an amino acid can be conjugated to other amino acids *via* peptide bonds. The glucosamine derivative of asparagine (commercially available from NovaBiochem) is one example of this:

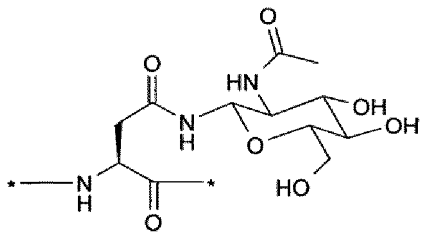

The molecular weight of the imaging agent is suitably up to 8,000 Daltons. Optionally, the molecular weight is in the range 2,800 to 6,000 Daltons, typically 3,000 to 4,500 Daltons, with 3,200 to 4,000 Daltons being most typical.

Imaging agents of the present invention may have both peptide termini protected by $M^{IG}$ groups, i.e. optionally both $Z^1$ and $Z^2$ are $M^{IG}$, which will usually be different. As noted above, either of $Z^1/Z^2$ may optionally equate to $-(L)_n[IM]$. Having both peptide termini protected in this way is important for *in vivo* imaging applications, since otherwise rapid metabolism would be expected with consequent loss of selective binding affinity for cMet. When both $Z^1$ and $Z^2$ are $M^{IG}$, optionally $Z^1$ is acetyl and $Z^2$ is a primary amide. $Z^1$ may be acetyl and $Z^2$ may be a primary amide and the $-(L)_n[IM]$ moiety may be attached to the epsilon amine side chain of a lysine residue of cMBP.

cMBP peptides of the present invention may have a $K_D$ for binding of cMet to cMet/HGF complex of less than about 10 nM (based on fluorescence polarisation assay measurements), most typically in the range 1 to 5 nM, with less than 3nM being the ideal.

The peptide sequence (SEQ-1):

$$Cys^a\text{-}Xaa^1\text{-}Cys^c\text{-}Xaa^2\text{-}Gly\text{-}Pro\text{-}Pro\text{-}Xaa^3\text{-}Phe\text{-}Glu\text{-}Cys^d\text{-}Trp\text{-}Cys^b\text{-}Tyr\text{-}Xaa^4\text{-}Xaa^5\text{-}Xaa^6$$

(SEQ-1)

of the cMBP of Formula I is a 17-mer peptide sequence, which is primarily responsible for the selective binding to cMet. When the cMBP peptide of the present invention comprises more than 17 amino acid residues, the remaining amino acids can be any amino acid apart from cysteine. Additional, unprotected cysteine residues could cause unwanted scrambling of the defined $Cys^a$-$Cys^b$ and $Cys^c$-$Cys^d$ disulfide bridges. The additional peptides preferably comprise at least one amino acid residue with a side chain suitable for facile conjugation of the $-(L)_n[IM]$ moiety. Suitable such residues include Asp or Glu residues for conjugation with amine-functionalised $-(L)_n[IM]$ groups, or a Lys residue for conjugation with a carboxy- or active ester- functionalised $-(L)_n[IM]$ group. The amino acid residues for conjugation of $-(L)_n[IM]$ are suitably located away from the 17-mer binding region of the cMBP peptide (SEQ-1), and are optionally located at the C- or N- terminus. Optionally, the amino acid residue for conjugation is a Lys residue.

Substitution of the tryptophan residue of SEQ-1 was evaluated with the known amino acid substitutes phenylalanine and napthylalanine. Loss of cMet affinity was, however, found suggesting that the tryptophan residue is important for activity. Optionally the cMBP peptide further comprises a N-terminal serine residue, giving the 18-mer (SEQ-2):

$$Ser\text{-}Cys^a\text{-}Xaa^1\text{-}Cys^c\text{-}Xaa^2\text{-}Gly\text{-}Pro\text{-}Pro\text{-}Xaa^3\text{-}Phe\text{-}Glu\text{-}Cys^d\text{-}Trp\text{-}Cys^b\text{-}Tyr\text{-}Xaa^4\text{-}Xaa^5\text{-}Xaa^6.$$

(SEQ-2)

In addition to SEQ-1, or SEQ-2, the cMBP may further comprise either:

(i) an Asp or Glu residue, or an analogue thereof, within 4 amino acid residues of either the C- or N- peptide terminus of the cMBP peptide, and-$(L)_n$[IM] is functionalised with an amine group which is conjugated to the carboxyl side chain of said Asp or Glu residue, or analogue thereof, to give an amide bond;

(ii) a Lys residue, or an analogue thereof, within 4 amino acid residues of either the C- or N- peptide terminus of the cMBP peptide, and $-(L)_n[IM]$ is functionalised with a carboxyl group which is conjugated to the epsilon amine side chain of said Lys residue, or analogue thereof, to give an amide bond.

Analogues of Asp and/or Glu may include one or more or 2-aminobutanedioic acid, 2-aminohexanedioic acid, 2-aminoheptanedioic acid, 2-aminooctanedioic acid, 2-aminononanedioic acid, 2-aminodecanedioic acid, 2-aminoundecanedioic acid, and 2-aminododecanedioic acid.

Analogues of Lys may include one or more of 2,3-diaminopropanoic acid, 2,4-diaminobutanoic acid, 2,5-diaminopentanoic acid, 2,7-diaminoheptanoic acid, 2,8-diaminooctanoic acid, 2,9-diaminononanoic acid, 2,10-diaminodecanoic acid, 2,11-diaminoundecanoic acid, 2,12-diaminododecanoic acid.

cMBP peptides may comprise the 22-mer amino acid sequence (SEQ-3):

Ala-Gly-Ser-$Cys^a$-$Xaa^1$-$Cys^c$-$Xaa^2$-Gly-Pro-Pro-$Xaa^3$-Phe-Glu-$Cys^d$-Trp-$Cys^b$-Tyr-$Xaa^4$-$Xaa^5$-$Xaa^6$-Gly-Thr.

(SEQ-3)

The cMBP peptides may have $Xaa^3$ equal to Arg.

The cMBP peptide may further comprise in addition to SEQ-1, SEQ-2 or SEQ-3, at either the N- or C- terminus a linker peptide which is chosen from: -Gly-Gly-Gly-Lys- (SEQ-4), -Gly-Ser-Gly-Lys- (SEQ-5) or -Gly-Ser-Gly-Ser-Lys- (SEQ-6).

The Lys residue of the linker peptide is a typical location for conjugation of the $-(L)_n[IM]$ moiety. Some cMBP peptides comprise SEQ-3 together with the linker peptide of SEQ-4, having the 26-mer amino acid sequence (SEQ-7):

Ala-Gly-Ser-$Cys^a$-Tyr-$Cys^c$-Ser-Gly-Pro-Pro-Arg-Phe-Glu-$Cys^d$-Trp-$Cys^b$-Tyr-Glu-Thr-Glu-Gly-Thr-Gly-Gly-Gly-Lys.

(SEQ-7)

cMBP peptides of SEQ-1, SEQ-2, SEQ-3 and SEQ-7 may have $Z^1 = Z^2 = M^{IG}$, and may have $Z^1$ = acetyl and $Z^2$ = primary amide.

The $-(L)_n[IM]$ moiety is suitably attached to either of the $Z^1$ or $Z^2$ groups or an amino acid residue of the cMBP peptide which is different to the cMet binding sequence of SEQ-1. Possible amino acid residues and sites of conjugation are as described above. When the $-(L)_n[IM]$ moiety is attached to $Z^1$ or $Z^2$, it may take the place of $Z^1$ or $Z^2$ by conjugation to the N- or C- terminus, and block *in vivo* metabolism in that way.

Typical IM groups have an extensive delocalized electron system, e.g. cyanines, merocyanines, indocyanines, phthalocyanines, naphthalocyanines, triphenylmethines, porphyrins, pyrilium dyes, thiapyrilium dyes, squarylium dyes, croconium dyes, azulenium dyes, indoanilines, benzophenoxazinium dyes, benzothiaphenothiazinium dyes, anthraquinones, napthoquinones, indathrenes, phthaloylacridones, *tris*phenoquinones, azo dyes, intramolecular and intermolecular charge-transfer dyes and dye complexes, tropones, tetrazines, *bis*(dithiolene) complexes, *bis*(benzene-dithiolate) complexes, iodoaniline dyes, *bis*(S,O-dithiolene) complexes. Fluorescent proteins, such as green fluorescent protein (GFP) and modifications of GFP that have different absorption/emission properties are also useful. Complexes of certain rare earth metals (e.g., europium, samarium, terbium or dysprosium) are used in certain contexts, as are fluorescent nanocrystals (quantum dots).

Examples of chromophores which may be used include: Atto 647, CF640R, Atto 647N, SiR650, CF633, Sulfo Cy5, T700-H, T700-F, CF647 SE, Quasar 670, Chromeo 642, Oyster 645, Oyster 647, Oyster 650, Iris 5, CyAL 5, IRDye 650, NIR4, AOI987, BODIPY® 650/665, DyLight 633, DyLight 650, DDAO (7-hydroxy-9H-(1,3-dichloro-9,9-dimethylacridin-2-one), Chromis 645 C, Chromis 645 Z, Chromis 645 A, HiLyte 647, PromoFluor 647P, Dy-630, Dy-631, Dy-632, Dy-633, Dy-636, Dy-650, Dy-651, Dy-652, Dy-654, Tracy 645, Tracy 652, Fluorescent red NIR 782, Atto 655, Atto 680, CF660 C, CF660 R, Quasar 705, CF680, IRDye680RD, NIR2, IRDye680LT, HiLyte 680, IRDye700DX, Oyster 680, Iris 5.5, HROMIS LSS 670Z, CHROMIS LSS 690Z, DyLight 680 (Pierce), Magnesium Phthalocyanine, Oxazin 750, SeTa-665, SeTa-667, SeTa-670, PromoFluor 670 (Promo Kine), Dy-682, PromoFluor 680, PromoFluor 700 P, Methylene Blue, Fluorescent Red NIR 730 Reactive, Fluorescent Red NIR 781 Reactive, BM104, BM105 (NHS ester of BM104), SiR720, Atto 740, CF750, Sulfo Cy7, IRDye750, NIR3, DyLight 755, DY-732, DY-734, DY-752, PromoFluor 750 P, HiLyte 750, DY-776, NIR1, IRDye800RS, PromoFluor 770 P, DY-778, CHROMIS 770 C, ZW800-1, ZW800-3, IRIS 7G-WS, IR 780, ESNF31, CF770, Vivotag 800, IRDye800CW, Alexa Fluor 790, CF790, Oyster 800, CHROMIS 770 A, CHROMIS 800 C, CHROMIS 800 A, CHROMIS 830 C, CHROMIS 830 A, DyLight 800, DY-777, DY-782, DY-800, PromoFluor 780 P, NIR-797, PromoFluor 840 P, fluorescein, sulforhodamine 101 (Texas Red), rhodamine B, rhodamine 6G, rhodamine 19, indocyanine green, Cy2, Cy3B, Cy3.5, Cy5, Cy5.5, Cy7, Cy7.5, Marina Blue, Pacific Blue, Oregon Green 488, Oregon Green 514, TAMRA (tetramethylrhodamine), TMR, Alexa Fluor 350, Alexa Fluor 430, Alexa Fluor

532, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, and Alexa Fluor 750. The cyanine dyes and derivatives and analogues thereof may typically be used.

Particular examples of chromophores which may be used include: Cy5, Cy5.5, Cy7, Cy7.5, Alexa Fluor 633, Alexa Fluor 647, Alexa Fluor 660, Alexa Fluor 680, Alexa Fluor 700, and Alexa Fluor 750, Atto 647, CF640R, Atto 647N, SiR650, CF633, Sulfo Cy5, T700-H, T700-F, CF647 SE, Quasar 670, Chromeo 642, Oyster 645, Oyster 647, Oyster 650, Iris 5, CyAL 5, IRDye 650, NIR4, AOI987, BODIPY® 650/665, DyLight 633, DyLight 650, DDAO (7-hydroxy-9H-(1,3-dichloro-9,9-dimethylacridin-2-one), Chromis 645 C, Chromis 645 Z, Chromis 645 A, HiLyte 647, PromoFluor 647P, Dy-630, Dy-631, Dy-632, Dy-633, Dy-636, Dy-650, Dy-651, Dy-652, Dy-654, Tracy 645, Tracy 652, Fluorescent red NIR 782, Atto 655, Atto 680, CF660 C, CF660 R, Quasar 705, CF680, IRDye680RD, NIR2, IRDye680LT, HiLyte 680, IRDye700DX, Oyster 680, Iris 5.5, HROMIS LSS 670Z, CHROMIS LSS 690Z, DyLight 680 (Pierce), Magnesium Phthalocyanine, Oxazin 750, SeTa-665, SeTa-667, SeTa-670, PromoFluor 670 (Promo Kine), Dy-682, PromoFluor 680, PromoFluor 700 P, Methylene Blue, Fluorescent Red NIR 730 Reactive, Fluorescent Red NIR 781 Reactive, BM104, BM105 (NHS ester of BM104), SiR720, Atto 740, CF750, Sulfo Cy7, IRDye750, NIR3, DyLight 755, DY-732, DY-734, DY-752, PromoFluor 750 P, HiLyte 750, DY-776, NIR1, IRDye800RS, PromoFluor 770 P, DY-778, CHROMIS 770 C, ZW800-1, ZW800-3, IRIS 7G-WS, IR 780, ESNF31, CF770, Vivotag 800, IRDye800CW, Alexa Fluor 790, CF790, Oyster 800, CHROMIS 770 A, CHROMIS 800 C, CHROMIS 800 A, CHROMIS 830 C, CHROMIS 830 A, DyLight 800, DY-777, DY-782, DY-800, PromoFluor 780 P, NIR-797, PromoFluor 840 P and indocyanine green. Licha *et al* have reviewed dyes and dye conjugates for *in vivo* optical imaging [Topics Curr.Chem., 222, 1-29 (2002); Adv.Drug Deliv.Rev., 57, 1087-1108 (2005)].

More particular examples of chromophores which may be used include: Cy5, Cy5**, Alexa Fluor 647, BODIPY® 630, Atto 647, Oyster 647, IRDye650, BODIPY® 650, DY631, DY632, Cy5.5, IRDye 680RD, Alexa Fluor 660, and Alexa Fluor 680.

The cyanine dyes may be fluorophores of Formula II:

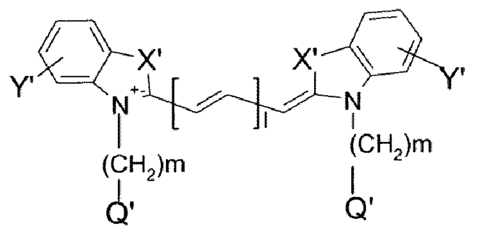

(Formula II)

wherein:

each X' is independently selected from: $-C(CH_3)_2-$, -S-, -O- or $-C[(CH_2)_aCH_3][(CH_2)_bM]-$, wherein a is an integer of value 0 to 5, b is an integer of value 1 to 5, and M is group G or is selected from $SO_3M^1$ or H;
each Y' independently represents 1 to 4 groups selected from the group consisting of: H, $-CH_2NH_2$, $-SO_3M^1$, $-CH_2COOM^1$, -NCS and F, and
wherein the Y' groups are placed in any of the positions of the aromatic ring;
Q' is independently selected from the group consisting of: H, $SO_3M^1$, $NH_2$, $COOM^1$, ammonium, ester groups, benzyl and a group G;
$M^1$ is H or $B^c$;
I is an integer from 1 to 3;
and m is an integer from 1 to 5;
wherein at least one of X', Y' and Q' comprises a group G;
G is a reactive or functional group suitable for attaching to the cMBP peptide.

The G group reacts with a complementary group of the cMBP peptide forming a covalent linkage between the cyanine dye fluorophore and the cMBP peptide. G may be a reactive group that may react with a complementary functional group of the peptide, or alternatively may include a functional group that may react with a reactive group of the cMBP peptide. Examples of reactive and functional groups include: active esters; isothiocyanate; maleimide; haloacetamide; acid halide; hydrazide; vinylsulphone; dichlorotriazine; phosphoramidite; hydroxyl; amino; sulphydryl; carbonyl; carboxylic acid and thiophosphate. G may be an active ester.

By the term "activated ester" or "active ester" is meant an ester derivative of the associated carboxylic acid which is designed to include a better leaving group, and hence permit more facile reaction with nucleophile, such as amines. Examples of suitable active esters are: N-hydroxysuccinimide (NHS), sulpho-succinimidyl ester, pentafluorophenol, pentafluorothiophenol, para-nitrophenol, hydroxybenzotriazole and PyBOP (i.e. benzotriazol-1-yl-oxytripyrrolidinophosphonium hexafluorophosphate). The active esters may be N-hydroxysuccinimide or pentafluorophenol esters, especially N-hydroxysuccinimide esters.

In one embodiment of Formula II:

each X' is selected from the group of $-C(CH_3)_2-$ and $-C(CH_3)[(CH_2)_4M]-$,
wherein M is a G group or $-SO_3M^1$;
each Y' represents $SO_3M^1$, H or 1 to 4 F atoms;
each Q' is selected from a G group and $SO_3M^1$;
1 is preferably 2 and m is preferably 3, 4 or 5;
wherein when either X' or Q' is a G group, it may be a succinimidyl ester.

The cyanine dye may be of Formula III:

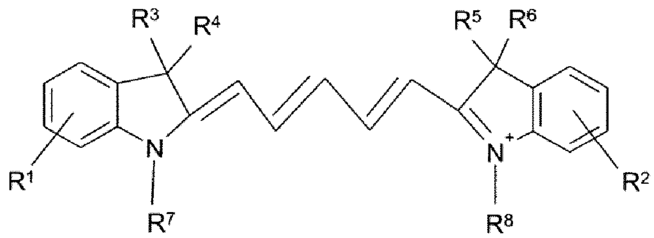

(Formula III)

where:

$R^1$ and $R^2$ are independently H or $SO_3M^1$, and at least one of $R^1$ and $R^2$ is $SO_3M^1$, where $M^1$ is H or $B^c$;
$R^3$ and $R^4$ are independently $C_{1-4}$ alkyl or $C_{1-6}$ carboxyalkyl;
$R^5$, $R^6$, $R^7$ and $R^8$ are independently $R^a$ groups;
wherein $R^a$ is $C_{1-4}$ alkyl, $C_{1-6}$ carboxyalkyl or $-(CH_2)_kSO_3M^1$, where k is an integer of value 3 or 4;
with the proviso that the cyanine dye has a total of 1 to 4 $SO_3M^1$ substituents in the $R^1$, $R^2$ and $R^a$ groups.

The dyes of Formula III may be chosen such that at least one $C_{1-6}$ carboxyalkyl group is present, in order to facilitate conjugation to the cMBP.

Possible individual dyes of Formula III are summarised in Table 1:

Table 1: Chemical Structures of Individual Cyanine Dyes

| | Dye name | | | |
|---|---|---|---|---|
| | Cy5(1) | Cy5(2) | Cy5** | Alexa647 |
| $R^1$ | H | $SO_3H$ | $SO_3H$ | $SO_3H$ |
| $R^2$ | $SO_3H$ | $SO_3H$ | $SO_3H$ | $SO_3H$ |
| $R^3$ | $CH_3$ | $CH_3$ | $CH_3$ | $R^f$ |
| $R^4$ | $CH_3$ | $CH_3$ | $CH_3$ | $CH_3$ |
| $R^5$ | $CH_3$ | $CH_3$ | $CH_3$ | $CH_3$ |
| $R^6$ | $CH_3$ | $CH_3$ | $-(CH_2)_4SO_3H$ | $CH_3$ |
| $R^7$ | $R^f$ | $R^f$ | $R^f$ | $-(CH_2)_4SO_3H$ |

(continued)

| | Dye name | | | |
|---|---|---|---|---|
| | Cy5(1) | Cy5(2) | Cy5** | Alexa647 |
| $R^8$ | $CH_3$ | Et | $-(CH_2)_4SO_3H$ | $-(CH_2)_4SO_3H$ |
| where $R^f = -(CH_2)_5COOH$. | | | | |

Dyes of Formula II that are typically used are Cy5** and Alexa647, with Cy5** being most typical.

When a synthetic linker group (L) is present, it may comprise terminal functional groups which facilitate conjugation to [IM] and $Z^1$-[cMBP]-$Z^2$. When L comprises a peptide chain of 1 to 10 amino acid residues, the amino acid residues may be chosen from glycine, lysine, arginine, aspartic acid, glutamic acid or serine. When L comprises a PEG moiety, it may comprise units derived from oligomerisation of the monodisperse PEG-like structures of Formulae IA (17-amino-5-oxo-6-aza-3, 9, 12, 15-tetraoxaheptadecanoic acid) or IB:

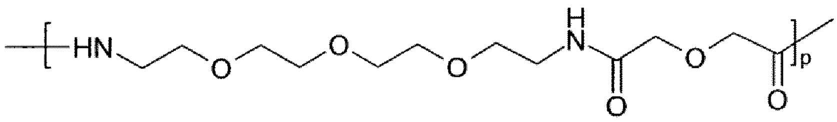

(Formula IA)

wherein p is an integer from 1 to 10. Alternatively, a PEG-like structure based on a propionic acid derivative of Formula IB can be used:

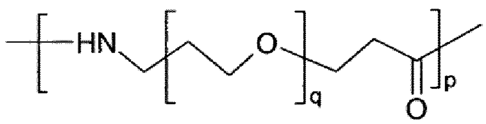

(Formula IB)

where p is as defined for Formula IA and q is an integer from 3 to 15. In Formula IB, p may be 1 or 2, and q may be 5 to 12.

When the linker group does not comprise PEG or a peptide chain, the L groups may have a backbone chain of linked atoms which make up the-$(A)_m$- moiety of 2 to 10 atoms, most preferably 2 to 5 atoms, with 2 or 3 atoms being especially preferred. A minimum linker group backbone chain of 2 atoms confers the advantage that the imaging moiety is well-separated so that any undesirable interaction is minimised.

In Formula I, n may be 0 or 1, typically 0, i.e. no linker group is present.

Imaging agents as described herein may be of Formula IV (comprising SEQ-7):

$M^{IG}$-Ala-Gly-Ser-$Cys^a$-Tyr-$Cys^c$-Ser-Gly-Pro-Pro-Arg-Phe-Glu-$Cys^d$-Trp-
$Cys^b$-Tyr-Glu-Thr-Glu-Gly-Thr-Gly-Gly-Gly-Lys-$M^{IG}$
|
(L)n[IM]

(Formula IV)

wherein the $(L)_n$[IM] group is attached to the epsilon amino group of the Lys residue. The imaging agents of Formula IV may have M (N-terminal Ala) equal to acetyl and $M^{IG}$ (C-terminal Lys) equal to primary amide. In Formula IV, n may be zero and IM may be a cyanine dye, typically a cyanine dye of Formula II. Imaging agents of Formula IV having IM = Cy5** or Alexa647, most typically Cy5** may be used.

Peptides of formula $Z^1$-[cMBP]-$Z^2$ may be obtained by a method of preparation which comprises:

(i) solid phase peptide synthesis of a linear peptide which has the same peptide sequence as the desired cMBP peptide and in which the $Cys^a$ and $Cys^b$ are unprotected, and the $Cys^c$ and $Cys^d$ residues have thiol-protecting groups;
(ii) cleavage from the solid support and treatment of the peptide from step (i) with aqueous base in solution to give a monocyclic peptide with a first disulphide bond linking $Cys^a$ and $Cys^b$;
(iii) removal of the $Cys^c$ and $Cys^d$ thiol-protecting groups and cyclisation to give a second disulphide bond linking $Cys^c$ and $Cys^d$, which is the desired bicyclic peptide product $Z^1$-[cMBP]-$Z^2$.

By the term "protecting group" is meant a group which inhibits or suppresses undesirable chemical reactions, but which is designed to be sufficiently reactive that it may be cleaved from the functional group in question under mild enough conditions that do not modify the rest of the molecule. After deprotection the desired product is obtained. Amine protecting groups are well known to those skilled in the art and are suitably chosen from: Boc (where Boc is tert-butyloxycarbonyl), Fmoc (where Fmoc is fluorenylmethoxycarbonyl), trifluoroacetyl, allyloxycarbonyl, Dde [i.e. 1-(4,4-dimethyl-2,6-dioxocyclohexylidene)ethyl] or Npys (i.e. 3-nitro-2-pyridine sulfenyl). Suitable thiol protecting groups are Trt (Trityl), Acm (acetamidomethyl), *t*-Bu (tert-butyl), *tert*-Butylthio, methoxybenzyl, methylbenzyl or Npys (3-nitro-2-pyridine sulfenyl). The use of further protecting groups are described in 'Protective Groups in Organic Synthesis', Theorodora W. Greene and Peter G. M. Wuts, (John Wiley & Sons, 1991). Typical amine protecting groups are Boc and Fmoc, most typically Boc. Other typical thiol protecting groups are Trt and Acm.

Examples 1 and 2 provide further specific details. Further details of solid phase peptide synthesis are described in P. Lloyd-Williams, F. Albericio and E. Girald; Chemical Approaches to the Synthesis of Peptides and Proteins, CRC Press, 1997. The cMBP peptides are best stored under inert atmosphere and kept in a freezer. When used in solution, it is best to avoid pH above 7 since that risks scrambling of the disulfide bridges, and it is best to avoid low pH as this triggers aggregation of the peptide.

A general method of preparation of the imaging agent of the first aspect, comprises one of steps (i) to (iv):

(i) reaction of a cMBP peptide of formula $Z^1$-[cMBP]-$Z^2$ wherein $Z^1$ is H and $Z^2$ is a $M^{IG}$ with a compound of formula $Y^1$-$(L)_n$-[IM], to give the imaging agent of Formula I wherein [IM] is conjugated at the Z1 position;
(ii) reaction of a cMBP peptide of formula $Z^1$-[cMBP]-$Z^2$ wherein $Z^1 = Z^2 = M^{IG}$ and cMBP comprises an Asp or Glu residue within 4 amino acid residues of either the C- or N- cMBP peptide terminus, and all other Asp/Glu residues of the cMBP peptide are protected, with a compound of formula $Y^2$-$(L)_n$-[IM], to give the imaging agent of Formula I wherein [IM] is conjugated at said Asp or Glu residue of the cMBP peptide;
(iii) reaction of a cMBP peptide of formula $Z^1$-[cMBP]-$Z^3$ wherein $Z^1$ is $M^{IG}$ and $Z^3$ is a $Z^2$ group or an activated ester and all other Asp/Glu residues of the cMBP peptide are protected, with a compound of formula $Y^2$-$(L)_n$-[IM], to give the imaging agent of Formula I wherein [IM] is conjugated at the $Z^2$ position;
(iv) reaction of a cMBP peptide of formula $Z^1$-[cMBP]-$Z^2$ wherein $Z^1 = Z^2 = M^{IG}$ and cMBP comprises a Lys within 4 amino acid residues of either the C- or N- cMBP peptide terminus, with a compound of formula $y^1$-$(L)_n$-[IM], to give the imaging agent of Formula I wherein [IM] is conjugated at a Lys residue of the cMBP peptide;

wherein Z, cMBP, Z , M , L, n and IM are as defined above, and $Z^3$ is a $Z^2$ group or an activated ester;
$Y^1$ is a carboxylic acid, activated ester, isothiocyanate or thiocyanate group;
$Y^2$ is an amine group.

The terms "activated ester" or "active ester" and embodiments thereof are as described above. $Y^2$ is optionally a primary or secondary amine group, typically a primary amine group.

The compound $Z^1$-[cMBP]-$Z^2$ may have both $Z^1$ and $Z^2$ equal to $M^{IG}$. Typical cMBP peptides and $Z^1$/$Z^2$ groups are as described above. In particular, it is typical that the cMBP peptide comprises an Asp, Glu or Lys residue to facilitate conjugation as described for the typical cMBP peptides described above. It is most typical that the cMBP peptide comprises a Lys residue, as described in step (iv).

The preparation of the $Z^1$-[cMBP]-$Z^2$ is described above. The $Z^1$-[cMBP]-$Z^3$ peptide where $Z^3$ is an active ester can be prepared from $Z^1$-[cMBP]-$Z^2$, where $Z^2$ is OH or a biocompatible cation ($B^c$), by conventional methods.

Optical reporter dyes (IM) functionalised suitable for conjugation to peptides are commercially available from GE Healthcare Limited, Atto-Tec, Dyomics, Molecular Probes and others. Most such dyes are available as NHS esters.

Methods of conjugating suitable optical reporters (IM), in particular dyes, to amino acids and peptides are described by Licha (*vide supra*), as well as Flanagan et al [Bioconj.Chem., 8, 751-756 (1997)]; Lin et al, [ibid, 13, 605-610 (2002)] and Zaheer [Mol.Imaging, 1(4), 354-364 (2002)]. Methods of conjugating the linker group (L) to the cMBP peptide use analogous chemistry to that of the dyes alone (see above), and are known in the art.

In addition to SEQ-1, the cMBP may further comprise an Asp or Glu residue within 4 amino acid residues of either C- or N- cMBP peptide terminus, and $-(L)_n$IM may be functionalised with an amine group, which is conjugated to the carboxyl side chain of said Asp or Glu residue to give an amide bond.

In addition to SEQ-1, the cMBP may comprise a Lys residue within 4 amino acid residues of either C- or N- cMBP peptide terminus, and $-(L)_nIM$ may be functionalised with a carboxyl group, which is conjugated to the epsilon amine side chain of said Lys residue to give an amide bond.

cMBP may comprise the amino acid sequence of either SEQ-2 or SEQ-3:

$Ser-Cys^a-Xaa^1-Cys^c-Xaa^2-Gly-Pro-Pro-Xaa^3-Phe-Glu-Cys^d-Trp-Cys^b-Tyr-Xaa^4-Xaa^5-Xaa^6$ (SEQ-2);

$Ala-Gly-Ser-Cys^a-Xaa^1-Cys^c-Xaa^2-Gly-Pro-Pro-Xaa^3-Phe-Glu-Cys^d-Trp-Cys^b-Tyr-Xaa^4-Xaa^5-Xaa^6-Gly-Thr$ (SEQ-3).

$Xaa^3$ may be Arg.

In addition to SEQ-1, SEQ-2 or SEQ-3, cMBP may further comprise at either the N- or C- terminus a linker peptide, which is chosen from -Gly-Gly-Gly-Lys (SEQ-4), -Gly-Ser-Gly-Lys- (SEQ-5) and -Gly-Ser-Gly-Ser-Lys (SEQ-6).

cMBP may have the amino acid sequence (SEQ-7):

$Ala-Gly-Ser-Cys^a-Tyr-Cys^c-Ser-Gly-Pro-Pro-Arg-Phe-Glu-Cys^d-Trp-Cys^b-Tyr-Glu-Thr-Glu-Gly-Thr-Gly-Gly-Gly-Lys$.

Both $Z^1$ and $Z^2$ may independently be $M^{IG}$.

$Z^1$ may be acetyl and $Z^2$ may be a primary amide.

n may be 0.

IM may be a dye having an absorbance maximum in the range 600 to 1,000 nm.

IM may be a cyanine dye.

The cyanine dye may have Formula III:

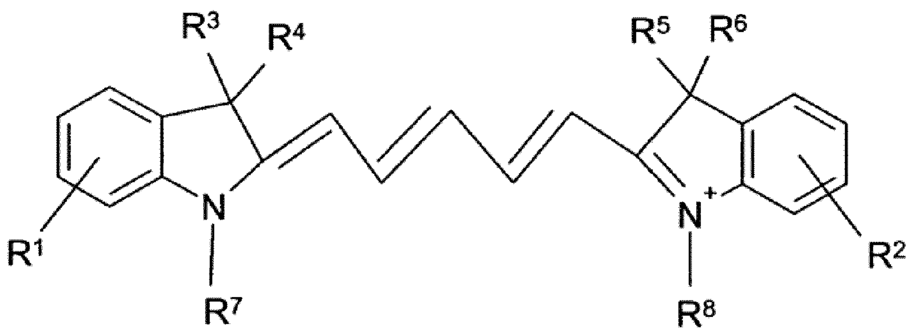

(Formula III)

wherein:

$R^1$ and $R^2$ are independently H or $SO_3M^1$, and at least one of $R^1$ and $R^2$ is $SO_3M^1$, where $M^1$ is H or $B^c$;
$R^3$ and $R^4$ are independently $C_{1-4}$ alkyl or $C_{1-6}$ carboxyalkyl;
$R^5$, $R^6$, $R^7$ and $R^8$ are independently $R^a$ groups;
wherein $R^a$ is $C_{1-4}$ alkyl, $C_{1-6}$ carboxyalkyl or $-(CH_2)_kSO_3M^1$, where k is an integer of value 3 or 4;
with the proviso that the cyanine dye has a total of 1 to 4 $SO_3M^1$ substituents in the $R^1$, $R^2$ and $R^a$ groups.

cMBP may have the amino acid sequence (SEQ-7):

Ala-Gly-Ser-Cys[a]-Tyr-Cys[c]-Ser-Gly-Pro-Pro-Arg-Phe-Glu-Cys[d]-Trp-Cys[b]-Tyr-Glu-Thr-Glu-Gly-Thr-Gly-Gly-Gly-Lys;

$Z^1$ may be acetyl and $Z^2$ may be a primary amide; and
IM may be a dye having an absorbance maximum in the range 600 to 1,000 nm, optionally a cyanine dye, optionally a cyanine dye having Formula III:

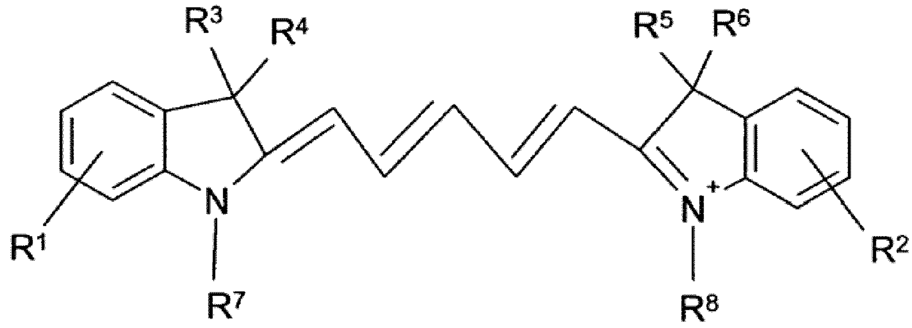

(Formula III)

wherein:

$R^1$ and $R^2$ are independently H or $SO_3M^1$, and at least one of $R^1$ and $R^2$ is $SO_3M^1$, where $M^1$ is H or $B^c$;
$R^3$ and $R^4$ are independently $C_{1-4}$ alkyl or $C_{1-6}$ carboxyalkyl;
$R^5$, $R^6$, $R^7$ and $R^8$ are independently $R^a$ groups;
wherein $R^a$ is $C_{1-4}$ alkyl, $C_{1-6}$ carboxyalkyl or $-(CH_2)_kSO_3M^1$, where k is an integer of value 3 or 4;
with the proviso that the cyanine dye has a total of 1 to 4 $SO_3M^1$ substituents in the $R^1$, $R^2$ and $R^a$ groups.

The formulation, when reconstituted, may have a pH of between approximately pH 6.8 and approximately pH 8.2, optionally between approximately pH 6.8 and approximately pH 8, optionally between approximately pH 7 and approximately pH 8.

The buffering agent may be present in an amount to provide, when reconstituted, a solution having a pH of between approximately pH 6.8 and approximately pH 9, optionally between approximately pH 6.8 and approximately pH 8.2, optionally between approximately pH 6.8 and approximately pH 8, optionally between approximately pH 7 and approximately pH 8.

The mole ratio of the API : buffer may be approximately 1 mole of API : approximately 17 to approximately 47 moles of buffering agent. The mole ratio of the API : buffering agent may be approximately 1 mole of API : approximately 27 to approximately 47 moles of buffering agent. The mole ratio of the API : buffering agent may be approximately 1 mole of API : approximately 30 to approximately 47 moles of buffering agent. The mole ratio of the API : buffering agent may be approximately 1 mole of API : approximately 34 to approximately 47 moles of buffering agent.

The mole ratio of the API : buffering agent may be approximately 1 mole of API : approximately 27 to approximately 38 moles of buffering agent. The mole ratio of the API : buffering agent may be approximately 1 mole of API : approximately 30 to approximately 38 moles of buffering agent. The mole ratio of the API : buffer may be approximately 1 mole of API : approximately 34 to approximately 38 moles of buffering agent.

The mole ratio of the API : buffering agent may be approximately 1 mole of API : approximately 38 moles of buffering agent.

The mole ratio of API : lyoprotectant may be approximately 1 mole API : approximately 105 to approximately 216 moles of lyoprotectant. The mole ratio of API : lyoprotectant may be approximately 1 mole API : approximately 105 to approximately 163 moles of lyoprotectant. The mole ratio of API : lyoprotectant may be approximately 1 mole API : approximately 105 to approximately 154 moles of lyoprotectant. The mole ratio of API : lyoprotectant may be approximately 1 mole API : approximately 105 to approximately 145 moles of lyoprotectant. The mole ratio of API : lyoprotectant may be approximately 1 mole API : approximately 105 to approximately 141 moles of lyoprotectant. The mole ratio of API : lyoprotectant may be approximately 1 mole API : approximately 105 to approximately 132 moles of lyoprotectant.

The mole ratio of API : lyoprotectant may be approximately 1 mole API : approximately 105 to approximately 129 moles of lyoprotectant.

The mole ratio of API : lyoprotectant may be approximately 1 mole API : approximately 129 to approximately 216 moles of lyoprotectant. The mole ratio of API : lyoprotectant may be approximately 1 mole API : approximately 129 to approximately 163 moles of lyoprotectant. The mole ratio of API : lyoprotectant may be approximately 1 mole API : approximately 129 to approximately 154 moles of lyoprotectant. The mole ratio of API : lyoprotectant may be approximately 1 mole API : approximately 129 to approximately 145 moles of lyoprotectant. The mole ratio of API : lyoprotectant may be approximately 1 mole API : approximately 129 to approximately 141 moles of lyoprotectant. The mole ratio of API : lyoprotectant may be approximately 1 mole API : approximately 129 to approximately 132 moles of lyoprotectant.

The mole ratio of API : lyoprotectant may be approximately 1 mole API : approximately 129 moles of lyoprotectant.

The formulation may comprise from approximately 8 to approximately 10 % by weight API, optionally approximately 9 % by weight API.

The formulation may comprise from approximately 7 to approximately 35 % by weight buffering agent, optionally from approximately 12 to approximately 35 % by weight buffering agent, optionally from approximately 15 to approximately 35 % by weight buffering agent, optionally from approximately 18 to approximately 35 % by weight buffering agent, optionally from approximately 25 to approximately 35 % by weight buffering agent, optionally from approximately 32 to approximately 35 % by weight buffering agent, optionally approximately 32 % by weight buffering agent.

The formulation may comprise from approximately 56 to approximately 82 % by weight lyoprotectant, optionally from approximately 56 to approximately 77 % by weight lyoprotectant, optionally from approximately 56 to approximately 75 % by weight lyoprotectant, optionally from approximately 56 to approximately 72 % by weight lyoprotectant, optionally from approximately 56 to approximately 66 % by weight lyoprotectant, optionally from approximately 56 to approximately 58 % by weight lyoprotectant, optionally approximately 58 % by weight lyoprotectant.

It will be understood that the components of the formulation are chosen such that the total amount is 100 % by weight.

The formulation may comprise at least 40mM of buffering agent.

The buffering agent may be at least one of a phosphate buffer and an alkanolamine buffer. The phosphate buffer may comprise hydrogen phosphate and dihydrogen phosphate. The phosphate buffer may be hydrated.

The alkanolamine buffer may comprise tris(hydroxymethyl)aminomethane. The alkanolamine buffer may comprise 2-amino-2-(hydroxymethyl)propane-1,3-diol.

The lyoprotectant may be at least one of a sugar, an alcohol and a sugar alcohol, and derivatives thereof. Optionally the lyoprotectant is at least one of a sugar and a sugar alcohol, and derivatives thereof. Optionally the lyoprotectant is at least one of a monosaccharaide, a disaccharide, an oligosaccharide, a polysaccharide, and derivatives thereof. The lyoprotectant may be at least one of sucrose and mannitol, and derivatives thereof. The lyoprotectant may be mannitol, or a derivative thereof.

The formulation may further comprise a tonicity regulator.

The lyoprotectant may also act a tonicity regulator, in which case it is a combined lyoprotectant and tonicity regulator.

According to a second aspect of the invention, there is provided a method of preparing a lyophilised formulation, the method comprising the steps of:

a) providing an active pharmaceutical ingredient (API), a buffering agent, and a lyoprotectant to a lyophilisation vessel;
b) performing a first water removal step; and
c) performing a second water removal step;

wherein the lyoprotectant and the buffering agent are added before the lyophilisation is carried out, and the API is an imaging agent comprising at least one cMet binding peptide, suitable for optically imaging the mammalian body, the imaging agent comprising a compound of Formula I:

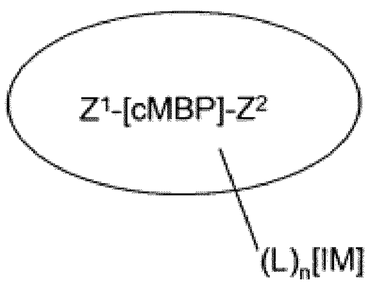

(Formula I)

wherein:

$Z^1$ is attached to the N-terminus of cMBP, and is H or $M^{IG}$;
$Z^2$ is attached to the C-terminus of cMBP, and is OH, $NH_2$, $OB^c$ or $M^{IG}$;

wherein $B^c$ is a biocompatible cation;
cMBP is a cMet binding cyclic peptide of 17 to 30 amino acids, which comprises the amino acid sequence (SEQ-1): $Cys^a$-$Xaa^1$-$Cys^c$-$Xaa^2$-Gly-Pro-Pro-$Xaa^3$-Phe-Glu-$Cys^d$-Trp-$Cys^b$-Tyr-$Xaa^4$-$Xaa^5$-$Xaa^6$;
wherein: $Xaa^1$ is Asn, His or Tyr;

$Xaa^2$ is Gly, Ser, Thr or Asn;
$Xaa^3$ is Thr or Arg;
$Xaa^4$ is Ala, Asp, Glu, Gly or Ser;
$Xaa^5$ is Ser or Thr;
$Xaa^6$ is Asp or Glu;

and $Cys^{a-d}$ are each cysteine residues such that residues a and b as well as c and d are cyclised to form two separate disulphide bonds;

$M^{IG}$ is a metabolism inhibiting group, which is a biocompatible group that inhibits or suppresses *in vivo* metabolism of the peptide;
L is a synthetic linker group of formula -(A)m- wherein each A is independently $-CR_2-$, -CR=CR-, -C=C-, $-CR_2CO_2-$,$-CO_2CR_2-$, -NRCO-, -CONR-, -NR(C=O)NR-, -NR(C=S)NR-,$-SO_2NR-$, $-NRSO_2-$, $CR_2OCR_2-$, $-CR_2SCR_2-$, $CR_2NRCR_2-$, a $C_{4-8}$ cycloheteroalkylene group, a $C_{4-8}$ cycloalkylene group, a $C_{5-12}$ arylene group, a $C_{3-12}$ heteroarylene group, an amino acid, a sugar or a monodisperse polyethyleneglycol (PEG) building block;
each R is independently chosen from H, $C_{1-4}$ alkyl, $C_{2-4}$ alkenyl, $C_{2-4}$ alkynyl, $C_{1-4}$ alkoxyalkyl or $C_{1-4}$ hydroxyalkyl;
m is an integer of value 1 to 20;
n is an integer of value 0 or 1;
IM is an optical reporter imaging moiety suitable for imaging the mammalian body using light of violet to near-infrared wavelength (400 to 1,200 nm);

wherein the formulation comprises from approximately 4 to approximately 12 % by weight API, from approximately 3 to approximately 35 % by weight buffering agent, and from approximately 56 to approximately 91 % by weight lyoprotectant; and wherein the formulation, when reconstituted, has a pH of between approximately pH 6.8 and approximately pH 9.

The first water removal step may be carried out at a temperature of approximately -30 °C or lower, optionally approximately -35 °C or lower, optionally approximately -38 °C or lower.

The second water removal step may be carried out at a temperature of approximately 10 °C or higher, optionally approximately 15 °C or higher, optionally approximately 20 °C or higher.

At least one of the first and second water removal steps may be carried out at a pressure of 50 μbar or less. Optionally both of the first and second water removal steps may be carried out at a pressure of 50 μbar or less.

The lyophilised formulation may be the lyophilised formulation of the first aspect of the invention.

According to a third aspect of the invention, there is provided a pharmaceutical composition comprising the formulation of the first aspect of the invention and a biocompatible carrier, in a form suitable for mammalian administration.

The biocompatible carrier may be a solvent, typically an aqueous solvent, typically water. The solvent may be a fluid.

The "biocompatible carrier" may be a fluid, especially a liquid, in which the imaging agent can be suspended or dissolved, such that the composition is physiologically tolerable, i.e. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is isotonic); an aqueous solution of one or more tonicity-adjusting substances (e.g., salts of plasma cations with biocompatible counterions), sugars (e.g., glucose or sucrose), sugar alcohols (e.g., sorbitol or mannitol), glycols (e.g., glycerol), or other non-ionic polyol materials (e.g., polyethyleneglycols, propylene glycols and the like). The biocompatible carrier may be pyrogen-free water for injection or isotonic saline. The imaging agents and biocompatible carrier are each supplied in suitable vials or vessels which comprise a sealed container which permits

maintenance of sterile integrity and/or radioactive safety, plus optionally an inert headspace gas (e.g., nitrogen or argon), whilst permitting addition and withdrawal of solutions by syringe or cannula. A preferred such container is a septum-sealed vial, wherein the gas-tight closure is crimped on with an overseal (typically of aluminium). The closure is suitable for single or multiple puncturing with a hypodermic needle (e.g., a crimped-on septum seal closure) whilst maintaining sterile integrity. Such containers have the additional advantage that the closure can withstand vacuum if desired (e.g., to change the headspace gas or degas solutions), and withstand pressure changes such as reductions in pressure without permitting ingress of external atmospheric gases, such as oxygen or water vapour.

The pharmaceutical composition may have a dosage suitable for a single patient and may be provided in a suitable syringe or container.

Multiple dose containers comprise a single bulk vial (e.g., of 10 to 30 $cm^3$ volume) which contains multiple patient doses, whereby single patient doses can thus be withdrawn into clinical grade syringes at various time intervals during the viable lifetime of the preparation to suit the clinical situation. Pre-filled syringes are designed to contain a single human dose, or "unit dose" and are therefore preferably a disposable or other syringe suitable for clinical use. The pharmaceutical compositions may have a dosage suitable for a single patient and may be provided in a suitable syringe or container, as described above.

The pharmaceutical composition may have a pH of between approximately pH 6.8 and approximately pH 9, optionally between approximately pH 6.8 and approximately pH 8.2, optionally between approximately pH 7 and approximately pH 8.

According to a fourth aspect of the invention, there is provided a kit for the preparation of the pharmaceutical composition of the third aspect of the invention, the kit comprising the formulation of the first aspect of the invention in sterile, solid form such that upon reconstitution with a sterile supply of the biocompatible carrier of the third aspect of the invention, dissolution occurs to give the desired pharmaceutical composition.

The sterile, solid form may be a lyophilised solid.

Disclosed herein, and not part of the claimed invention, there is provided a method of imaging of the mammalian body which comprises use of at least one of the formulation of the first aspect of the invention and the pharmaceutical composition of the third aspect of the invention.

It will be appreciated that for *in vivo* use, the formulation is made into a form suitable for mammalian administration by, for example, reconstitution with a biocompatible carrier.

The imaging may be *in vivo.*

The imaging may be optical imaging.

By the term "optical imaging" is meant any method that forms an image for detection, staging or diagnosis of disease, follow up of disease development or for follow up of disease treatment based on interaction with light in the red to near-infrared region (wavelength 400-1,200 nm). Optical imaging further includes all methods from direct visualization without use of any device and involving use of devices such as various scopes, catheters and optical imaging equipment, e.g., computer-assisted hardware for tomographic presentations. The modalities and measurement techniques include, but are not limited to: fluorescence imaging, luminescence imaging; endoscopy; fluorescence endoscopy; optical coherence tomography; transmittance imaging; time resolved transmittance imaging; confocal imaging; nonlinear microscopy; photoacoustic imaging; acousto-optical imaging; spectroscopy; reflectance spectroscopy; interferometry; coherence interferometry; diffuse optical tomography and fluorescence mediated diffuse optical tomography (continuous wave, time domain and frequency domain systems), and measurement of light scattering, absorption, polarization, luminescence, fluorescence lifetime, quantum yield, and quenching. Further details of these techniques are provided by: (Tuan Vo-Dinh (editor): "Biomedical Photonics Handbook" (2003), CRC Press LCC; Mycek & Pogue (editors): "Handbook of Biomedical Fluorescence" (2003), Marcel Dekker, Inc.; Splinter & Hopper: "An Introduction to Biomedical Optics" (2007), CRC Press LCC.

The imaging may be to obtain images of sites of cMet over-expression or localisation.

The formulation of the first aspect of the invention or the pharmaceutical composition of the third aspect of the invention may have been previously administered to the mammalian body.

The optical imaging method may use Fluorescence Reflectance Imaging (FRI). In FRI, the imaging agent of the present invention is administered to a subject to be diagnosed, and subsequently a tissue surface of the subject is illuminated with an excitation light - usually continuous wave (CW) excitation. The light excites the reporter molecule (IM). Fluorescence from the imaging agent, which is generated by the excitation light, is detected using a fluorescence detector. The returning light may be filtered to separate out the fluorescence component (solely or partially). An image is formed from the fluorescent light. Usually minimal processing is performed (no processor to compute optical parameters such as lifetime, quantum yield etc.) and the image maps the fluorescence intensity. The imaging agent is designed to concentrate in the disease area, producing higher fluorescence intensity. Thus the disease area produces positive contrast in a fluorescence intensity image. The image may be obtained using a CCD camera or chip, such that real-time imaging is possible.

The wavelength for excitation varies depending on the type of dye used. The apparatus for generating the

excitation light may be a conventional excitation light source such as: a laser (e.g., ion laser, dye laser or semiconductor laser); halogen light source or xenon light source. Various optical filters may optionally be used to obtain the optimal excitation wavelength.

The method may comprise the steps of:

a) illuminating a tissue surface of interest with an excitation light;
b) detecting fluorescence from the imaging agent, which is generated by excitation of the imaging agent;
c) optionally filtering the light detected by the fluorescence detector to separate out the fluorescent component; and
d) forming an image of the tissue surface of interest from the fluorescent light of steps (b) or (c).

The excitation light of step (a) may be continuous wave (CW) in nature.

Step (a) may be carried out within the mammalian body.

An alternative imaging method uses FDPM (frequency-domain photon migration). This has advantages over continuous-wave (CW) methods where greater depth of detection of the IM within tissue is important [Sevick-Muraca et al, Curr.Opin.Chem.Biol., 6, 642-650 (2002)]. For such frequency/time domain imaging, it is advantageous if the IM has fluorescent properties which can be modulated depending on the tissue depth of the lesion to be imaged, and the type of instrumentation employed.

The method may comprise the steps of:

a) exposing light-scattering biologic tissue of said mammalian body having a heterogeneous composition to light from a light source with a pre-determined time varying intensity to excite the imaging agent, the tissue multiply-scattering the excitation light;
b) detecting a multiply-scattered light emission from the tissue in response to said exposing;
c) quantifying a fluorescence characteristic throughout the tissue from the emission by establishing a number of values with a processor, the values each corresponding to a level of the fluorescence characteristic at a different position within the tissue, the level of the fluorescence characteristic varying with heterogeneous composition of the tissue; and
d) generating an image of the tissue by mapping the heterogeneous composition of the tissue in accordance with the values of step (c).

The optical imaging method may comprise fluorescence imaging, optionally fluorescence endoscopy.

The method may be used to assist in detection, diagnosis, surgery, staging, treatment, monitoring of treatment, monitoring of disease progression or monitoring therapy.

The method may be used to assist in detection, diagnosis, surgery, staging, treatment, monitoring of treatment, monitoring of disease progression or monitoring therapy of cancer, optionally colorectal cancer.

According to a further aspect of the invention, there is provided at least one of the formulation of the first aspect of the invention and the pharmaceutical composition of the third aspect of the invention for use as an imaging agent in imaging of the mammalian body.

According to a further aspect of the invention, there is provided at least one of the formulation of the first aspect of the invention and the pharmaceutical composition of the third aspect of the invention for use as a medicament.

According to a further aspect of the invention, there is provided at least one of the formulation of the first aspect of the invention and the pharmaceutical composition of the third aspect of the invention for use in detection, diagnosis, surgery, staging, treatment, monitoring of treatment, monitoring of disease progression or monitoring therapy.

According to a further aspect of the invention there is provided at least one of the formulation of the first aspect of the invention and the pharmaceutical composition of the third aspect of the invention for use in the detection, diagnosis, surgery, staging, treatment, monitoring of treatment, monitoring of disease progression or monitoring therapy of one or more of a precancerous condition and cancer, optionally one or more of colorectal cancer, oesophegal cancer, breast cancer, prostate cancer, head cancer, neck cancer, ovarian cancer, rectal cancer, pancreatic cancer, thyroid cancer, gastric cancer and sarcoma.

According to a further aspect of the invention, there is provided at least one of the formulation of the first aspect of the invention and the pharmaceutical composition of the third aspect of the invention for use in the detection, diagnosis, surgery, staging, treatment, monitoring of treatment, monitoring of disease progression or monitoring therapy of sites of cMet over-expression or localisation.

According to a further aspect of the invention, there is provided at least one of the formulation of the first aspect of the invention and the pharmaceutical composition of the third aspect of the invention for use in obtaining an image of sites of cMet over-expression or localisation, optionally *in vivo*.

Disclosed herein, and not part of the claimed invention, there is provided a method of detection, diagnosis, surgery, staging, treatment, monitoring of treatment, monitoring of disease progression or monitoring therapy using at

least one of the formulation of the first aspect of the invention and the pharmaceutical composition of the third aspect of the invention.

Disclosed herein, and not part of the claimed invention, there is provided a method of imaging the mammalian body using at least one of the formulation of the first aspect of the invention and the pharmaceutical composition of the third aspect of the invention.

Disclosed herein, and not part of the claimed invention, there is provided a method of detection, diagnosis, surgery, staging, treatment, monitoring of treatment, monitoring of disease progression or monitoring therapy of one or more of a precancerous condition and cancer, optionally one or more of colorectal cancer, oesophegal cancer, breast cancer, prostate cancer, head cancer, neck cancer, ovarian cancer, rectal cancer, pancreatic cancer, thyroid cancer, gastric cancer and sarcoma, using at least one of the formulation of the first aspect of the invention and the pharmaceutical composition of the third aspect of the invention.

Disclosed herein, and not part of the claimed invention, there is provided a method of detection, diagnosis, surgery, staging, treatment, monitoring of treatment, monitoring of disease progression or monitoring therapy of sites of cMet over-expression or localisation using at least one of the formulation of the first aspect of the invention and the pharmaceutical composition of the third aspect of the invention.

Disclosed herein, and not part of the claimed invention, there is provided a method of obtaining an image of sites of cMet over-expression or localisation, optionally *in vivo,* using at least one of the formulation of the first aspect of the invention and the pharmaceutical composition of the third aspect of the invention.

Disclosed herein, and not part of the claimed invention, there is provided the use of at least one of the formulation of the first aspect of the invention and the pharmaceutical composition of the third aspect of the invention.

Disclosed herein, and not part of the claimed invention, there is provided the use of at least one of the formulation of the first aspect of the invention and the pharmaceutical composition of the third aspect of the invention in detection, diagnosis, surgery, staging, treatment, monitoring of treatment, monitoring of disease progression or monitoring therapy.

Disclosed herein, and not part of the claimed invention, there is provided use of at least one of the formulation of the first aspect of the invention and the pharmaceutical composition of the third aspect of the invention as an imaging agent.

Disclosed herein, and not part of the claimed invention, there is provided the use of at least one of the formulation of the first aspect of the invention and the pharmaceutical composition of the third aspect of the invention in the detection, diagnosis, surgery, staging, treatment, monitoring of treatment, monitoring of disease progression or monitoring therapy of one or more of a precancerous condition and cancer, optionally one or more of colorectal cancer, oesophegal cancer, breast cancer, prostate cancer, head cancer, neck cancer, ovarian cancer, rectal cancer, pancreatic cancer, thyroid cancer, gastric cancer and sarcoma.

Disclosed herein, and not part of the claimed invention, there is provided the use of at least one of the formulation of the first aspect of the invention and the pharmaceutical composition of the third aspect of the invention in the detection, diagnosis, surgery, staging, treatment, monitoring of treatment, monitoring of disease progression or monitoring therapy of sites of cMet over-expression or localisation.

Disclosed herein, and not part of the claimed invention, there is provided the use of at least one of the formulation of the first aspect of the invention and the pharmaceutical composition of the third aspect of the invention in obtaining an image of sites of cMet over-expression or localisation, optionally *in vivo.*

The alternative features and different embodiments as described apply to each and every aspect and each and every embodiment thereof *mutatis mutandis.*

## Brief Description of the Drawings

Embodiments of the invention will now be described, by way of example, with reference to the drawings, in which: Fig. 1 depicts an API (EMI-137).

## Detailed Description

An optical imaging agent as described in WO2008/139207 was used to run a series of experiments to find a more stable formulation that illustrated better reconstitution, in particular at an appropriate pH and/or tonicity for intravenous use. The compound used was EMI-137 as shown in Fig. 1.

The experiments discussed below used the acetate salt of EMI-137, but all weights and calculations were made using the free ion.

After reconstitution, an intravenous drug product should be an isotonic solution and have a physiological pH to make it suitable for intravenous administration. It should also be homogenous and should solubilise without formation of agglomerates. However, when dissolved in water EMI-137 has a pH of 4.6, and shows a significant degree of formation of larger structures or agglomerates. This agglomeration is only partly reversible upon increasing the pH of the solution,

thus making it unsuitable for intravenous administration. Therefore, a formulation is required that on reconstitution will be a pH suitable for intravenous administration, and that is substantially homogeneous and substantially free from agglomerates or particles. Furthermore, EMI-137 has a reasonably short shelf-life, and therefore for practical and safety purposes is not very useable in its isolated form. Therefore, a formulation is required that will extend the stability and shelf-life of EMI-137.

## Preparation of and Imaging Using EMI-137

Example 1 provides the synthesis of a cMBP peptide (Compound 1 (comprising SEQ-7)). Example 2 provides the synthesis of a related peptide as a negative control, in which the peptide sequence of Compound 1 is scrambled, to give compound 2, which has the peptide sequence:

Thr-Gly-Glu-Cys-Thr-Cys-Pro-Tyr-Trp-Glu-Phe-Arg-Pro-Cys-Glu-Cys-Gly-Ser-Tyr-Ser-Gly-Ala-Gly-Gly-Gly-Lys

(SEQ-8).

Example 3 provides the synthesis of cyanine dye Cy5**. Example 4 provides the synthesis of an active ester of Cy5**. Example 5 provides the conjugation of cyanine dyes to peptides (cMBP peptide and control). Compounds 3 (comprising SEQ-7), 4 (comprising SEQ-8), 5 (comprising SEQ-7), 6 (comprising SEQ-7) and 7 (comprising SEQ-8) were compared in this way. Example 6 provides a method of determination of the affinity of the peptides to cMet *in vitro.* The results show that the binding is selective, even when an optical reporter imaging moiety (a cyanine dye) is attached. Example 7 provides data on the *in vivo* testing of Compounds 5 and 7 in an animal model of cancer. Superior tumour: background ratios were seen with Compound 5, whereas Compound 7 (negative control) did not discriminate between tumour and background.

Structures of the Compounds are provided in Table 2 below.

Table 2: Structures of Compounds

| No. | Structure of Compounds. |
|---|---|
| 1 | Ac-AGSCYCSGPPRFECWCYFTEGTGGGK-$NH_2$ |
| 2 | Ac- TGECTCPYWEFRPCECGSYSGAGGGK-$NH_2$ (negative control) |
| 3 | 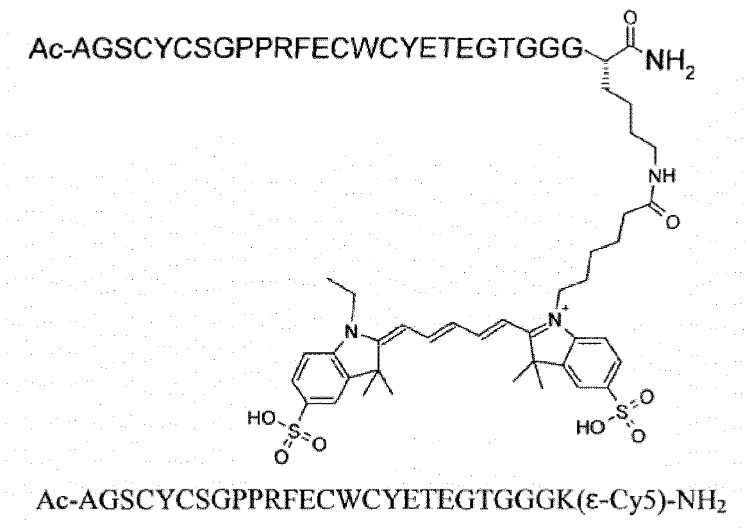 Ac-AGSCYCSGPPRFECWCYETEGTGGGK(ε-Cy5)-$NH_2$ |

(continued)

| No. | Structure of Compounds. |
|---|---|
| 4 | 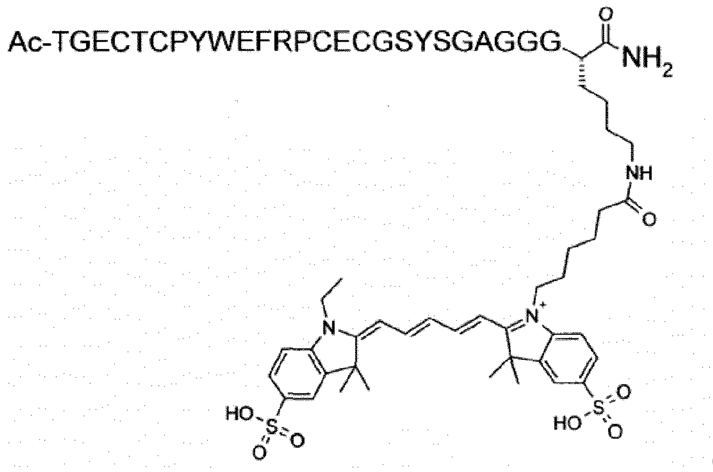 (negative control) |
| 5 | 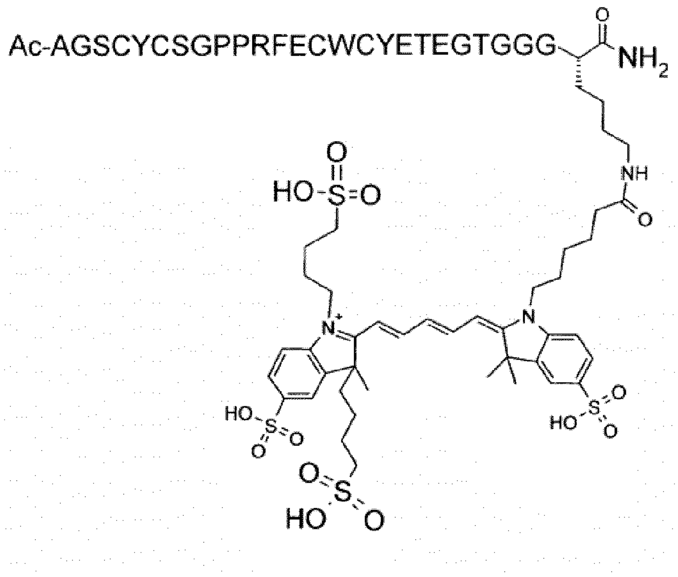 Ac-AGSCYCSGPPRFECWCYETEGTGGGK(ε-Cy5**)-$NH_2$ |
| 6 | Ac-AGSCYCSGPPRFECWCYCTEGTGGGK(ε-Alexa647)-$NH_2$ |
| 7 | Ac-TGECTCPYWEFRPCECGSYSGAGGGK(ε-Cy5**)-$NH_2$ (negative control). |

**Example 1: Synthesis of Compound 1**

*Step (a): synthesis of protected precursor linear peptide.*

The precursor linear peptide has the structure:

Ac-Ala-Gly-Ser-Cys-Tyr-Cys(Acm)-Ser-Gly-Pro-Pro-Arg-Phe-Glu-Cys(Acm)-Trp-Cys-Tyr-Glu-Thr-Glu-Gly-Thr-Gly-Gly-Gly-Lys-$NH_2$

(comprising SEQ-7).

The peptidyl resin H-Ala-Gly-Ser(tBu)-Cys(Trt)-Tyr(tBu)-Cys(Acm)-Ser(tBu)-Gly-Pro-Pro-Arg(Pbf)-Phe-Glu(OtBu)-Cys(Acm)-Trp(Boc)-Cys(Trt)-Tyr(tBu)-Glu(OtBu)-Thr($\Psi^{Me,Me}$pro)-Glu(OtBu)-Gly-Thr(tBu)-Gly-Gly-Gly-Lys(Boc)-Polymer (comprising SEQ-7) was assembled on an Applied Biosystems 433A peptide synthesizer using Fmoc

chemistry starting with 0.1 mmol Rink Amide Novagel resin. An excess of 1 mmol pre-activated amino acids (using HBTU) was applied in the coupling steps. Glu-Thr pseudoproline (Novabiochem 05-20-1122) was incorporated in the sequence. The resin was transferred to a nitrogen bubbler apparatus and treated with a solution of acetic anhydride (1 mmol) and NMM (1 mmol) dissolved in DCM (5 mL) for 60 min. The anhydride solution was removed by filtration and the resin washed with DCM and dried under a stream of nitrogen.

The simultaneous removal of the side-chain protecting groups and cleavage of the peptide from the resin was carried out in TFA (10 mL) containing 2.5 % TIS, 2.5 % 4-thiocresol and 2.5 % water for 2 hours and 30 min. The resin was removed by filtration, TFA removed *in vacuo* and diethyl ether added to the residue. The formed precipitate was washed with diethyl ether and air-dried affording 264 mg of crude peptide.

Purification by preparative HPLC (gradient: 20-30 % B over 40 min where A = $H_2O$/0.1 % TFA and B = ACN/0.1 % TFA, flow rate: 10 mL/min, column: Phenomenex Luna 5μ C18 (2) 50 x 21.20 mm, detection: UV 214 nm, product retention time: 30 min) of the crude peptide afforded 100 mg of pure Compound 1 linear precursor. The pure product was analysed by analytical HPLC (gradient: 10-40 % B over 10 min where A = H2CVO.1 % TFA and B = ACN/0.1 % TFA, flow rate: 0.3 mL/min, column:

Phenomenex Luna 3 μ C18 (2) 50 x 2 mm, detection: UV 214 nm, product retention time: 6.54 min). Further product characterisation was carried out using electrospray mass spectrometry ($MH_2^{2+}$ calculated: 1464.6, $MH_2^{2+}$ found: 1465.1).

*Step (b): Formation of Monocyclic Cys4-16 disulfide bridge*

Cys4-16; Ac-Ala-Gly-Ser-Cys-Tyr-Cys(Acm)-Ser-Gly-Pro-Pro-Arg-Phe-Glu-Cys(Acm)-Trp-Cys-Tyr-Glu-Thr-Glu-Gly-Thr-Gly-Gly-Gly-Lys-$NH_2$

(comprising SEQ-7).

The linear precursor from step (a) (100 mg) was dissolved in 5 % DMSO/water (200 mL) and the solution adjusted to pH 6 using ammonia. The reaction mixture was stirred for 5 days. The solution was then adjusted to pH 2 using TFA and most of the solvent removed by evaporation *in vacuo.* The residue (40 mL) was injected in portions onto a preparative HPLC column for product purification.

Purification by preparative HPLC (gradient: 0 % B for 10 min, then 0-40 % B over 40 min where A = $H_2O$/0.1 % TFA and B = ACN/0.1 % TFA, flow rate: 10 mL/min, column: Phenomenex Luna 5μ C18 (2) 250 x 21.20 mm, detection: UV 214 nm, product retention time: 44 min) of the residue afforded 72 mg of pure Compound 1 monocyclic precursor. The pure product (as a mixture of isomers P1 to P3) was analysed by analytical HPLC (gradient: 10-40 % B over 10 min where A = $H_2O$/0.1 % TFA and B = ACN/0.1 % TFA, flow rate: 0.3 mL/min, column: Phenomenex Luna 3μ C18 (2) 50 x 2 mm, detection: UV 214 nm, product retention time: 5.37 min (P1); 5.61 min (P2); 6.05 min (P3)). Further product characterisation was carried out using electrospray mass spectrometry ($MH_2^{2+}$ calculated: 1463.6, $MH_2^{2+}$ found: 1464.1 (P1); 1464.4 (P2); 1464.3 (P3)).

*Step (c): Formation of Second Cys6-14 disulfide bridge (Compound 1)*

The monocyclic precursor from step (b) (72 mg) was dissolved in 75 % AcOH/water (72 mL) under a blanket of nitrogen. 1 M HCl (7.2 mL) and 0.05 M $I_2$ in AcOH (4.8 mL) were added in that order and the mixture stirred for 45 min. 1 M ascorbic acid (1 mL) was added giving a colourless mixture. Most of the solvents were evaporated *in vacuo* and the residue (18 mL) diluted with water/0.1 % TFA (4 mL) and the product purified using preparative HPLC.

Purification by preparative HPLC (gradient: 0 % B for 10 min, then 20-30 % B over 40 min where A = $H_2O$/0.1 % TFA and B = ACN/0.1 % TFA, flow rate: 10 mL/min, column: Phenomenex Luna 5μ C18 (2) 250 x 21.20 mm, detection: UV 214 nm, product retention time: 43-53 min) of the residue afforded 52 mg of pure Compound 1. The pure product was analysed by analytical HPLC (gradient: 10-40 % B over 10 min where A = $H_2O$/0.1 % TFA and B = ACN/0.1 % TFA, flow rate: 0.3 mL/min, column: Phenomenex Luna 3μ C18 (2) 50 x 2 mm, detection: UV 214 nm, product retention time: 6.54 min). Further product characterisation was carried out using electrospray mass spectrometry ($MH_2^{2+}$ calculated: 1391.5, $MH_2^{2+}$ found: 1392.5).

**Example 2: Synthesis of Compound 2**

Ac-Thr-Gly-Glu-Cys-Thr-Cys(Acm)-Pro-Tyr-Trp-Glu-Phe-Arg-Pro-Cys(Acm)-Glu- Cys-Gly-Ser-Tyr-Ser-Gly-Ala-Gly-Gly-Gly-Lys-$NH_2$

Compound 2 (comprising SEQ-8) is a negative control, where the peptide sequence of Compound 1 has been scrambled.

*Step (a): Synthesis of protected precursor linear peptide*

The peptidyl resin H-Thr(tBu)-Gly-Glu(OtBu)-Cys(Trt)-Thr(tBu)-Cys(Acm)-Pro-Tyr(tBu)-Trp(Boc)-Glu(OtBu)-Phe-Arg(Pbf)-Pro-Cys(Acm)-Glu(OtBu)-Cys(Trt)-Gly-Ser(tBu)-Tyr(tBu)-Ser($\Psi^{Me,Me}$pro)-Gly-Ala-Gly-Gly-Gly-Lys(Boc)-Polymer (comprising SEQ-8) was assembled on an Applied Biosystems 433A peptide synthesizer using Fmoc chemistry starting with 0.1 mmol Rink Amide Novagel resin. An excess of 1 mmol pre-activated amino acids (using HBTU) was applied in the coupling steps. Tyr-Ser pseudoproline (Novabiochem 05-20-1014) was incorporated in the sequence. The resin was transferred to a nitrogen bubbler apparatus and treated with a solution of acetic anhydride (1 mmol) and NMM (1 mmol) dissolved in DCM (5 mL) for 60 min. The anhydride solution was removed by filtration and the resin washed with DCM and dried under a stream of nitrogen.

The simultaneous removal of the side-chain protecting groups and cleavage of the peptide from the resin was carried out in TFA (10 mL) containing 2.5 % TIS, 2.5 % 4- thiocresol and 2.5 % water for 2 hours and 10 min. The resin was removed by filtration, TFA removed *in vacuo* and diethyl ether added to the residue. The formed precipitate was washed with diethyl ether and air-dried affording 216 mg of crude peptide.

Purification by preparative HPLC (gradient: 20-30 % B over 40 min where A = $H_2O$/0.1 % TFA and B = ACN/0.1 % TFA, flow rate: 50 mL/min, column: Phenomenex Luna 5$\mu$ C18 (2) 250 x 50 mm, detection: UV 214 nm, product retention time: 34.1 min) of the crude peptide afforded pure DX-1662 negative control linear precursor dissolved in 200 mL of ACN/water. The pure product was analysed by analytical HPLC (gradient: 10-40 % B over 5 min where A = $H_2O$/0.1 % TFA and B = ACN/0.1 % TFA, flow rate: 0.6 mL/min, column: Phenomenex Luna 3$\mu$ C18 (2) 20 x 2 mm, detection: UV 214 nm, product retention time: 3.52 min). Further product characterisation was carried out using electrospray mass spectrometry ($MH_2^{2+}$ calculated: 1464.6, $MH_2^{2+}$ found: 1464.9).

*Step (b): Formation of Monocyclic Cvs4-16 disulfide bridge.*

Cys4-16; Ac-Thr-Gly-Glu-Cys-Thr-Cys(Acm)-Pro-Tyr-Trp-Glu-Phe-Arg-Pro-Cys(Acm)-Glu-Cys-Gly-Ser-Tyr-Ser-Gly-Ala-Gly-Gly-Gly-Lys-$NH_2$ (comprising SEQ-8).

DMSO (10 mL) was added to the negative control linear precursor solution from step (a) (200 mL, see 4.3.1) and the solution adjusted to pH 7 using ammonia. The reaction mixture was heated at 40 °C for 18 hours, then at 60 °C for 60 min. The solution was adjusted to pH 2 using TFA and ACN removed by evaporation *in vacuo.* The residue was subjected to preparative HPLC purification.

Purification by preparative HPLC (gradient: 0 % B for 5 min, then 20-30 % B over 60 min where A = $H_2O$/0.1 % TFA and B = ACN/0.1 % TFA, flow rate: 50 mL/min, column: Phenomenex Luna 5$\mu$ C18 (2) 250 x 50 mm, detection: UV 214 ran, product retention time: 29.6 min) of the residue afforded pure negative control monocyclic precursor in 100 mL of ACN/water. The pure product was analysed by analytical HPLC (gradient: 10-40 % B over 5 min where A = $H_2O$/0.1 % TFA and B = ACN/0.1 % TFA, flow rate: 0.6 mL/min, column: Phenomenex Luna 3$\mu$ C18 (2) 20 x 2 mm, detection: UV 214 nm, product retention time: 3.46 min). Further product characterisation was carried out using electrospray mass spectrometry ($MH_2^{2+}$ calculated: 1463.6, $MH_2^{2+}$ found: 1463.7).

*Step (c): Formation of Second Cvs6-14 disulfide bridge (Compound 2)*

Cys4-16, 6-14; Ac-Thr-Gly-Glu-Cys-Thr-Cys-Pro-Tyr-Trp-Glu-Phe-Arg-Pro-Cys-Glu-Cys-Gly-Ser-Tyr-Ser-Gly-Ala-Gly-Gly-Gly-Lys-$NH_2$ (comprising SEQ-8).

The negative control monocyclic precursor solution from step (b) (100 mL) was diluted with AcOH (100 mL). 1 M HCl (5 mL) and 0.05 M $I_2$ in AcOH (7 mL) were added in that order under a blanket of argon and the mixture stirred for 20 min. 1 M ascorbic acid (1 mL) was added giving a colourless mixture. Most of the solvents were evaporated *in vacuo* and the residue (30 mL) diluted with water/0.1 % TFA (100 mL) and the product purified using preparative HPLC. Purification by preparative HPLC (gradient: 0 % B for 10 min, then 20-30 % B over 60 min where A = $H_2O$/0.1 % TFA and B = ACN/0.1 % TFA, flow rate: 50 mL/min, column: Phenomenex Luna 5μ C18 (2) 250 x 50 mm, detection: UV 214 nm, product retention time: 32.8 min) of the residue afforded 30 mg of pure Compound 2. The pure product was analysed by analytical HPLC (gradient: 10-40 % B over 10 min where A = $H_2O$/0.1 % TFA and B = ACN/0.1 % TFA, flow rate: 0.3 mL/min, column: Phenomenex Luna 3μ C18 (2) 50 x 2 mm, detection: UV 214 nm, product retention time: 6.54 min). Further product characterisation was carried out using electrospray mass spectrometry ($MH_2^{2+}$ calculated: 1391.5, $MH_2^{2+}$ found: 1392.5).

**Example 3: Synthesis of the Cyanine Dye 2-{(1*E*,3*E*,I5E)-5-[1-(5-carboxvpentvl)-3,3-dimethyl-5-sulfo-1,3-dihydro-2*H*-indol-2-vlidene]penta-1,3-dienyl-3-methyl-1,3-*bis*(4-sulfobutyl)-3*H*-indolium-5-sulfonate (Cy5**)**

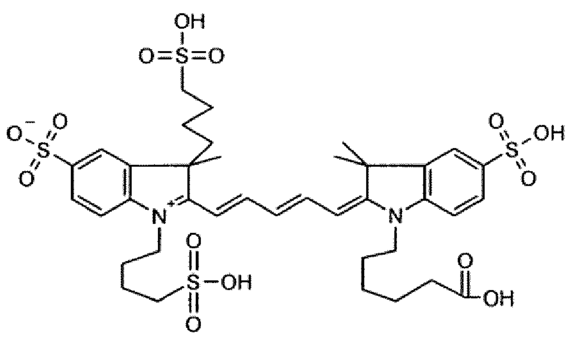

Cy5**

*(3a) 5-Methyl-6-oxoheptane-1-sulfonic acid*

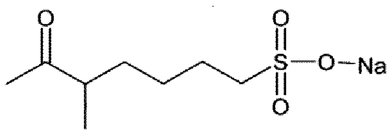

Ethyl 2-methylacetoacetate (50g) in DMF (25ml) was added to a suspension of sodium hydride (12.0g of 60% NaH in mineral oil) in DMF (100ml), dropwise with ice-bath cooling over 1 hour, (internal temperature 0-4 °C). This mixture was allowed to warm to ambient temperature for 45mins with stirring before re-cooling. A solution of 1,4-butanesultone (45g) in DMF (25ml) was then added dropwise over 15 minutes. The final mixture was heated at 60 °C for 18 hours. The solvent was removed by rotary evaporation and the residue partitioned between water and diethyl ether. The aqueous layer was collected, washed with fresh diethyl ether and rotary evaporated to yield a sticky foam. This intermediate was dissolved in water (100ml) and sodium hydroxide (17.8g) added over 15 minutes with stirring. The mixture was heated at 90 °C for 18 hours. The cooled reaction mixture was adjusted to ~pH2 by the addition of concentrated hydrochloric acid (~40ml). The solution was rotary evaporated and dried under vacuum. The yellow solid was washed with ethanol containing 2% hydrochloric acid (3x150ml). The ethanolic solution was filtered, rotary evaporated and dried under vacuum to yield a yellow solid. Yield 70g.

*(3b) 2,3-Dimethyl-3-(4-sulfobutyl)-3H-indole-5-sulfonic acid, dipotassium salt*

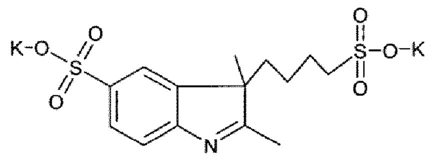

4-Hydrazinobenzenesulfonic acid (40g), 5-methyl-6-oxoheptane-1-sulfonic acid (from 3a; 60g) and acetic acid (500ml) were mixed and heated under reflux for 6 hours. The solvent was filtered, rotary evaporated and dried under vacuum. The solid was dissolved in methanol (1L). To this was added 2M methanolic potassium hydroxide (300ml). The mixture was stirred for 3 hours and then the volume of solvent reduced by 50% using rotary evaporation. The resulting precipitate was filtered, washed with methanol and dried under vacuum. Yield 60g. MS (LCMS) : $MH^+$ 362. Acc. Mass: Found, 362.0729. $MH^+$ = $C_{14}H_2ONO_6S_2$ requires m/z 362.0732 (-0.8ppm).

*(3c) 2,3-Dimethyl-13-bis(4-sulfobutyl)-3H-indolium-5-sulfonate, dipotassium salt*

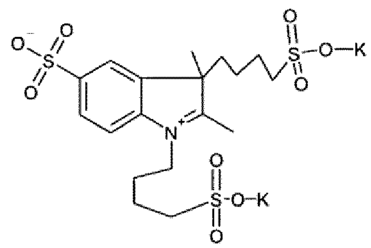

2,3-Dimethyl-3-(4-sulfobutyl)-3*H*-indole-5-sulfonic acid (from 3b; 60g) was heated with 1,4 butane sultone (180g) and tetramethylene sulfone (146ml) at 140 °C for 16 hours. The resulting red solid was washed with diethyl ether, ground into a powder and dried under vacuum. Yield 60g.

*(3d) Cv5**,* as *TFA salt*

1-(5'-Carboxypentyl)-2,3,3-trimethyl-indolenium bromide-5-sulfonic acid, $K^+$ salt (2.7g), malonaldehyde *bis*(phenylimine) monohydrochloride (960mg), acetic anhydride (36ml) and acetic acid (18ml) were heated at 120 °C for 1 hour to give a dark brown-red solution. The reaction mixture was cooled to ambient temperature. 2,3-Dimethyl-1,3-bis(4-sulfobutyl)-3*H*-indolium-5-sulfonate (from 3c; 8.1g) and potassium acetate (4.5g) were added to the mixture, which was stirred for 18 hours at ambient temperature. The resulting blue solution was precipitated using ethyl acetate and dried under vacuum. The crude dye was purified by liquid chromatography ($RPC_{18}$. Water + 0.1% TFA/MeCN + 0.1%TFA gradient). Fractions containing the principal dye peak were collected, pooled and evaporated under vacuum to give the title dye, 2g. UV/Vis (Water + 0.1%TFA): 650nm. MS (MALDI-TOF): $MH^+$ 887.1. $MH^+$ = $C_{38}H_{50}N_2O_{14}S_4$ requires m/z 887.1.

**Example 4: Synthesis of the 2-[(1*E*,3*E*,5*E*)-5-(1-{6-[2,5-dioxopyrrolidin-1-yl)oxy]-6-oxohexyl}-3,3-dimethyl-5-sulfo-1,3-dihydro-2*H*-indol-2-ylidene]penta-1,3-dienyl-3-methyl-1,3-bis(4-sulfobutyl)-3*H*-indolium-5-sulfonate, diisopropylethylamine salt (NHS Ester of Cy5**)**

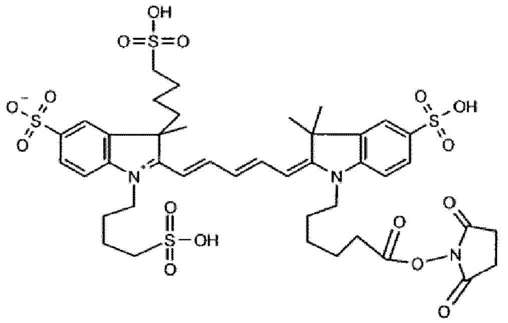

Cy5** (Example 3; 10mg) was dissolved in anhydrous DMSO (3ml); to this were added HSPyU (20mg) and N,N'-diisopropylethylamine (80μl). The resulting solution was mixed for 3 hours, whereupon TLC ($RPC_{18}$. Water/MeCN) revealed complete reaction. The dye was isolated by precipitation in ethyl acetate/diethyl ether, filtered, washed with ethyl acetate and dried under vacuum. UV/Vis (Water) 650nm. MS (MALDI-TOF) $MH^+$ 983.5. $MH^+ = C_{42}H_{53}N_3O_{16}S_4$ requires m/z 984.16.

**Example 5: Conjugation of Dyes, Synthesis of Compounds 3 to 7**

Cys4-16, 6-14; Ac-Ala-Gly-Ser-Cys-Tyr-Cys-Ser-Gly-Pro-Pro-Arg-Phe-Glu-Cys-Trp-Cys-Tyr-Glu-Thr-Glu-Gly-Thr-Gly-Gly-Gly-Lys(Cy5)-$NH_2$ (Compound 3) (comprising SEQ-7).

Compound 1 (10 mg), NMM (4 μL) and Cy5 NHS ester (5.7 mg; GE Healthcare PAI 5104) were dissolved in NMP (1 mL) and the reaction mixture stirred for 7 hours. The reaction mixture was then diluted with 5 % ACN/water (8 mL) and the product purified using preparative HPLC.

Purification by preparative HPLC (gradient: 5-50 % B over 40 min where A = $H_2O$/0.1 % HCOOH and B = ACN/0.1 % HCOOH, flow rate: 10 mL/min, column: Phenomenex Luna 5μ C18 (2) 250 x 21.20 mm, detection: UV 214 nm, product retention time: 35.5 min) of the crude peptide afforded 8.1 mg of pure Compound 3. The pure product was analysed by analytical HPLC (gradient: 5-50 % B over 10 min where A = $H_2O$/0.1% HCOOH and B = ACN/0.1 % HCOOH, flow rate: 0.3 mL/min, column: Phenomenex Luna 3μ C18 (2) 50 × 2 mm, detection: UV 214 nm, product retention time: 8.15 min). Further product characterisation was carried out using electrospray mass spectrometry ($MH_2^{2+}$ calculated: 1710.6, $MH_2^{2+}$ found: 1711.0).

Compound 4 was prepared in a similar manner - electrospray mass spectrometry ($MH_2^{2+}$ calculated: 1710.6, $MH_2^{2+}$ found: 1710.9).

Other dye-peptide conjugates (Compounds 5 to 7) were prepared by analogous methods. Alexa647 was purchased from Molecular Probes (A20106):

Compound 5 ($MH_2^{2+}$ calculated: 1825.7, $MH_2^{2+}$ found: 1825.9),
Compound 6 ($MH_2^{2+}$ calculated: 1811.7, $MH_2^{2+}$ found: 1812.0),
Compound 7 ($MH_2^{2+}$ calculated: 1825.7, $MH_2^{2+}$ found: 1826.2).

**Example 6: *In Vitro* Fluorescence polarisation assay**

The principle of the fluorescence polarisation method can briefly be described as follows:
Monochromatic light passes through a horizontal polarizing filter and excites fluorescent molecules in the sample. Only those molecules that oriented properly in the vertically polarized plane adsorb light, become excited, and subsequently emit light. The emitted light is measured in both horizontal and vertical planes. The anisotropy value (A), is the ratio between the light intensities following the equation:

A = (Intensity with horizontal polarizer - Intensity with vertical polarizer)/(Intensity with horizontal polarizer + 2* Intensity with vertical polarizer).

The fluorescence anisotropy measurements were performed in 384-well microplates in a volume of 10 μL in binding buffer (PBS, 0.01%Tween-20, pH 7.5) using a Tecan Safire fluorescence polarisation plate reader (Tecan , US) at ex646/em678 nm. The concentration of dye-labelled peptide was held constant (20nM) and the concentrations of the

human or mouse cMet/ Fc chimera (R&D Systems) or Semaphorin 6A (R&D Systems) were varied from 0-150 nM. Binding mixtures were equilibrated in the microplate for 10 min at 30 °C. The observed change in anisotropy was fit to the equation:

$$r_{obs} = r_{free} + (r_{bound} - r_{free}) \frac{(K_D + cMet + P) - \sqrt{(K_D + cMet + P)2 - 4 \cdot cMet \cdot P}}{2 \cdot P}$$

where $r_{obs}$ is the observed anisotropy, rfree is the anisotropy of the free peptide, $r_{bound}$ is the anisotropy of the bound peptide, $K_D$ is the dissociation constant, cMet is the total cMet concentration, and P is the total dye-labelled peptide concentration. The equation assumes that the synthetic peptide and the receptor form a reversible complex in solution with 1:1 stoichiometry. Data fitting was done *via* nonlinear regression using GraphPad Prism software to obtain the $K_D$ value (one-site binding).

Compounds 3 and 4 were tested for binding towards human and mouse cMet (Fc chimera). The results showed a $K_D$ of 3 +/- 1 nM for the binding of Compound 3 to human c-Met. There was no binding of Compound 4 to human cMet. Furthermore, Compounds 3 and 4 showed no binding to mouse cMet in the tested range.

Using the same method, Compound 5 was found to have a $K_D$ for human cMet of 1.1 nM.

**Example 7: *In Vivo* testing of Compounds 5 and 7**

*(a) Animal Model*

54 Female BALB c/A nude (Bom) mice were used in the study. The use of the animals was approved by the local ethics committee. BALB c/A nude is an inbred immunocompromised mouse strain with a high take rate for human tumours as compared to other nude mice strains. The mice were 4 weeks old upon arrival and with a body weight of approx. 20 grams at the start of the study. The animals were housed in individually ventilated cages (IVC, Scanbur BK) with HEPA filtered air. The animals had ad *libitum* access to "Rat and Mouse nr. 3 Breeding" diet (Scanbur BK) and tap water acidified by addition of HCl to a molar concentration of 1 mM (pH 3.0).

The colon cancer cell HCT-15 is derived from human colon carcinomas and is reported to express cMet according to Zeng et al [Clin. Exp. Metastasis, 21, 409-417. (2004)]. The cell line was proven to be tumorigenic when inoculated subcutaneously into nude mice [Flatmark et al, Eur. J. Cancer 40,1593-1598 (2004)].

HCT-15 cells were grown and prepared for subcutaneous inoculation in RPMI (Sigma Cat # R0883) with 10% serum and penicillin/streptomycin. Stocks were made at passage number four (P4) and frozen down for storage in liquid nitrogen at $3 \times 10^7$ cells/vial in the culture media containing 5% DMSO. On the day of the transplantation, the cells were thawed quickly in a 37 °C water bath (approx. 2 min), washed and resuspended in PBS/2% serum (centrifugation at 1200 rpm for 10 min). Thorough mixing of cells in the vials was ensured every time cells were aspirated into the dosing syringe. Volumes of 0.1 ml of cell suspension were injected s.c. at the shoulder and at the back using a fine bore needle (25 G) while the animals were under light gas anaesthesia. The animals were then returned to their cages and the tumours were allowed to grow for 13-17 days. The animals were allowed an acclimatisation period of at least 5 days before the inoculation procedure.

*(b) Procedure*

All test substances were reconstituted with PBS from freeze-dried powder. A small stack of white printer paper was imaged to obtain a flat field image which was used to correct for illumination inhomogeneities. The test substances were injected intravenously in the lateral tail vein during physical fixation. The injection volume was 0.1ml, which corresponds to a dose of 1 nmol test substance per animal. After injection the animals were returned to their cages. The animals were sacrificed immediately before imaging by cervical dislocation. The optimal imaging time point for each test substance was estimated based on comparison of wash out rates in the skin and in muscle tissue in a limited number of animals (n=1-6). The imaging time point for Compounds 3 and 4 was 120 minutes post injection. For each animal the subcutaneously grown tumours were excised post mortem. A thin slice, approximately 1.6mm thick and 3-4mm in diameter, was cut off the edge of one of the tumours. The tumour slice was then imaged against an area of normal colon from the same animal.

*(c) Imaging*

Imaging was performed through a clinical laparoscope adapted to use a light source to excite the reporter and a filtering system to extract the fluorescence component. A 635nm laser was used for excitation of the reporter molecule.

A Hamamatsu ORCA ERG CCD camera was used as the detector. The camera was operated in 2x2 binning mode with 0 gain. Standard exposure time for colon imaging was 10s. System calibration measurements indicate that the 10s exposure time with the animal imaging system corresponds to 40ms exposure with a clinically relevant light source, field of view, and distance to the tissue surface. The intensity distribution in the image was corrected for illumination inhomogeneities through system calibration data. A target to background ratio was computed from regions of interest placed over the tumour, and normal colon background. The images were visually scored using the standard scoring system employed for receiver operating characteristic analysis.

*(d) Results*

Compound 5 had a tumour to normal ratio of 1.46:1 and the corresponding scrambled control peptide with the same dye (Compound 7) had a ratio of 1.04:1. Compound 5 had a readily identifiable tumour, whereas nothing was discernible against background with Compound 7.

**Preparation of Lyophilised EMI-137 Formulation**

The formulation needs to freeze dry effectively (e.g., without bumping or collapse) to provide a homogenous solid that is stable. As noted previously, the pH of EMI-137 in water is approximately 4.6, which is unsuitable for intravenous administration. Therefore, further requirements of the formulation are that on reconstitution it is a pH that is suitable for intravenous administration. Furthermore, the tonicity of the formulation on reconstitution must be suitable for intravenous administration. A further benefit would be for the formulation to be storable at room temperature, albeit refrigerated storage would also be acceptable.

Initial formulation studies showed that formulations that had a pH of less than approximately pH 6.8 tended to agglomerate. Therefore, any formulation that provided a pH below 6.8 was deemed unacceptable. Furthermore, it is known at a pH of more than approximately pH 8 (in some instances up to approximately pH 9) there is a risk of degradation of disulphide bridges (of which there are two in EMI-137). Therefore, it would be of benefit to arrive at a formulation that on reconstitution provides a pH that is high enough to avoid agglomeration, but is low enough to mitigate the risk of degradation of disulphide bridges.

Thus, to maintain the pH of the EMI-137 solution within an acceptable range on reconstitution, it is necessary to include a buffer prior to freeze-drying. Studies have shown that the viscosity of the EMI-137 solution increases in the presence of potassium chloride enriched phosphate buffered saline (PBS), and that a substantial gel formation develops. This gelling effect was not observed when EMI-137 was dissolved in sodium phosphate buffer at pH 7.4. Therefore, the use of NaCl and KCl was discounted (due to gelling issues).

The lyophilisation process was carried out as highlighted in Table 3.

Table 3: Lyophilisation Parameters

| Batch No. | FFF169/096-904 | | | | |
|---|---|---|---|---|---|
| Freeze dryer | Lyomax 2 MTE 1500, Development Pharmaceuticals Oslo | | | | |
| Freeze drying | | | | | |
| Phase | Shelf temp. °C | Time Hr:min | Chamber pressure μbar | Temperature ramp rate | Note |
| Loading | -40 | - | - | - | Uses time needed to load freeze dryer |
| Freezing | -55 | 0:10 | - | 1.5 °C/min | Temperature ramp to reach freezing temperature |
| | -55 | 02:05 | - | - | Steady state freezing conditions |
| Primary drying | -55 | 00:45 | 50 | - | Time necessary to prepare freeze dryer for vacuum phase |
| | -38 | 00:15 | 50 | 1 °C/min | Prepare primary drying |
| | -38 | 211:00 | 50 | - | Steady state primary drying conditions |

(continued)

| | | | | | |
|---|---|---|---|---|---|
| Batch No. | FFF169/096-904 | | | | |
| Freeze dryer | Lyomax 2 MTE 1500, Development Pharmaceuticals Oslo | | | | |
| Freeze drying | | | | | |
| Primary to secondary drying transition phase (ramp phase) | 30 | 10:00 | 50 | 6.8 °C/hour | - |
| Secondary drying | 30 | 6:03 | 50 | - | Steady state secondary drying conditions |
| | 20 | 00:10 | 50 | 1 °C/min | Bring chamber to ambient temperature before vial headspace gas is added |
| | 20 | 00:30 | 50 | - | - |
| Storage/unloading | 5 | n/a | n/a | - | Store product at cold temperature during unloading and capping |
| Special functions | | | | | |
| Pre-aeration | Yes | | | | |
| Pressure setpoint | 0.80 bara | | | | |
| Gas | Nitrogen | | | | |
| Stoppering Semi-auto/Auto Stoppering pressure | Semi-Auto 80 BarA | | | | |
| Aeration Air/nitrogen | Air | | | | |
| Vacuum control | Nitrogen bleed | | | | |
| Vacuum gauge | MKS | | | | |
| Pressure deviation | 20 μbar | | | | |
| Product protection | 80 μbar | | | | |
| Moisture test | No | | | | |
| Nominal time | 230:58 | (Real 239: 29 including loading) | | | |

The lyophilisation was carried out as follows.

The constituents of the formulations as highlighted in Tables 4A to 4F below were added to a suitable lyophilisation vessel. Note that all components listed (including the buffer) were added prior to lyophilisation.

The loading phase is carried out at approximately -40 °C. The temperature is then reduced to approximately -55 °C. The rate of cooling may be approximately 1.5 °C per minute. The mixture is then held at steady state freezing conditions for at least two hours. The pressure is then reduced to 50 μBar, which takes approximately 45 minutes. The temperature is then increased to approximately -38 °C for the primary drying phase. The rate of heating may be approximately 1 °C per minute. The mixture is then held at steady state freezing conditions for approximately nine days. The mixture is then heated to approximately 30 °C at a rate of approximately 6.8 °C per hour over at least a ten-hour period (primary to secondary drying transition phase, or "ramp phase"). The mixture is then held at steady state secondary drying conditions for approximately six hours. The mixture is then cooled to approximately 20 °C at a rate of approximately 1 °C per minute, before addition of headspace gas (i.e., and inert gas such as argon or nitrogen), thus releasing the vacuum in the lyophilisation vessel. The product is then unloaded/stored at 5 °C.

The primary drying step, the ramp phase and the secondary drying step are all carried out by placing the mixture under vacuum of approximately 50 μbar. This pressure may be introduced in the primary drying phase and then maintained until the storage/unloading phase.

The use of a buffer such as sodium phosphate is known to cause a pH shift towards low pH during freezing and thus the formulation was expected to be challenging to freeze dry without collapse, as the glass transition temperatures of the freeze concentrates would be low. As such, the current invention includes a combined tonicity regulator and lyoprotectant. Mannitol is one of the most widely used and documented bulking agents in freeze drying, but several thermal issues and crystallization complications have been reported, which could potentially affect production and product stability. As such, the use of mannitol alone is not normally thought to be advantageous on lyophilisation and is known to give rise to several problems.

As the combination of buffer and lyoprotectants in the present invention was difficult to freeze-dry without experiencing collapse of the freeze-dried cake, a tailored freeze-drying cycle was required. The freeze-drying cycle was selected to have sufficiently low vacuum and shelf temperature to achieve a low ice temperature to prevent this melt-back / collapse of the freeze-dried cake. The collapse of the freeze-dried cake must be avoided to prevent EMI-137 from forming larger structures/agglomerates during the drying step of the freeze-drying process (this is found to happen at pH less than 6.8 and is not entirely reversible).

After some initial formulation studies, and in view of the above, the following variables were chosen for further testing:

- Buffer: sodium phosphate or Tris HCl (25, 40 and 55 mM);
- Lyoprotectant: sucrose or mannitol.

## Examples of Lyophilised Formulations

The formulation candidates were investigated using a factorial design with four variables combined with a mixed level design as shown in Tables 4A to 4F. The design was made up of a fractional two-level design with four variables (samples 1 to 8), four centerpoints (samples 10 to 13), and a mixed level design with three levels for the active pharmaceutical ingredient (API) and two levels for the categorical variables of buffer type and lyoprotectant type (samples 14 to 21). In addition, various reference samples were included (samples 22 to 24). Two additional samples (samples 25 and 26) were also studied. Sample 25 refers to the composition of the solution before freeze drying in 1 mL of water, and sample 26 refers to a further typical formulation that was prepared based on the results of the previous samples.

Table 4A: Formulation Screening Design (mg/ml)

| Sample | Water (ml) | Buffer type | Buffer conc. | Cryo type | API conc. (mg/ml) | Cryo conc. (mg/ml) | Buffer conc. (mg/ml) |
|---|---|---|---|---|---|---|---|
| 1 | 5 | Tris HCl | 25mM | Mannitol | 4.3 | 46.2 | 3.03 |
| 2 | 5 | Na Phosphate (1) | 25mM | Mannitol | 5.3 | 40.76 | 3.5 |
| 3 | 5 | Tris HCl | 55mM | Mannitol | 5.3 | 40.79 | 6.66 |
| 4 | 5 | Na Phosphate (2) | 55mM | Mannitol | 4.3 | 30.17 | 7.7 |
| 5 | 5 | Tris HCl | 25mM | Sucrose | 5.3 | 84.29 | 3.03 |
| 6 | 5 | Na Phosphate (1) | 25mM | Sucrose | 4.3 | 74.36 | 3.5 |
| 7 | 5 | Tris HCl | 55mM | Sucrose | 4.3 | 74.43 | 6.66 |
| 8 | 5 | Na Phosphate (2) | 55mM | Sucrose | 5.3 | 52.59 | 7.7 |
| 10 | 5 | Tris HCl | 40mM | Mannitol | 4.8 | 43.5 | 4.85 |
| 11 | 5 | Na Phosphate (3) | 40mM | Mannitol | 4.8 | 34.8 | 5.6 |
| 12 | 5 | Tris HCl | 40mM | Sucrose | 4.8 | 79.36 | 4.85 |
| 13 | 5 | Na Phosphate (3) | 40mM | Sucrose | 4.8 | 65.25 | 5.6 |
| 14 | 5 | Tris HCl | 40mM | Mannitol | 4.3 | 43.5 | 4.85 |

(continued)

| Sample | Water (ml) | Buffer type | Buffer conc. | Cryo type | API conc. (mg/ml) | Cryo conc. (mg/ml) | Buffer conc. (mg/ml) |
|---|---|---|---|---|---|---|---|
| 15 | 5 | Tris HCl | 40mM | Mannitol | 5.3 | 43.5 | 4.85 |
| 16 | 5 | Tris HCl | 40mM | Sucrose | 4.3 | 79.36 | 4.85 |
| 17 | 5 | Tris HCl | 40mM | Sucrose | 4.3 | 79.36 | 4.85 |
| 18 | 5 | Na Phosphate (3) | 40mM | Mannitol | 4.3 | 34.8 | 5.6 |
| 19 | 5 | Na Phosphate (3) | 40mM | Mannitol | 5.3 | 34.8 | 5.6 |
| 20 | 5 | Na Phosphate (3) | 40mM | Sucrose | 4.3 | 65.25 | 5.6 |
| 21 | 5 | Na Phosphate (3) | 40mM | Sucrose | 4.3 | 65.25 | 5.6 |
| 22 | 5 | Tris HCl | 40mM | None | 4.8 | 0 | 4.85 |
| 23 | 5 | Na Phosphate (3) | 40mM | None | 4.8 | 0 | 5.6 |
| 24 | 5 | None | 0 | None | 4.8 | 0 | 0 |
| 25* | 1 | Na Phosphate (4) | 50mM | Mannitol | 4.80 | 30.82 | 7.00 |
| 26** | 5 | Na Phosphate (4) | 50mM | Mannitol | 4.80 | 30.82 | 7.00 |

Table 4B: Formulation Screening Design (mg)

| Sample | Water (ml) | Buffer type | Cryo type | Total API (mg) | Total Cryo (mg) | Total Anhydrous Buffer (mg) | Total Hydrated Buffer (mg) |
|---|---|---|---|---|---|---|---|
| 1 | 5 | Tris HCl | Mannitol | 21.5 | 231 | 15.15 | 15.15 |
| 2 | 5 | Na Phosphate (1) | Mannitol | 26.5 | 203.8 | 17.5 | 42.61 |
| 3 | 5 | Tris HCl | Mannitol | 26.5 | 203.95 | 33.3 | 33.30 |
| 4 | 5 | Na Phosphate (2) | Mannitol | 21.5 | 150.85 | 38.5 | 93.72 |
| 5 | 5 | Tris HCl | Sucrose | 26.5 | 421.45 | 15.15 | 15.15 |
| 6 | 5 | Na Phosphate (1) | Sucrose | 21.5 | 371.8 | 17.5 | 42.61 |
| 7 | 5 | Tris HCl | Sucrose | 21.5 | 372.15 | 33.3 | 33.30 |
| 8 | 5 | Na Phosphate (2) | Sucrose | 26.5 | 262.95 | 38.5 | 93.72 |
| 10 | 5 | Tris HCl | Mannitol | 24 | 217.5 | 24.25 | 24.25 |

(continued)

| Sample | Water (ml) | Buffer type | Cryo type | Total API (mg) | Total Cryo (mg) | Total Anhydrous Buffer (mg) | Total Hydrated Buffer (mg) |
|---|---|---|---|---|---|---|---|
| 11 | 5 | Na Phosphate (3) | Mannitol | 24 | 174 | 28 | 68.14 |
| 12 | 5 | Tris HCl | Sucrose | 24 | 396.8 | 24.25 | 24.25 |
| 13 | 5 | Na Phosphate (3) | Sucrose | 24 | 326.25 | 28 | 68.14 |
| 14 | 5 | Tris HCl | Mannitol | 21.5 | 217.5 | 24.25 | 24.25 |
| 15 | 5 | Tris HCl | Mannitol | 26.5 | 217.5 | 24.25 | 24.25 |
| 16 | 5 | Tris HCl | Sucrose | 21.5 | 396.8 | 24.25 | 24.25 |
| 17 | 5 | Tris HCl | Sucrose | 21.5 | 396.8 | 24.25 | 24.25 |
| 18 | 5 | Na Phosphate (3) | Mannitol | 21.5 | 174 | 28 | 68.14 |
| 19 | 5 | Na Phosphate (3) | Mannitol | 26.5 | 174 | 28 | 68.14 |
| 20 | 5 | Na Phosphate (3) | Sucrose | 21.5 | 326.25 | 28 | 68.14 |
| 21 | 5 | Na Phosphate (3) | Sucrose | 21.5 | 326.25 | 28 | 68.14 |
| 22 | 5 | Tris HCl | None | 24 | 0 | 24.25 | 24.25 |
| 23 | 5 | Na Phosphate (3) | None | 24 | 0 | 28 | 68.14 |
| 24 | 5 | None | None | 24 | 0 | 0 | 0.00 |
| 25* | 1 | Na Phosphate (4) | Mannitol | 24.00 | 154.10 | 35.02 | 85.24 |
| 26** | 5 | Na Phosphate (4) | Mannitol | 24.00 | 154.10 | 35.02 | 85.24 |

Table 4C: Formulation Screening Design (mmol)

| Sample | Water (ml) | Buffer type | Cryo type | API (mmol) | Cryo (mmol) | Buffer (mmol) |
|---|---|---|---|---|---|---|
| 1 | 5 | Tris HCl | Mannitol | 0.01 | 1.27 | 0.13 |
| 2 | 5 | Na Phosphate (1) | Mannitol | 0.01 | 1.12 | 0.12 |
| 3 | 5 | Tris HCl | Mannitol | 0.01 | 1.12 | 0.27 |
| 4 | 5 | Na Phosphate (2) | Mannitol | 0.01 | 0.83 | 0.27 |
| 5 | 5 | Tris HCl | Sucrose | 0.01 | 1.23 | 0.13 |

(continued)

| Sample | Water (ml) | Buffer type | Cryo type | API (mmol) | Cryo (mmol) | Buffer (mmol) |
|---|---|---|---|---|---|---|
| 6 | 5 | Na Phosphate (1) | Sucrose | 0.01 | 1.09 | 0.12 |
| 7 | 5 | Tris HCl | Sucrose | 0.01 | 1.09 | 0.27 |
| 8 | 5 | Na Phosphate (2) | Sucrose | 0.01 | 0.77 | 0.27 |
| 10 | 5 | Tris HCl | Mannitol | 0.01 | 1.19 | 0.20 |
| 11 | 5 | Na Phosphate (3) | Mannitol | 0.01 | 0.96 | 0.20 |
| 12 | 5 | Tris HCl | Sucrose | 0.01 | 1.16 | 0.20 |
| 13 | 5 | Na Phosphate (3) | Sucrose | 0.01 | 0.95 | 0.20 |
| 14 | 5 | Tris HCl | Mannitol | 0.01 | 1.19 | 0.20 |
| 15 | 5 | Tris HCl | Mannitol | 0.01 | 1.19 | 0.20 |
| 16 | 5 | Tris HCl | Sucrose | 0.01 | 1.16 | 0.20 |
| 17 | 5 | Tris HCl | Sucrose | 0.01 | 1.16 | 0.20 |
| 18 | 5 | Na Phosphate (3) | Mannitol | 0.01 | 0.96 | 0.20 |
| 19 | 5 | Na Phosphate (3) | Mannitol | 0.01 | 0.96 | 0.20 |
| 20 | 5 | Na Phosphate (3) | Sucrose | 0.01 | 0.95 | 0.20 |
| 21 | 5 | Na Phosphate (3) | Sucrose | 0.01 | 0.95 | 0.20 |
| 22 | 5 | Tris HCl | None | 0.01 | 0.00 | 0.20 |
| 23 | 5 | Na Phosphate (3) | None | 0.01 | 0.00 | 0.20 |
| 24 | 5 | None | None | 0.01 | 0.00 | 0.00 |
| 25* | 1 | Na Phosphate (4) | Mannitol | 0.00 | 0.17 | 0.05 |
| 26** | 5 | Na Phosphate (4) | Mannitol | 0.01 | 0.85 | 0.25 |

Table 4D: Formulation Screening Design (mole ratio)

| Sample | Water (ml) | Buffer type | Cryo type | API (mole ratio) | Cryo (mole ratio) | Buffer (mole ratio) |
|---|---|---|---|---|---|---|
| 1 | 5 | Tris HCl | Mannitol | 1.00 | 215.29 | 21.23 |
| 2 | 5 | Na Phosphate (1) | Mannitol | 1.00 | 154.10 | 17.20 |
| 3 | 5 | Tris HCl | Mannitol | 1.00 | 154.22 | 37.87 |
| 4 | 5 | Na Phosphate (2) | Mannitol | 1.00 | 140.59 | 46.66 |
| 5 | 5 | Tris HCl | Sucrose | 1.00 | 169.60 | 17.23 |
| 6 | 5 | Na Phosphate (1) | Sucrose | 1.00 | 184.42 | 21.20 |
| 7 | 5 | Tris HCl | Sucrose | 1.00 | 184.59 | 46.67 |
| 8 | 5 | Na Phosphate (2) | Sucrose | 1.00 | 105.82 | 37.86 |
| 10 | 5 | Tris HCl | Mannitol | 1.00 | 181.59 | 30.45 |
| 11 | 5 | Na Phosphate (3) | Mannitol | 1.00 | 145.27 | 30.40 |
| 12 | 5 | Tris HCl | Sucrose | 1.00 | 176.32 | 30.45 |
| 13 | 5 | Na Phosphate (3) | Sucrose | 1.00 | 144.97 | 30.40 |
| 14 | 5 | Tris HCl | Mannitol | 1.00 | 202.71 | 33.99 |
| 15 | 5 | Tris HCl | Mannitol | 1.00 | 164.46 | 27.57 |

(continued)

| Sample | Water (ml) | Buffer type | Cryo type | API (mole ratio) | Cryo (mole ratio) | Buffer (mole ratio) |
|---|---|---|---|---|---|---|
| 16 | 5 | Tris HCl | Sucrose | 1.00 | 196.82 | 33.99 |
| 17 | 5 | Tris HCl | Sucrose | 1.00 | 196.82 | 33.99 |
| 18 | 5 | Na Phosphate (3) | Mannitol | 1.00 | 162.17 | 33.94 |
| 19 | 5 | Na Phosphate (3) | Mannitol | 1.00 | 131.57 | 27.54 |
| 20 | 5 | Na Phosphate (3) | Sucrose | 1.00 | 161.83 | 33.94 |
| 21 | 5 | Na Phosphate (3) | Sucrose | 1.00 | 161.83 | 33.94 |
| 22 | 5 | Tris HCl | None | 1.00 | 0.00 | 30.45 |
| 23 | 5 | Na Phosphate (3) | None | 1.00 | 0.00 | 30.40 |
| 24 | 5 | None | None | 1.00 | 0.00 | 0.00 |
| 25* | 1 | Na Phosphate (4) | Mannitol | 1.00 | 128.66 | 38.02 |
| 26** | 5 | Na Phosphate (4) | Mannitol | 1.00 | 128.66 | 38.02 |

Table 4E: Formulation Screening Design (%w/w before freeze drying)

| Sample | Water (ml) | Buffer type | Cryo type | Total Mass of Compone nts (mg) - before freeze drying | API (% w/w) | Cryo (% w/w) | Buffer (% w/w) |
|---|---|---|---|---|---|---|---|
| 1 | 5 | Tris HCl | Mannitol | 267.65 | 8.03 | 86.31 | 5.66 |
| 2 | 5 | Na Phosphate (1) | Mannitol | 247.80 | 10.69 | 82.24 | 7.06 |
| 3 | 5 | Tris HCl | Mannitol | 263.75 | 10.05 | 77.33 | 12.63 |
| 4 | 5 | Na Phosphate (2) | Mannitol | 210.85 | 10.20 | 71.54 | 18.26 |
| 5 | 5 | Tris HCl | Sucrose | 463.10 | 5.72 | 91.01 | 3.27 |
| 6 | 5 | Na Phosphate (1) | Sucrose | 410.80 | 5.23 | 90.51 | 4.26 |
| 7 | 5 | Tris HCl | Sucrose | 426.95 | 5.04 | 87.16 | 7.80 |
| 8 | 5 | Na Phosphate (2) | Sucrose | 327.95 | 8.08 | 80.18 | 11.74 |
| 10 | 5 | Tris HCl | Mannitol | 265.75 | 9.03 | 81.84 | 9.13 |
| 11 | 5 | Na Phosphate (3) | Mannitol | 226.00 | 10.62 | 76.99 | 12.39 |
| 12 | 5 | Tris HCl | Sucrose | 445.05 | 5.39 | 89.16 | 5.45 |
| 13 | 5 | Na Phosphate (3) | Sucrose | 378.25 | 6.35 | 86.25 | 7.40 |
| 14 | 5 | Tris HCl | Mannitol | 263.25 | 8.17 | 82.62 | 9.21 |
| 15 | 5 | Tris HCl | Mannitol | 268.25 | 9.88 | 81.08 | 9.04 |

(continued)

| Sample | Water (ml) | Buffer type | Cryo type | Total Mass of Compone nts (mg) - before freeze drying | API (% w/w) | Cryo (% w/w) | Buffer (% w/w) |
|---|---|---|---|---|---|---|---|
| 16 | 5 | Tris HCl | Sucrose | 442.55 | 4.86 | 89.66 | 5.48 |
| 17 | 5 | Tris HCl | Sucrose | 442.55 | 4.86 | 89.66 | 5.48 |
| 18 | 5 | Na Phosphate (3) | Mannitol | 223.50 | 9.62 | 77.85 | 12.53 |
| 19 | 5 | Na Phosphate (3) | Mannitol | 228.50 | 11.60 | 76.15 | 12.25 |
| 20 | 5 | Na Phosphate (3) | Sucrose | 375.75 | 5.72 | 86.83 | 7.45 |
| 21 | 5 | Na Phosphate (3) | Sucrose | 375.75 | 5.72 | 86.83 | 7.45 |
| 22 | 5 | Tris HCl | None | 48.25 | 49.74 | 0.00 | 50.26 |
| 23 | 5 | Na Phosphate (3) | None | 52.00 | 46.15 | 0.00 | 53.85 |
| 24 | 5 | None | None | 24.00 | 100.00 | 0.00 | 0.00 |
| 25* | 1 | Na Phosphate (4) | Mannitol | 213.12 | 11.26 | 72.31 | 16.43 |
| 26** | 5 | Na Phosphate (4) | Mannitol | --- | --- | --- | --- |

Table 4F: Formulation Screening Design (%w/w after freeze drying)

| Sample | Water (ml) | Buffer type | Cryo type | Total Mass of Components (mg) - per freeze dried vial | API (% w/w) | Cryo (% w/w) | Buffer (% w/w) |
|---|---|---|---|---|---|---|---|
| 1 | 5 | Tris HCl | Mannitol | 267.65 | 8.03 | 86.31 | 5.66 |
| 2 | 5 | Na Phosphate (1) | Mannitol | 272.91 | 9.71 | 74.68 | 15.61 |
| 3 | 5 | Tris HCl | Mannitol | 263.75 | 10.05 | 77.33 | 12.63 |
| 4 | 5 | Na Phosphate (2) | Mannitol | 266.07 | 8.08 | 56.70 | 35.22 |
| 5 | 5 | Tris HCl | Sucrose | 463.10 | 5.72 | 91.01 | 3.27 |
| 6 | 5 | Na Phosphate (1) | Sucrose | 435.91 | 4.93 | 85.29 | 9.78 |
| 7 | 5 | Tris HCl | Sucrose | 426.95 | 5.04 | 87.16 | 7.80 |
| 8 | 5 | Na Phosphate (2) | Sucrose | 383.17 | 6.92 | 68.63 | 24.46 |
| 10 | 5 | Tris HCl | Mannitol | 265.75 | 9.03 | 81.84 | 9.13 |

(continued)

| Sample | Water (ml) | Buffer type | Cryo type | Total Mass of Components (mg) - per freeze dried vial | API (% w/w) | Cryo (% w/w) | Buffer (% w/w) |
|---|---|---|---|---|---|---|---|
| 11 | 5 | Na Phosphate (3) | Mannitol | 266.14 | 9.02 | 65.38 | 25.60 |
| 12 | 5 | Tris HCl | Sucrose | 445.05 | 5.39 | 89.16 | 5.45 |
| 13 | 5 | Na Phosphate (3) | Sucrose | 418.39 | 5.74 | 77.98 | 16.29 |
| 14 | 5 | Tris HCl | Mannitol | 263.25 | 8.17 | 82.62 | 9.21 |
| 15 | 5 | Tris HCl | Mannitol | 268.25 | 9.88 | 81.08 | 9.04 |
| 16 | 5 | Tris HCl | Sucrose | 442.55 | 4.86 | 89.66 | 5.48 |
| 17 | 5 | Tris HCl | Sucrose | 442.55 | 4.86 | 89.66 | 5.48 |
| 18 | 5 | Na Phosphate (3) | Mannitol | 263.64 | 8.16 | 66.00 | 25.84 |
| 19 | 5 | Na Phosphate (3) | Mannitol | 268.64 | 9.86 | 64.77 | 25.36 |
| 20 | 5 | Na Phosphate (3) | Sucrose | 415.89 | 5.17 | 78.45 | 16.38 |
| 21 | 5 | Na Phosphate (3) | Sucrose | 415.89 | 5.17 | 78.45 | 16.38 |
| 22 | 5 | Tris HCl | None | 48.25 | 49.74 | 0.00 | 50.26 |
| 23 | 5 | Na Phosphate (3) | None | 92.14 | 26.05 | 0.00 | 73.95 |
| 24 | 5 | None | None | 24.00 | 100.00 | 0.00 | 0.00 |
| 25* | 1 | Na Phosphate (4) | Mannitol | --- | --- | --- | --- |
| 26** | 5 | Na Phosphate (4) | Mannitol | 263.34 | 9.11 | 58.52 | 32.37 |

Reference to (1), (2), (3) and (4) in Tables 4A to 4F above are as follows:

(1) 0.25 mg/mL anhydr $NaH_2PO_4$ + 3.25 mg/mL anhydr $Na_2HPO_4$

(2) 0.56 mg/mL anhydr $NaH_2PO_4$ + 7.14 mg/mL anhydr $Na_2HPO_4$

(3) 0.41 mg/mL anhydr $NaH_2PO_4$ + 5.19 mg/mL anhydr $Na_2HPO_4$

(4) 0.50877 mg/mL anhydr $NaH_2PO_4$ + 6.49467 mg/mL anhydr $Na_2HPO_4$

The amounts of anhydrous and hydrated sodium phosphate used are illustrated below in Table 5.

| | $NaH_2PO_4$ (mmol/m L) | $Na_2HPO_4$ (mmol/m L) | Total Buffer (mmol/ mL) | Total Buffer (mmol) | $NaH_2PO_4$ $2H_2O$ (mmol) | $NaH_2PO_4$ $2H_2O$ (mg) | $Na_2HPO_4$ $12H_2O$ (mmol) | $Na_2HPO_4$ $12H_2O$ (mg) | Total Buffer (mg) |
|---|---|---|---|---|---|---|---|---|---|
| (1) | 0.00208 | 0.02289 | 0.0249 7 | 0.1248 5 | 0.0104 | 1.62 | 0.11445 | 40.99 | 42.61 |
| (2) | 0.00467 | 0.05030 | 0.0549 7 | 0.2748 5 | 0.02335 | 3.64 | 0.2515 | 90.07 | 93.72 |
| (3) | 0.00342 | 0.03656 | 0.0399 8 | 0.1999 0 | 0.0171 | 2.67 | 0.1828 | 65.47 | 68.14 |
| (4) | 0.00425 | 0.04575 | 0.0500 0 | 0.0500 0 | 0.02125 | 3.32 | 0.22875 | 81.92 | 85.24 |

Table 5: Equivalent Amount of Hydrated Sodium Phosphate

The molar masses used in the formulations were as follows:

- Sodium dihydrogen phosphate (anhydr) = 119.98 g/mol
- Disodium hydrogen phosphate (anhydr) = 141.96 g/mol
- Tris HCl = 121.14 g/mol
- Mannitol = 182.17 g/mol
- Sucrose = 342.29 g/mol
- EMI-137 = 3,650.3 g/mol

From the above, it can be seen that the sodium phosphate buffer range = 4.26 - 18.26 % w/w before freeze drying and 9.78 - 35.22 % w/w after freeze drying, whereas the Tris HCL buffer range = 3.27 - 12.63 % w/w before freeze drying and 3.27 - 12.63 % w/w after freeze drying.

The materials used in the formulations are listed in Table 6 below.

Table 6: Materials Used in the Formulations for Screening

| Material | Vendor / article no. |
|---|---|
| $Na_2HPO_4$ 12 $H_2O$ | Merck 1.06573 |
| $NaH_2PO_4$ 2 $H_2O$ | Merck 1.06345 |
| Mannitol | Merck 1.05980 |
| Sucrose | Merck 1.07653 |
| Tris(hydroxymerthyl)aminomethane | Merck 1.08386 |
| Water for injection | GEHC in-house bulk WFI |
| EMI-137 ammonium acetate | GEHC in-house |

The screening study was followed by a 4 week pre-stability study at 25°C and 40°C. The set of experiments was designed to achieve both screening of selected buffers and lyoprotectants, and to test robustness around production relevant API concentrations (target 4.8 mg/ml). A ±10% range around the target concentration was studied. The design would also uncover potential interactions between EMI-137, buffer and lyoprotectants. As all of the samples were made isotonic, the buffer concentration and lyoprotectant concentration were covariables. The test parameters are shown in Table 7. In addition to in-process testing of the bulk solution before freeze drying, the testing also included samples from the freezing step to uncover whether a potential instability could be ascribed to the process of freezing or drying.

Table 7: Testing Parameters

| Parameters | Bulk solution | Sample from freezing step, not dried | Freeze dried |
|---|---|---|---|
| Visual appearance of the freeze dried cake | n.a. | n.a. | X |
| Reconstitution | n.a. | n.a. | X |
| pH | X | n.a. | X |
| HPLC purity | X | X | X |
| Agglomeration as measured by PCS | X | X | X |
| n.a. = not applicable | | | |

**Lyophilised Formulation Results**

Stability data for up to 102 months at 2 to 8°C, 61 months at 30°C and 6 months at 40°C is illustrated in Tables 8A to 8C, 9A to 9C and 10 respectively (the following key/abbreviations are used: Amb = ambient humidity, not regulated; NMT = not more than; ND = not detected; ---: not performed; *24 months sampling point postponed due to summer vacation; **additional sampling point).

Table 8A: Stability of EMI-137 Formulation Stored at 5 °C for 102 Months

| DETERMINATION | | SPECIFICATION | SAMPLING POINT (MONTHS) | | | |
|---|---|---|---|---|---|---|
| | | | 0 | | 6 | 12 |
| | | | Jul-09 | | Jan-10 | Jul-10 |
| Appearance of dry product | | Heterogeneous blue lyophilisate | Blue powder cake or | | Blue cake or powder | Blue cake or powder |
| Appearance of reconstituted product | | Clear, dark blue solution, practically free from visible particles | Clear, dark blue solution, practically free from particles | | Clear, dark blue solution, practically free from particles | Clear, dark blue solution, practically free from particles |
| Identification by HPLC, retention time | | Conforms to reference | Conforms to reference | | Conforms to reference | Conforms to reference |
| Content of EMI-137 | | 20.0-26.5 mg/vial | 24.0, 24.7 | | --- | --- |
| pH | | 6.8 to 8.0 | 7.5, 7.5, 7.5 | | 7.5 | 7.5 |
| Osmolality | | 250-330 mOsm/kg | 304, 278, 278 | | --- | 291 |
| Related substances by HPLC | Each single related substance | Report value, x.xx% area | RRT 0.27 | 0.24 | --- | --- |
| | | | RRT 0.86 | 0.12 | --- | --- |
| | | | RRT 0.92 | 0.11 | --- | --- |
| | | | RRT 0.94 | 0.14 | --- | --- |
| | | | RRT 1.08 | 0.25 | --- | --- |
| | | | RRT 1.15 | 0.22 | --- | --- |
| | | | RRT 1.17 | 0.15 | --- | --- |
| | | | RRT 1.20 | 0.14 | --- | --- |
| | | | RRT 1.69 | 0.71 | --- | --- |
| | | | RRT 1.70 | 0.12 | --- | --- |
| | Sum of related substances | NMT 8.00% area | 2.19 | | 2.15, 2.16 | 1.74, 1.72 |
| Oxygen in head space | | Report value x.x μl/vial | 26.5, 10.7, 54.7, 13.0, 2.9, ND or NMT 2.0 | | --- | --- |
| Water | | NMT 5.0% m/m | 2 | | 1.4, 1.5, 1.3 | 1.7, 1.8, 2.0 |
| Bacterial Endotoxins | | NMT 110 EU/vial | NMT 0.5 | | --- | --- |
| Sterility | | Passes Ph.Eur./USP | Passes Ph.Eur./USP | | --- | --- |
| Particulate contamination | ≥10um | NMT 6000 particles/container | NOT IN SPECS FOR PRECLINICAL | | --- | --- |
| | ≥25 um | NMT 600 particles/container | NOT IN SPECS FOR PRECLINICAL | | --- | --- |

(continued)

| DETERMINATION | SPECIFICATION | SAMPLING POINT (MONTHS) | | | |
|---|---|---|---|---|---|
| | | 0 | | 6 | 12 |
| | | Jul-09 | | Jan-10 | Jul-10 |
| | Uniformity of mass of single- dose preparation | Passes Ph. Eur./USP | --- | --- | --- |
| | Diastereomers, ratio by HPLC | Report value, x.xx | 1.01, 1.01 | 1.01, 1.01 | 1.01, 1.01 |
| | Stopper | --- | --- | --- | --- |

Table 8B: Stability of EMI-137 Formulation Stored at 5 °C for 102 Months

| DETERMINATION | | SPECIFICATION | SAMPLING POINT (MONTHS) | | | |
|---|---|---|---|---|---|---|
| | | | 25* | 39** | 61** | |
| | | | Aug-11 | Oct-12 | Aug-14 | |
| Appearance of dry product | | Heterogeneous blue lyophilisate | Blue cake or powder | Heterogeneous blue lyophilisate | Heterogeneous blue lyophilisate | |
| Appearance of reconstituted product | | Clear, dark blue solution, practically free from visible particles | Clear, dark blue solution, practically free from particles | Clear, dark blue solution, practically free from particles | Clear, dark blue solution, practically free from particles | |
| Identification by HPLC, retention time | | Conforms to reference | Conforms to reference | Conforms to reference | --- | |
| Content of EMI-137 | | 20.0-26.5 mg/vial | 22.9, 23.8 | 23.9, 23.9 | 22.1, 23.2 | |
| pH | | 6.8 to 8.0 | 7.5 | 7.6 | 7.4 | |
| Osmolality | | 250-330 mOsm/kg | --- | --- | --- | |
| Related substances by HPLC | Each single related substance | Report value, x.xx% area | --- | --- | RRT 0.27 | 0.24 |
| | | | --- | --- | --- | --- |
| | | | --- | --- | --- | --- |
| | | | --- | --- | RRT 0.94 | 0.13 |
| | | | --- | --- | RRT 1.08 | 0.21 |
| | | | --- | --- | RRT 1.15 | 0.19 |
| | | | --- | --- | RRT 1.17 | 0.13 |
| | | | --- | --- | RRT 1.20 | 0.13 |
| | | | --- | --- | RRT 1.67 | 0.71 |
| | | | --- | --- | --- --- | |
| | Sum of related substances | NMT 8.00% area | 1.90, 1.89 | 1.86, 1.84 | 1.74 | |
| Oxygen in head space | | Report value x.x µl/ vial | 58.6, 62.4, 48.6 | 76.8, 71.0, 73.9 | ---, 150.4, 138.7 | |

(continued)

| DETERMINATION | | SPECIFICATION | SAMPLING POINT (MONTHS) | | |
|---|---|---|---|---|---|
| | | | 25* | 39** | 61** |
| | | | Aug-11 | Oct-12 | Aug-14 |
| Water | | NMT 5.0% m/m | 1.7, 1.7, 1.8 | 1.2, 1.4, 1.6 | 1.5, 1.5, 1.6 |
| Bacterial Endotoxins | | NMT 110 EU/vial | --- | --- | --- |
| Sterility | | Passes Ph.Eur./USP | --- | --- | --- |
| Particulate contamination | ≥10um | NMT 6000 particles/container | --- | --- | --- |
| | ≥25 um | NMT 600 particles/ container | --- | --- | --- |
| Uniformity of mass of single-dose preparation | | Passes Ph. Eur./USP | --- | --- | --- |
| Diastereomers, ratio by HPLC | | Report value, x.xx | 1.01,1.01 | --- | --- |
| Stopper | | --- | --- | --- | Passes Fragmentation and Penetrability according to Ph. Eur 1.5 years after expiry date (5 years) |

Table 8C: Stability of EMI-137 Formulation Stored at 5 °C for 102 Months

| DETERMINATION | | SPECIFICATION | SAMPLING POINT (MONTHS) | | |
|---|---|---|---|---|---|
| | | | 91 | 95 | 102 |
| | | | Feb-17 | Jun-17 | Jan-18 |
| Appearance of dry product | | Heterogeneous blue lyophilisate | Complies | - | Complies |
| Appearance of reconstituted product | | Clear, dark blue solution, practically free from visible particles | Complies | - | Complies |
| Identification by HPLC, retention time | | Conforms to reference | Complies | - | Complies |
| Content of EMI-137 | | 20.0-26.5 mg/vial | 23 | - | 22.7 |
| pH | | 6.8 to 8.0 | 7.5 | - | 7.6 |
| Osmolality | | 250-330 mOsm/kg | 299 | - | 292 |
| Related substances by HPLC | Each single related substance | Report value, x.xx% area | | | |
| | Sum of related substances | NMT 8.00% area | 0.41 | - | 2.01 |
| Oxygen in head space | | Report value x.x µl/vial | --- | - | - |
| Water | | NMT 5.0% m/m | 1.1 | - | 0.9 |
| Bacterial Endotoxins | | NMT 110 EU/vial | - | Passes | Passes |
| Sterility | | Passes Ph.Eur./USP | - | Passes | Passes |

(continued)

| DETERMINATION | | SPECIFICATION | SAMPLING POINT (MONTHS) | | |
|---|---|---|---|---|---|
| | | | 91 | 95 | 102 |
| | | | Feb-17 | Jun-17 | Jan-18 |
| Particulate contamination | ≥10um | NMT 6000 particles/container | Passes | - | Passes |
| | ≥25 um | NMT 600 particles/container | Passes | --- | Passes |
| Uniformity of mass of single-dose preparation | | Passes Ph. Eur./USP | Complies | - | Complies |
| Diastereomers, ratio by HPLC | | Report value, x.xx | - | - | - |
| Stopper | | - | - | - | - |

Table 9A: Stability of EMI-137 Formulation Stored at 30 °C for 61 Months

| DETERMINATION | | SPECIFICATION | SAMP LING POINT (MONTHS) | | | |
|---|---|---|---|---|---|---|
| | | | 0 | | 3 | 6 |
| | | | Jul-09 | | Oct-09 | Jan-10 |
| Appearance of dry product | | Heterogeneous blue lyophilisate | Blue cake or powder | | Blue cake or powder | Blue cake or powder |
| Appearance of reconstituted product | | Clear, dark blue solution, practically free from visible particles | Clear, dark blue solution, practically free from particles | | Clear, dark blue solution, practically free from particles | Clear, dark blue solution, practically free from particles |
| Identification by HPLC, retention time | | Conforms to reference | Conforms to reference | | --- | --- |
| Content of EMI-137 | | 20.0-26.5 mg/vial | 24.0, 24.7 | | --- | --- |
| pH | | 6.8 to 8.0 | 7.5, 7.5, 7.5 | | 7.5 | 7.5 |
| Osmolality | | 250-330 mOsm/kg | 304, 278, 278 | | --- | --- |
| Related substances by HPLC | Each single related substance | Report value, x.xx% area | RRT 0.27 | 0.24 | --- | --- |
| | | | RRT 0.86 | 0.12 | --- | --- |
| | | | RRT 0.92 | 0.11 | --- | --- |
| | | | RRT 0.94 | 0.14 | --- | --- |
| | | | RRT 1.08 | 0.25 | --- | --- |

(continued)

| DETERMINATION | | SPECIFICATION | SAMP LING POINT (MONTHS) | | | |
|---|---|---|---|---|---|---|
| | | | 0 | | 3 | 6 |
| | | | Jul-09 | | Oct-09 | Jan-10 |
| | | | RRT 1.15 | 0.22 | --- | --- |
| | | | RRT 1.17 | 0.15 | --- | --- |
| | | | RRT 1.20 | 0.14 | --- | --- |
| | | | RRT 1.69 | 0.71 | --- | --- |
| | | | RRT 1.70 | 0.12 | --- | --- |
| | Sum of related substances | NMT 8.00% area | 2.19 | | 2.04, 2.05 | 2.15, 2.14 |
| Oxygen in head space | | Report value x.x µl/ vial | 26.5, 10.7, 54.7, 13.0, 2.9, ND or NMT 2.0 | | --- | --- |
| Water | | NMT 5.0% m/m | 2 | | 2.4, 2.0 | 1.4, 1.4, 1.3 |
| Bacterial Endotoxins | | NMT 110 EU/vial | NMT 0.5 | | --- | --- |
| Sterility | | Passes Ph.Eur./USP | Passes Ph.Eur./USP | | --- | --- |
| Particulate contamination | ≥10um | NMT 6000 particles/ container | NOT IN SPECS FOR PRECLINICAL | | --- | --- |
| | ≥25 um | NMT 600 particles/ container | NOT IN SPECS FOR PRECLINICAL | | --- | --- |
| Uniformity of mass of single-dose preparation | | Passes Ph. Eur./USP | --- | | --- | --- |
| Diastereomers, ratio by HPLC | | Report value, x.xx | 1.01, 1.01 | | --- | 1.01, 1.01 |

Table 9B: Stability of EMI-137 Formulation Stored at 30 °C for 61 Months

| DETERMINATION | SPECIFICATION | SAMPLING (MON THS) | | |
|---|---|---|---|---|
| | | 9 | 12 | 18 |
| | | Apr-10 | Jul-10 | Oct-10 |
| Appearance of dry product | Heterogeneous blue lyophilisate | Blue cake or powder | Blue cake or powder | Blue cake or powder |
| Appearance of reconstituted product | Clear, dark blue solution, practically free from visible particles | Clear, dark blue solution, practically free from particles | Clear, dark blue solution, practically free from particles | Clear, dark blue solution, practically free from particles |
| Identification by HPLC, retention time | Conforms to reference | --- | --- | --- |
| Content of EMI-137 | 20.0-26.5 mg/vial | --- | --- | 24.6, 23.4 |
| pH | 6.8 to 8.0 | 7.5 | 7.5 | 7.5 |

(continued)

| DETERMINATION | | SPECIFICATION | SAMPLING (MON THS) | | |
|---|---|---|---|---|---|
| | | | 9 | 12 | 18 |
| | | | Apr-10 | Jul-10 | Oct-10 |
| Osmolality | | 250-330 mOsm/kg | --- | 291 | 292 |
| Related substances by HPLC | Each single related substance | Report value, x.xx% area | --- | --- | --- |
| | | | --- | --- | --- |
| | | | --- | --- | --- |
| | | | --- | --- | --- |
| | | | --- | --- | --- |
| | | | --- | --- | --- |
| | | | --- | --- | --- |
| | | | --- | --- | --- |
| | | | --- | --- | --- |
| | | | --- | --- | --- |
| | Sum of related substances | NMT 8.00% area | 2.01, 2.03 | 1.76, 1.74 | 2.27, 2.23 |
| Oxygen in head space | | Report value x.x µl/ vial | --- | --- | --- |
| Water | | NMT 5.0% m/m | 1.2, 1.4, 1.2 | 1.5, 1.6, 1.6 | 1.4, 1.3, 1.5 |
| Bacterial Endotoxins | | NMT 110 EU/vial | --- | --- | --- |
| Sterility | | Passes Ph.Eur./USP | --- | --- | --- |
| Particulate contamination | ≥10um | NMT 6000 particles/ container | --- | --- | --- |
| | ≥25 um | NMT 600 particles/ container | --- | --- | --- |
| Uniformity of mass of single-dose preparation | | Passes Ph. Eur./USP | --- | --- | --- |
| Diastereomers, ratio by HPLC | | Report value, x.xx | --- | 1.01,1.01 | --- |

Table 9C: Stability of EMI-137 Formulation Stored at 30 °C for 61 Months

| DETERMINATION | SPECIFICATION | SAMPLING POINT (MONTHS) | | |
|---|---|---|---|---|
| | | 25 | 39 | 61 |
| | | Aug-11 | Oct-12 | Aug-14 |
| Appearance of dry product | Heterogeneous blue lyophilisate | Blue cake or powder | Heterogeneous blue lyophilisate | Heterogeneous blue lyophilisate |
| Appearance of reconstituted product | Clear, dark blue solution, practically free from visible particles | Clear, dark blue solution, practically free from particles | Clear, dark blue solution, practically free from particles | Clear, dark blue solution, practically free from particles |
| Identification by HPLC, retention time | Conforms to reference | --- | --- | --- |

(continued)

| DETERMINATION | | SPECIFICATION | SAMPLING POINT (MONTHS) | | | |
|---|---|---|---|---|---|---|
| | | | 25 | 39 | 61 | |
| | | | Aug-11 | Oct-12 | Aug-14 | |
| Content of EMI-137 | | 20.0-26.5 mg/vial | 23.6, 23.5 | 23.4, 24.7 | 22.5, 24.3 | |
| pH | | 6.8 to 8.0 | 7.5 | 7.6 | 7.4 | |
| Osmolality | | 250-330 mOsm/kg | --- | --- | --- | |
| Related substances by HPLC | Each single related substance | Report value, x.xx% area | --- | --- | RRT 0.27 | 0.25 |
| | | | --- | --- | RRT 0.86 | 0.13 |
| | | | --- | --- | RRT 0.94 | 0.13 |
| | | | --- | --- | RRT 1.08 | 0.19 |
| | | | --- | --- | RRT 1.15 | 0.28 |
| | | | --- | --- | RRT 1.17 | 0.20 |
| | | | --- | --- | RRT 1.20 | 0.13 |
| | | | --- | --- | RRT 1.23 | 0.11 |
| | | | --- | --- | RRT 1.68 | 0.66 |
| | | | --- | --- | RRT 1.68 | 0.11 |
| | Sum of related substances | NMT 8.00% area | 1.98,1.95 | 1.98, 1.95 | 2.19 | |
| Oxygen in head space | | Report value x.x µl/ vial | MT 136.0, MT 136.0, MT 136.0 | MT 136.0, MT 136.0, MT 136.0 | --- | |
| Water | | NMT 5.0% m/m | 1.7, 1.7, 1.7 | 1.8, 1.6, 1.4 | 1.8, 2.3, 2.4 | |
| Bacterial Endotoxins | | NMT 110 EU/vial | NMT 0.50 | --- | --- | |
| Sterility | | Passes Ph.Eur./USP | Ph.Eur./USP | --- | --- | |
| Particulate contamination | ≥10um ≥25 um | NMT 6000 particles/ container | --- | --- | --- | |
| | | NMT 600 particles/ container | --- | --- | --- | |
| Uniformity of mass of single-dose preparation | | Passes Ph. Eur./USP | --- | --- | --- | |
| Diastereomers, ratio by HPLC | | Report value, x.xx | 1.01,1.01 | --- | --- | |

Table 10: Stability of EMI-137 Formulation Stored at 40 °C for 6 Months

| DETERMINATION | | SPECIFICATION | SAMPLING POINT (MONTHS) | | | | |
|---|---|---|---|---|---|---|---|
| | | | 0 | | 1 | 3 | 6 |
| | | | Jul-09 | | Aug-09 | Oct-09 | Jan-10 |
| Appearance of dry product | | Heterogeneous blue lyophilisate | Blue cake or powder | | Blue cake or powder | Blue cake or powder | Blue cake or powder |
| Appearance of reconstituted product | | Clear, dark blue solution, practically free from visible particles | Clear, dark blue solution, practically free from particles | | Clear, dark blue solution, practically free from particles | Clear, dark blue solution, practically free from particles | Clear, dark blue solution, practically free from particles |
| Identification by HPLC, retention time | | Conforms to reference | Conforms to reference | | --- | --- | --- |
| Content of EMI-137 | | 20.0-26.5 mg/vial | 24.0, 24.7 | | --- | --- | --- |
| pH | | 6.8 to 8.0 | 7.5, 7.5, 7.5 | | 7.5 | 7.5 | 7.5 |
| Osmolality | | 250-330 mOsm/kg | 304, 278, 278 | | --- | --- | --- |
| Related substances by HPLC | Each single related substance | Report value, x.xx% area | RRT 0.27 | 0.24 | --- | --- | --- |
| | | | RRT 0.86 | 0.12 | --- | --- | --- |
| | | | RRT 0.92 | 0.11 | --- | --- | --- |
| | | | RRT 0.94 | 0.14 | --- | --- | --- |
| | | | RRT 1.08 | 0.25 | --- | --- | --- |
| | | | RRT 1.15 | 0.22 | --- | --- | --- |
| | | | RRT 1.17 | 0.15 | --- | --- | --- |
| | | | RRT 1.20 | 0.14 | --- | --- | --- |
| | | | RRT 1.69 | 0.71 | --- | --- | --- |
| | | | RRT 1.70 | 0.12 | --- | --- | --- |
| | Sum of related substances | NMT 8.00% area | 2.19 | | 2.22, 2.34 | 2.15, 2.05 | 2.10, 2.41 |
| Oxygen in head space | | Report value x.x µl/vial | 26.5, 10.7, 54.7, 13.0, 2.9, ND or NMT 2.0 | | --- | --- | --- |

(continued)

| DETERMINATION | | SPECIFICATION | SAMPLING POINT (MONTHS) | | | |
|---|---|---|---|---|---|---|
| | | | 0 | 1 | 3 | 6 |
| | | | Jul-09 | Aug-09 | Oct-09 | Jan-10 |
| Water | | NMT 5.0% m/m | 2 | 2.4, 2.0 | 1.5, 1.5, 1.7 | 2.0, 2.3 |
| Bacterial Endotoxins | | NMT 110 EU/vial | NMT 0.5 | --- | --- | --- |
| Sterility | | Passes Ph.Eur./USP | Passes Ph.Eur./USP | --- | --- | --- |
| Particulate contamination | ≥10um | NMT 6000 particles/container | NOT IN SPECS FOR PRECLINICAL | --- | --- | --- |
| | ≥25 um | NMT 600 particles/container | NOT IN SPECS FOR PRECLINICAL | --- | --- | --- |
| Uniformity of mass of single-dose preparation | | Passes Ph. Eur./USP | - | --- | --- | --- |
| Diastereomers, ratio by HPLC | | Report value, x.xx | 1.01, 1.01 | --- | --- | 1.01,1.01 |

The testing parameters are shown above (Table 7). Purity was tested by HPLC with UV/visual detection at 650 nm and expressed as % area units.

The agglomeration was expressed as scattering intensity measured by Photon Correlation Spectroscopy (PCS). Evaluation of the reconstitution and visual appearance after freeze drying followed a rating system developed to describe various characteristics of the freeze-dried cakes.

Visual Appearance After Freeze Drying

In general, the good bulking properties of the mannitol gave a very good structure to all mannitol containing formulations. The mannitol-phosphate containing cakes appeared to have an inhomogeneous blue/whitish colour on the vial wall as opposed to mannitol-Tris-HCl formulations. Since the drug substance is intensely blue, these colour gradients became very pronounced. The inhomogeneous appearance was expected to originate partly from the inherently random nature of freezing and partly from properties of the formulation. When reconstituted with water the product was a homogeneous solution. The other analytical results, both initially and during the pre-stability period indicated that the inhomogeneous appearance could be considered as purely a cosmetic issue. Sucrose in combination with Tris-HCl tended to collapse/melt to a variable degree during freeze drying. Considerable vial-to-vial variation was seen, as 50% of the batch had freeze dried cakes with no apparent collapse. Upon storage at 40°C, the partly collapsed plugs gradually developed into completely collapsed material, already seen after seven days. This may be due to an elevated water concentration in collapsed material. The massive collapse seen during storage had a negative effect on purity, reconstitution and the tendency to form agglomerates.

Reconstitution After Freeze Drying

All of the buffered formulations containing lyoprotectant were easily reconstituted using very gentle swirling for a few seconds. The results were unaltered for the samples with non-collapsed and moderately collapsed plugs after four weeks of storage (25°C and 40°C). Massively collapsed freeze dried plugs were very difficult to reconstitute. These products also had inferior purity and contained agglomerates. The formulations without lyoprotectants (samples 22 to 24) needed more mixing for reconstitution, but were still considered acceptable.

pH

For sodium phosphate buffered formulations the pH was stable during freeze drying but decreased during the four weeks storage at 40°C. The Tris-HCl buffered formulations had a pH decrease during freeze drying but were generally more stable during storage at 40°C. The pH results were difficult to interpret and chemometric analysis did not give a clear picture. No trends could be ascribed to mannitol or sucrose. It was, however, clear that an increase in phosphate buffer concentration would reduce the pH drop during storage. At low buffer concentration the pH drop would increase with higher EMI-137 concentration. The Tris-HCl formulations were even more sensitive to increased EMI-137 drug substance concentration. This indicated that a buffer concentration in the upper test range should be selected.

Agglomerates (PCS)

Each formulation was tested by PCS for presence of large structures/agglomerates before and after freeze drying, and at the end of the four weeks pre-stability period. The centerpoint formulations (samples 10 to 13) were also tested after one week and two weeks. Values below approximately 20 ks/s were considered as background and were not judged as agglomerates. No agglomerates were detected in any of the bulk solutions before freeze drying. All of the lyoprotectant containing formulations were judged free of larger structures/agglomerates after freeze drying. After four weeks of storage at 40°C all samples were still free of agglomerates 4, except for a massively collapsed sample (sample 17) and the non-buffered sample 24 containing EMI-137 in water. The latter formulation had a pH of 4.2. Agglomeration at low pH was consistent with previous initial findings. Phosphate buffered formulation without lyoprotectant (sucrose or mannitol) did, however, contain agglomerates after freeze drying (sample 23). Analysis of the reconstituted samples showed the presence of approximately 100nm large structures. The agglomerates were formed during the drying process, not during the freezing step, as no agglomerates were found in samples withdrawn after the freezing. Sodium phosphate buffer is known to form pH gradients and shift the pH towards low values during freezing due to difference in solubility and crystallization of the phosphate salts. pH as low as 3.5 has been reported. At this pH agglomerates would be formed in the EMI-137 solution. Though frozen, the prolonged period at this pH during the freeze drying could potentially lead to agglomerate formation. It is believed that mannitol and sucrose prevented this from happening. (No lyoprotectant was needed in the Tris-HCl formulations. The Tris- HCl would shift the pH towards high pH (>9) during freezing.) It was concluded that significant melt back/collapse will affect the agglomerate level of the drug product and should be avoided.

It was confirmed that agglomerates would form at low pH. When phosphate buffer was used as constituent during freeze drying, a lyoprotectant was needed to prevent EMI-137 from forming larger structures/agglomerates during the drying step of the freeze drying process.

Differential Scanning Calorimetrv (DSC)

Formulations representing the centerpoints in the formulation design were analysed by DSC as freeze concentrated samples. Approximately 20μl sample was contained in a sealed aluminum crucible and analyzed using a Perkin Elmer Diamond DSC instrument. The samples were cooled to - 80°C at 5°C/min and held at -80°C for 10 minutes, then heated to 24°C at 5°C/min under nitrogen atmosphere. All calculations were performed on data collected upon heating.

*Mannitol formulations*

The Tris-HCl-mannitol formulation had complex thermograms, showing three different glass transitions and two crystallization peaks. No visual collapse of the freeze dried cake was observed in spite of one glass transition below -60°C. This was probably due to the bulking properties of the crystallized mannitol. The phosphate-mannitol formulation thermogram showed two glass transitions and one crystallization peak. One of the glass transitions occurred between -50°C and -60°C, which is significantly lower than the practical operating temperature of a normal freeze dryer. The other glass transition took place around -41°C, which is close to the operating limit when designing a freeze drying cycle. Apart from a slight shrinking of the freeze dried cake, there was no macroscopic meltback/collapse of phosphate-mannitol formulations. It was anticipated that mannitol had partly crystallized, and that the crystallized portion of the mannitol was sufficient to give mechanical stability to the freeze dried product. The exotherm crystallization peak observed at -27.6°C (Tcryst onset) corresponded to the potential devitrification temperature of mannitol, as a recrystallization may occur slightly above the Tg' of mannitol (-33°C to -27°C) upon heating of the formulation.

*Sucrose formulations*

The thermograms of the sucrose containing formulations showed two thermal events consistent with glass transitions well below -50°C and at approximately -35°C to -37°C, respectively. Crystallization or eutectic melting peaks were not observed for any of the sucrose containing formulations, consistent with the content of sucrose. However, the heat flow from very small crystallization reactions would not be discernible if overlapping with the much higher heat flow from the low temperature side of the endothermic melting of water. Significant visual collapse was seen in some of the TrisHCl-sucrose containing vials, but not in the phosphate-sucrose containing formulation. Although the DSC data could be interpreted in favour of the phosphate-sucrose containing formulation (sample 13), it was concluded that the batch size of only twelve vials was too small to determine if a similar problem could be expected to occur with phosphate-sucrose formulation.

Near Infrared analysis (NIR)

It is well known that mannitol has a strong tendency to crystallize from frozen aqueous solutions, both during cooling and reheating, and has also been observed to continue to crystallize after freeze drying, as the freeze drying process may produce partially amorphous, partially crystalline material. The crystallization during freeze drying can lead to different anhydrous polymorphs (a, β, δ) and their mixtures, which leaves room for polymorphic transformations during storage.

Preliminary NIR studies combined with Principal Component Analysis (PCA) indicated the presence of structural differences between vials, that was ascribed to various degree of crystallization.

HPLC Purity

The HPLC purity profiles showed that both the phosphate and Tris-HCl formulations had a very similar purity profiles and also a similar degradation patterns compared to the pure EMI-137. Also, sucrose and mannitol containing formulations had similar purity profiles. Some small ratio differences between some of the peaks were found. No new peaks were seen in any of the formulations when compared to the EMI-137 drug substance. The buffer concentration did not seem to affect the. However, during the visual appearance analysis it was observed that sucrose in combination with Tris-HCl gave rise to vial-to-vial variability with regards to collapsed vials, as some vials had a perfect appearance while others had significantly collapsed freeze dried cakes. As both Tris HCl and sodium phosphate are substances with low Tg', the batch size of twelve vials was judged to be too small to uncover potential similar problems with phosphate-sucrose containing formulations.

Chemometric Analysis Summary

The chemometric modeling focused on changes in HPLC purity as a function of the various freeze dried formulations over a period of up to four weeks at 40°C, chemical changes in the NIR spectra to identify significant changes in degraded versus non-degraded freeze dried vials, and changes in agglomerates measured by PCS. The chemometric modeling indicated that all formulations containing Tris-HCl would have more degradation compared to the phosphate buffered formulations, though this could not easily be detected from visual comparison of the HPLC purity data. The response surfaces of the chemometric analysis indicated that increase of Tris-HCl concentration lead to increased degradation and chemical interactions. Generally, the Tris HCl containing formulations were concluded to be less predictable than phosphate regarding chemical degradation. This was supported by principal component analysis (PCA) of the NIR spectra obtained from samples 10 to 12 (representing centerpoints in the screening study) and correlation between NIR and HPLC purity.

The chemometric models made with sodium phosphate containing formulations were precise and reliable. In combination with sucrose the phosphate buffered formulations were chemically very stable within the formulation space tested, as very little degradation took place. However, more testing was needed to confirm that collapse during freeze drying would not be a potential problem. Sodium phosphate in combination with mannitol was concluded to give an adequate formulation judged from all of the analysed parameters.

Robustness/Design Space

Though the HPLC traces showed that the buffer concentration did not seem to be crucial for the degradation profile, chemometric analysis indicated that increased phosphate concentration could be expected to give a more chemically stable product when the formulation contained mannitol. The response surface showed a particularly stable area above 40mM buffer concentration, where degradation was not significantly influenced by the EMI-137 concentration. A design space of ±10% around the EMI-137 target concentration was tested. This covers a normal specification range for content of drug substance. It was found that a variation in the EMI-137 concentration within this range hardly affected purity at higher buffer concentrations, giving sufficient robustness to the formulation during manufacturing. The response surface of the pH changes demonstrated that increase in sodium phosphate buffer concentration reduced the changes of pH during storage. It was also confirmed that the formulation would not be robust towards changes in the EMI-137 concentration if the buffer concentration was too low. At buffer concentrations above 40mM the pH changes were less than 0.2 pH units, when the EMI-137 concentration varied ±10% from the target concentration (4.8 mg/ml). Above 50mM the predicted pH changes during storage were small and stable towards variations of both EMI-137 concentration and buffer concentration, giving good robustness to the formulation during manufacturing. The multivariate analysis showed that the buffer concentration can vary between 40-55 mM with a drug substance concentration varying between 4.3 and 5.3 mg/ml without affecting the quality of the drug product.

cMet Receptor Affinity *In Vitro* Test

Sample 11 was tested for its affinity to c-Met receptor, and showed binding data equivalent to those of the pure EMI-137 drug substance. It was concluded that the mannitol-40mM phosphate formulation did not adversely affect the binding of the peptide to the target receptor.

**Discussion of Results of Lyophilised Formulation**

As is illustrated in the tables above, the EMI-137 formulation remains stable over a long period of time (102 months), even at elevated temperature, thus it has an extended shelf-life. It is also notable that there is no trend of degradation of the product over the timescale of measurement, which is evidence of exceptional stability. Also, it is surprising that practically no related substances (i.e., impurities) form over the time period of measurement, that that there is no trend in the formation of these. Furthermore, on reconstitution, the formulation has a pH suitable for intravenous administration (approximately pH 7.5) and solubilises without agglomeration. In addition, the dry lyophilised solid retains a heterogenous blue appearance. It is also worth noting that the reconstituted formulation remains stable and substantially free from related substances up to 24 hours after reconstitution. Therefore, the EMI-137 formulation has been shown to be very stable in both dry (lyophilised) and reconstituted form.

Based on the analytical results and chemometric analysis the conclusions below were drawn.

It is necessary to have buffer in the formulation during preparation and freeze drying to maintain a neutral pH and avoid agglomeration. The sodium phosphate buffered formulations were judged to be more robust than the TrisHCl

formulations based on chemometric analysis of all results. The minimum phosphate buffer concentration should be 40 mM. Lyoprotectant was needed in order to freeze dry the phosphate buffered formulation to prevent agglomerate formation during the drying process. Mannitol was shown to have good lyoprotectant activity. Massively collapsed freeze dried cakes were difficult to reconstitute, had inferior purity and a tendency to form agglomerates. This was not seen in vials with moderate but still clearly visible melt back/collapse. Sodium phosphate-sucrose containing formulations were considered the chemically most robust formulations, but were potentially not as robust from a freeze drying point of view. The freeze dried sodium phosphate-mannitol formulations had an inhomogeneous appearance and were less "pharmaceutically elegant" than Tris HCl-mannitol formulations as colour gradients appear in the freeze dried cakes. This was anticipated to originate from the freezing phase and concluded to be acceptable, as the product formed a homogeneous solution upon reconstitution. The Tris HCI containing formulations had generally less predictive data, and based on chemometric analysis these formulations were considered potentially a riskier (albeit still valid) choice. The overall conclusion from the study was that the sodium phosphate-mannitol containing formulation was considered to provide a satisfactory balance of risk, predictability and acceptability based on the limited pre-stability data available, the chemometric analysis of the analytical data and robustness considerations, albeit Tris HCI and sucrose may also be independently used as alternatives.

## Summary

The present invention teaches that in order to obtain adequate stability of the API, a freeze-dried drug product formulation is required. After reconstitution, the drug product should be an isotonic solution and have a physiological pH to make it suitable for intravenous administration. EMI-137 in water has a pH of 4.6, and shows a significant degree of formation of larger structures or agglomerates. This agglomeration is only partly reversible upon increasing the pH of the solution, thus making it unsuitable for intravenous administration. As such, a pH of greater than 6.8 and less than 9 (or potentially lower) is advantageous, since the formation of agglomerates is not observed in this pH range. This pH range should be maintained at all times, and should also be applied during the manufacturing of the bulk drug product solution. Thus, to maintain the pH of the EMI-137 solution within an acceptable range, it is beneficial to include a buffer to the solution prior to freeze-drying. It is also beneficial to include a lyoprotectant to ensure that EMI-137 does not degrade during freeze-drying, and a tonicity regulator (which may be the same material as the lyoprotectant) to ensure that the reconstituted product is isotonic and/or suitable for intravenous administration.

The use of the buffer and lyoprotectant/tonicity regulator as described above provides a homogenous solid that is stable and that, on reconstitution, is at a pH and tonicity that is suitable for intravenous administration. In addition, the formulation described retains a sufficient pH during lyophilisation that prevents agglomeration from taking place, and that does not result in the breakdown of the disulphide bridges in EMI-137. Furthermore, the formulation is storable at room temperature for elongated periods of time without notable degradation, and remains stable on and after reconstitution.

As noted above, to maintain the pH of the EMI-137 solution within an acceptable range on reconstitution, it is necessary to include a buffer prior to freeze-drying.

### Abbreviations

Conventional single letter or 3-letter amino acid abbreviations are used.

Acm: Acetamidomethyl
ACN (or MeCN): Acetonitrile
Boc: *tert*-Butyloxycarbonyl
DCM: Dichloromethane
DMF: Dimethylformamide DMSO: Dimethylsulfoxide
Fmoc: 9-Fluorenylmethoxycarbonyl
HBTU: O-Benzotriazol-1-yl-N,N,N',N'-tetramethyluronium hexafluorophosphate
HPLC: High performance liquid chromatography
HSPyU: O-(N-succinimidyl)-N,N,N',N'-tetramethyleneuronium hexafluorophosphate
NHS: *N*-hydroxy-succinimide
NMM: *N*-Methylmorpholine
NMP : 1-Methyl-2-pyrrolidinone
Pbf: 2,2,4,6,7-Pentamethyldihydrobenzofuran-5-sulfonyl
PBS: Phosphate-buffered saline
tBu: *t*-butyl
TFA: Trifluoroacetic acid

TIS: Triisopropylsilane
Trt: Trityl
Tris HCl: 2-Amino-2-(hydroxymethyl)-1,3-propanediolhydrochloride

Sequence Listing Free Text

SEQ-1

Cys$^a$-Xaa$^1$-Cys$^c$-Xaa$^2$-Gly-Pro-Pro-Xaa$^3$-Phe-Glu-Cys$^d$-Trp-Cys$^b$-Tyr-Xaa$^4$-Xaa$^5$-Xaa$^6$

Xaa$^1$ is Asn, His or Tyr;
Xaa$^2$ is Gly, Ser, Thr or Asn;
Xaa$^3$ is Thr or Arg;
Xaa$^4$ is Ala, Asp, Glu, Gly or Ser;
Xaa$^5$ is Ser or Thr; and
Xaa$^6$ is Asp or Glu.
17-mer cMET binding peptide.

SEQ-2

Ser-Cys$^a$-Xaa$^1$-Cys$^c$-Xaa$^2$-Gly-Pro-Pro-Xaa$^3$-Phe-Glu-Cys$^d$-Trp-Cys$^b$-Tyr-Xaa$^4$-Xaa$^5$-Xaa$^6$

Xaa$^1$ is Asn, His or Tyr;
Xaa$^2$ is Gly, Ser, Thr or Asn;
Xaa$^3$ is Thr or Arg;
Xaa$^4$ is Ala, Asp, Glu, Gly or Ser;
Xaa$^5$ is Ser or Thr; and
Xaa$^6$ is Asp or Glu.
18-mer cMET binding peptide.

SEQ-3

Ala-Gly-Ser-Cys$^a$-Xaa$^1$-Cys$^c$-Xaa$^2$-Gly-Pro-Pro-Xaa$^3$-Phe-Glu-Cys$^d$-Trp-Cys$^b$-Tyr-Xaa$^4$-Xaa$^5$-Xaa$^6$-Gly-Thr

Xaa$^1$ is Asn, His or Tyr;
Xaa$^2$ is Gly, Ser, Thr or Asn;
Xaa$^3$ is Thr or Arg;
Xaa$^4$ is Ala, Asp, Glu, Gly or Ser;
Xaa$^5$ is Ser or Thr; and
Xaa$^6$ is Asp or Glu.
22-mer cMET binding peptide.

SEQ-4
Gly-Gly-Gly-Lys
Tetrapeptide sequence that is part of cMET binding peptide.
SEQ-5
Gly-Ser-Gly-Lys

Tetrapeptide sequence that is part of cMET binding peptide.
SEQ-6
Gly-Ser-Gly-Ser-Lys
Five peptide sequence that is part of cMET binding peptide.
SEQ-7

Ala-Gly-Ser-Cys-Tyr-Cys-Ser-Gly-Pro-Pro-Arg-Phe-Glu-Cys-Trp-Cys-Tyr-Glu-Thr-Glu-Gly-Thr-Gly-Gly-Gly-Lys

26-mer cMET binding peptide.
SEQ-8

Thr-Gly-Glu-Cys-Thr-Cys-Pro-Tyr-Trp-Glu-Phe-Arg-Pro-Cys-Glu-Cys-Gly-Ser-Tyr-Ser-Gly-Ala-Gly-Gly-Gly-Lys

26-mer srambled cMET binding peptide (negative control).

**Claims**

**1.** A lyophilised formulation comprising:

(i) an active pharmaceutical ingredient (API);
(ii) a buffering agent; and
(iii) a lyoprotectant;

wherein the API is an imaging agent comprising at least one cMet binding peptide, suitable for optically imaging the mammalian body, the imaging agent comprising a compound of Formula I:

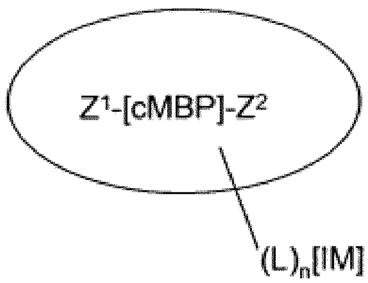

(Formula I)

wherein:

$Z^1$ is attached to the N-terminus of cMBP, and is H or $M^{IG}$;
$Z^2$ is attached to the C-terminus of cMBP, and is OH, $NH_2$, $OB^c$ or $M^{IG}$;

wherein $B^c$ is a biocompatible cation;

cMBP is a cMet binding cyclic peptide of 17 to 30 amino acids, which comprises the amino acid sequence (SEQ-1):

$$Cys^a\text{-}Xaa^1\text{-}Cys^c\text{-}Xaa^2\text{-}Gly\text{-}Pro\text{-}Pro\text{-}Xaa^3\text{-}Phe\text{-}Glu\text{-}Cys^d\text{-}Trp\text{-}Cys^b\text{-}Tyr\text{-}Xaa^4\text{-}Xaa^5\text{-}Xaa^6;$$

wherein: $Xaa^1$ is Asn, His or Tyr;
$Xaa^2$ is Gly, Ser, Thr or Asn;
$Xaa^3$ is Thr or Arg;
$Xaa^4$ is Ala, Asp, Glu, Gly or Ser;
$Xaa^5$ is Ser or Thr;
$Xaa^6$ is Asp or Glu;

and $Cys^{a-d}$ are each cysteine residues such that residues a and b as well as c and d are cyclised to form two separate disulphide bonds;
$M^{IG}$ is a metabolism inhibiting group, which is a biocompatible group that inhibits or suppresses *in vivo* metabolism of the peptide;
L is a synthetic linker group of formula -(A)m- wherein each A is independently $-CR_2-$, -CR=CR-, -C≡C-, $-CR_2CO_2-$, - $CO_2CR_2-$, -NRCO-, -CONR-, -NR(C=O)NR-, -NR(C=S)NR-, - $SO_2NR-$, $-NRSO_2-$, $CR_2OCR_2-$, $-CR_2SCR_2-$, $CR_2NRCR_2-$, a $C_{4-8}$ cycloheteroalkylene group, a $C_{4-8}$ cycloalkylene group, a C5-12 arylene group, a $C_{3-12}$ heteroarylene group, an amino acid, a sugar or a monodisperse polyethyleneglycol (PEG) building block;
each R is independently chosen from H, $C_{1-4}$ alkyl, $C_{2-4}$ alkenyl, $C_{2-4}$ alkynyl, $C_{1-4}$ alkoxyalkyl or $C_{1-4}$ hydroxyalkyl;
m is an integer of value 1 to 20;
n is an integer of value 0 or 1;
IM is an optical reporter imaging moiety suitable for imaging the mammalian body using light of violet to near-infrared wavelength (400 to 1,200 nm);

wherein the formulation comprises from approximately 4 to approximately 12 % by weight API, from approximately 3 to approximately 35 % by weight buffering agent, and from approximately 56 to approximately 91 % by weight

lyoprotectant; and wherein the formulation, when reconstituted, has a pH of between approximately pH 6.8 and approximately pH 9.

2. The lyophilised formulation of claim 1, wherein in addition to SEQ-1, the cMBP further comprises an Asp or Glu residue, or an analogue thereof, within 4 amino acid residues of either C- or N- cMBP peptide terminus, and $-(L)_nIM$ is functionalised with an amine group, which is conjugated to the carboxyl side chain of said Asp or Glu residue, or analogue thereof, to give an amide bond; and/or wherein in addition to SEQ-1, the cMBP comprises a Lys residue, or an analogue thereof, within 4 amino acid residues of either C- or N- cMBP peptide terminus, and $-(L)_nIM$ is functionalised with a carboxyl group, which is conjugated to the epsilon amine side chain of said Lys residue, or analogue thereof, to give an amide bond.

3. The lyophilised formulation of claim 1 or claim 2, wherein cMBP comprises the amino acid sequence of either SEQ-2 or SEQ-3:

Ser-Cys$^a$-Xaa$^1$-Cys$^c$-Xaa$^2$-Gly-Pro-Pro-Xaa$^3$-Phe-Glu-Cys$^d$-Trp-Cys$^b$-Tyr-Xaa$^4$-Xaa$^5$-Xaa$^6$ (SEQ-2);

Ala-Gly-Ser-Cys$^a$-Xaa$^1$-Cys$^c$-Xaa$^2$-Gly-Pro-Pro-Xaa$^3$-Phe-Glu-Cys$^d$-Trp-Cys$^b$-Tyr-Xaa$^4$-Xaa$^5$-Xaa$^6$-Gly-Thr (SEQ-3).

4. The lyophilised formulation of any preceding claim, wherein Xaa$^3$ is Arg.

5. The lyophilised formulation of any preceding claim, wherein in addition to SEQ-1, SEQ-2 or SEQ-3, cMBP further comprises at either the N- or C- terminus a linker peptide, which is chosen from - Gly-Gly-Gly-Lys (SEQ-4), -Gly-Ser-Gly-Lys- (SEQ-5) and -Gly-Ser-Gly-Ser-Lys (SEQ-6).

6. The lyophilised formulation of any preceding claim, wherein cMBP comprises the amino acid sequence (SEQ-7):

Ala-Gly-Ser-Cys$^a$-Tyr-Cys$^c$-Ser-Gly-Pro-Pro-Arg-Phe-Glu-Cys$^d$-Trp-Cys$^b$-Tyr-Glu-Thr-Glu-Gly-Thr-Gly-Gly-Gly-Lys.

7. The lyophilised formulation of any preceding claim, wherein both $Z^1$ and $Z^2$ are independently $M^{IG}$, optionally wherein $Z^1$ is acetyl and $Z^2$ is a primary amide.

8. The lyophilised formulation of any preceding claim, wherein IM is a dye having an absorbance maximum in the range 600 to 1,000 nm, optionally wherein IM is a cyanine dye, optionally wherein the cyanine dye has Formula III:

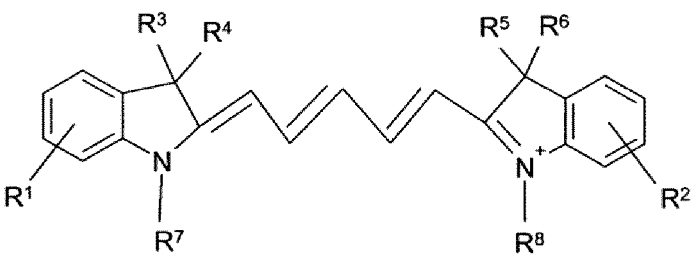

(Formula III)

wherein:

$R^1$ and $R^2$ are independently H or $SO_3M^1$, and at least one of $R^1$ and $R^2$ is $SO_3M^1$, where $M^1$ is H or $B^c$;
$R^3$ and $R^4$ are independently $C_{1-4}$ alkyl or $C_{1-6}$ carboxyalkyl;
$R^5$, $R^6$, $R^7$ and $R^8$ are independently $R^a$ groups;
wherein $R^a$ is $C_{1-4}$ alkyl, $C_{1-6}$ carboxyalkyl or $-(CH_2)_kSO_3M^1$, where k is an integer of value 3 or 4;
with the proviso that the cyanine dye has a total of 1 to 4 $SO_3M^1$ substituents in the $R^1$, $R^2$ and $R^a$ groups.

**9.** The lyophilised formulation of any preceding claim, wherein the formulation, when reconstituted, has a pH of between approximately pH 6.8 and approximately pH 8.2, optionally between approximately pH 6.8 and approximately pH 8, optionally between approximately pH 7 and approximately pH 8.

**10.** The lyophilised formulation of any preceding claim, wherein the buffering agent is present in an amount to provide, when reconstituted, a solution having a pH of between approximately pH 6.8 and approximately pH 9, optionally between approximately pH 6.8 and approximately pH 8.2, optionally between approximately pH 6.8 and approximately pH 8, optionally between approximately pH 7 and approximately pH 8.

**11.** The lyophilised formulation of any preceding claim, wherein the formulation comprises from approximately 8 to approximately 10 % by weight API, optionally approximately 9 % by weight API.

**12.** The lyophilised formulation of any preceding claim, wherein the formulation comprises from approximately 7 to approximately 35 % by weight buffering agent, optionally from approximately 12 to approximately 35 % by weight buffering agent, optionally from approximately 15 to approximately 35 % by weight buffering agent, optionally from approximately 18 to approximately 35 % by weight buffering agent, optionally from approximately 25 to approximately 35 % by weight buffering agent, optionally from approximately 32 to approximately 35 % by weight buffering agent, optionally approximately 32 % by weight buffering agent.

**13.** The lyophilised formulation of any preceding claim, wherein the formulation comprises from approximately 56 to approximately 82 % by weight lyoprotectant, optionally from approximately 56 to approximately 77 % by weight lyoprotectant, optionally from approximately 56 to approximately 75 % by weight lyoprotectant, optionally from approximately 56 to approximately 72 % by weight lyoprotectant, optionally from approximately 56 to approximately 66 % by weight lyoprotectant, optionally from approximately 56 to approximately 58 % by weight lyoprotectant, optionally approximately 58 % by weight lyoprotectant.

**14.** The lyophilised formulation of any preceding claim, wherein the buffering agent is at least one of a phosphate buffer and an alkanolamine buffer, optionally wherein the phosphate buffer comprises hydrogen phosphate and dihydrogen phosphate, optionally wherein the phosphate buffer is hydrated, optionally wherein the alkanolamine buffer is tris(hydroxymethyl)aminomethane.

**15.** The lyophilised formulation of any preceding claim, wherein the lyoprotectant is at least one of sucrose and mannitol, and derivatives thereof, optionally wherein the lyoprotectant is mannitol, or a derivative thereof.

**16.** The lyophilised formulation of any preceding claim, wherein the formulation further comprises a tonicity regulator, optionally wherein the lyoprotectant also acts as a tonicity regulator.

**17.** A method of preparing a lyophilised formulation, the method comprising the steps of:

a) providing an active pharmaceutical ingredient (API), a buffering agent, and a lyoprotectant to a lyophilisation vessel;
b) performing a first water removal step; and
c) performing a second water removal step;

wherein the lyoprotectant and the buffering agent are added before the lyophilisation is carried out, and the API is an imaging agent comprising at least one cMet binding peptide, suitable for optically imaging the mammalian body, the imaging agent comprising a compound of Formula I:

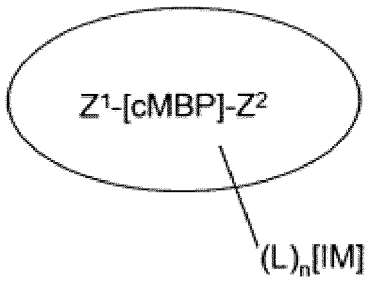

(Formula I)

wherein:

$Z^1$ is attached to the N-terminus of cMBP, and is H or $M^{IG}$;
$Z^2$ is attached to the C-terminus of cMBP, and is OH, $NH_2$, $OB^c$ or $M^{IG}$;

wherein $B^c$ is a biocompatible cation;

cMBP is a cMet binding cyclic peptide of 17 to 30 amino acids, which comprises the amino acid sequence (SEQ-1):

$Cys^a$-$Xaa^1$-$Cys^c$-$Xaa^2$-Gly-Pro-Pro-$Xaa^3$-Phe-Glu-$Cys^d$-Trp-$Cys^b$-Tyr-$Xaa^4$-$Xaa^5$-$Xaa^6$;

wherein: $Xaa^1$ is Asn, His or Tyr;
$Xaa^2$ is Gly, Ser, Thr or Asn;
$Xaa^3$ is Thr or Arg;
$Xaa^4$ is Ala, Asp, Glu, Gly or Ser;
$Xaa^5$ is Ser or Thr;
$Xaa^6$ is Asp or Glu;

and $Cys^{a-d}$ are each cysteine residues such that residues a and b as well as c and d are cyclised to form two separate disulphide bonds;
$M^{IG}$ is a metabolism inhibiting group, which is a biocompatible group that inhibits or suppresses *in vivo* metabolism of the peptide;
L is a synthetic linker group of formula -(A)m- wherein each A is independently $-CR_2-$, -CR=CR-, -C≡C-, $-CR_2CO_2-$, - $CO_2CR_2-$, -NRCO-, -CONR-, -NR(C=O)NR-, -NR(C=S)NR-, - $SO_2NR-$, $-NRSO_2-$, $CR_2OCR_2-$, $-CR_2SCR_2-$, $CR_2NRCR_2-$, a $C_{4-8}$ cycloheteroalkylene group, a $C_{4-8}$ cycloalkylene group, a C5-12 arylene group, a $C_{3-12}$ heteroarylene group, an amino acid, a sugar or a monodisperse polyethyleneglycol (PEG) building block;
each R is independently chosen from H, $C_{1-4}$ alkyl, $C_{2-4}$ alkenyl, $C_{2-4}$ alkynyl, $C_{1-4}$ alkoxyalkyl or $C_{1-4}$ hydroxyalkyl;
m is an integer of value 1 to 20;
n is an integer of value 0 or 1;
IM is an optical reporter imaging moiety suitable for imaging the mammalian body using light of violet to near-infrared
wavelength (400 to 1,200 nm);

wherein the formulation comprises from approximately 4 to approximately 12 % by weight API, from approximately 3 to approximately 35 % by weight buffering agent, and from approximately 56 to approximately 91 % by weight lyoprotectant; and wherein the formulation, when reconstituted, has a pH of between approximately pH 6.8 and approximately pH 9.

**18.** The method of claim 17, wherein the first water removal step is carried out at a temperature of approximately -30 °C or lower, optionally approximately -35 °C or lower, optionally approximately - 38 °C or lower.

**19.** The method of claim 17 or claim 18, wherein the second water removal step is carried out at a temperature of approximately 10 °C or higher, optionally approximately 15 °C or higher, optionally approximately 20 °C or higher.

**20.** The method of any one of claims 17 to 19, wherein at least one of the first and second water removal steps is carried out at a pressure of 50 μbar or less, optionally wherein both of the first and second water removal steps are carried out at a pressure of 50 μbar or less.

**21.** The method of any one of claims 17 to 20, wherein the lyophilised formulation is the lyophilised formulation any one of claim 1 to 16.

**22.** A pharmaceutical composition comprising the formulation of any one of claims 1 to 16 and a biocompatible carrier, in a form suitable for mammalian administration.

**23.** A kit for the preparation of the pharmaceutical composition of claim 22, the kit comprising the formulation of any one of claims 1 to 16 in sterile, solid form such that upon reconstitution with a sterile supply of the biocompatible carrier of claim 22, dissolution occurs to give the desired pharmaceutical composition.

**24.** The formulation of any one of claims 1 to 16, or the pharmaceutical composition of claim 22, for use as an imaging agent in imaging of the mammalian body.

**25.** The formulation of any one of claims 1 to 16, or the pharmaceutical composition of claim 22, for use as a medicament.

**26.** The formulation of any one of claims 1 to 16, or the pharmaceutical composition of claim 22, for use in detection, diagnosis, surgery, staging, treatment, monitoring of treatment, monitoring of disease progression or monitoring therapy.

**27.** The formulation of any one of claims 1 to 16, or the pharmaceutical composition of claim 22, for use in the detection, diagnosis, surgery, staging, treatment, monitoring of treatment, monitoring of disease progression or monitoring therapy of one or more of a precancerous condition and cancer, optionally one or more of colorectal cancer, oesophageal cancer, breast cancer, prostate cancer, head cancer, neck cancer, ovarian cancer, rectal cancer, pancreatic cancer, thyroid cancer, gastric cancer and sarcoma.

**28.** The formulation of any one of claims 1 to 16, or the pharmaceutical composition of claim 22, for use in the detection, diagnosis, surgery, staging, treatment, monitoring of treatment, monitoring of disease progression or monitoring therapy of sites of cMet over-expression or localisation.

**29.** The formulation of any one of claims 1 to 16, or the pharmaceutical composition of claim 22, for use in obtaining an image of sites of cMet over-expression or localisation, optionally *in vivo.*

**Patentansprüche**

**1.** Lyophilisierte Formulierung, umfassend:

(i) einen aktiven pharmazeutischen Wirkstoff (API);
(ii) ein Puffermittel; und
(iii) ein Lyoprotektivum;

wobei der API ein Bildgebungsmittel ist, das mindestens ein cMet-bindendes Peptid umfasst, das zur optischen Bildgebung des Säugerkörpers geeignet ist, wobei das Bildgebungsmittel eine Verbindung der Formel I umfasst:

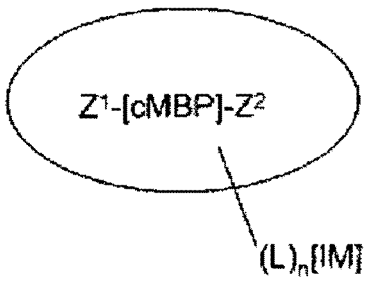

(Formula I)

worin:

$Z^1$ an den N-Terminus von cMBP gebunden ist und für H oder$^{IG}$ steht;
$Z^2$ an den C-Terminus von cMBP gebunden ist und für OH, $NH^2$, $OB^C$ oder $M^{IG}$ steht;
worin $B^c$ für ein biokompatibles Kation steht;
cMBP für ein cMet bindendes cyclisches Peptid mit 17 bis 30 Aminosäuren steht, das die Aminosäuresequenz (SEQ-1) umfasst: $Cys^a$-$Xaa^1$-$Cys^c$-$Xaa^2$-Gly-Pro-Pro-$Xaa^3$-Phe-Glu-$Cys^d$-Trp- $Cys^b$-Tyr-$Xaa^4$-$Xaa^5$-$Xaa^6$;

worin:

$Xaa^1$ für Asn, His oder Tyr steht;
$Xaa^2$ für Gly, Ser, Thr oder Asn steht;
$Xaa^3$ für Thr oder Arg steht;
$Xaa^4$ für Ala, Asp, Glu, Gly oder Ser steht;
$Xaa^5$ für Ser oder Thr steht;
$Xaa^6$ für Asp oder Glu steht;

und $Cys^{a-d}$ jeweils für Cysteinreste stehen, so dass Reste a und b sowie c und d cyclisiert sind, um zwei separate Disulfidbindungen zu bilden;

$M^{IG}$ für eine Metabolismus inhibierende Gruppe steht, die eine biokompatible Gruppe ist, die *in vivo* Metabolismus des Peptids hemmt oder unterdrückt;
L für eine synthetische Linker-Gruppe der Formel -(A)m- steht, worin jedes A unabhängig steht für $-CR_2-$, -CR=CR- -C≡C-, $-CR_2CO_2-$, - $CO_2CR_2-$, -NRCO-, - CONR-, -NR(C=O)NR- -NR(C=S)NR- - $SO_2NR-$, $-NRSO_2-$, $CR_2OCR_2-$, $-CR_2SCR_2-$, $CR_2-NRCR_2-$, eine $C_{4-8}$-Cycloheteroalkylengruppe, eine $C_{4-8}$-Cycloalkylengruppe, eine $C_{5-12}$-Arylengruppe, eine $C_{3-12}$-Heteroarylengruppe, eine Aminosäure, einen Zucker oder einen monodispersen Polyethylenglykol-(PEG)-Bildungsblock;
jedes R unabhängig ausgewählt ist aus H, $C_{1-4}$-Alkyl, $C_{2-4}$-Alkenyl, $C_{2-4}$-Alkinyl, $C_{1-4}$-Alkoxyalkyl oder $C_{1-4}$-Hydroxyalkyl; m für eine ganze Zahl mit dem Wert von 1 bis 20 steht;
n für eine ganze Zahl mit dem Wert 0 oder 1 steht;
IM für eine optische Reporterbildgebungseinheit steht, die für die Bildgebung des Säugerkörpers unter Verwendung von Licht mit violetter bis
nahinfraroter Wellenlänge (400 bis 1.200 nm) geeignet ist;

wobei die Formulierung etwa 4 bis etwa 12 Gew.-% API, etwa 3 bis etwa 35 Gew.-% Puffermittel und etwa 56 bis etwa 91 Gew.-% Lyoprotektivum umfasst; und wobei die Formulierung, wenn sie rekonstituiert ist, einen pH-Wert zwischen etwa pH 6,8 und etwa pH 9 aufweist.

**2.** Lyophilisierte Formulierung nach Anspruch 1, wobei zusätzlich zu SEQ-1 das cMBP weiter einen Asp- oder Glu-Rest oder ein Analogon davon innerhalb von 4 Aminosäureresten entweder des C- oder N- cMBP-Peptidterminus umfasst und $-(L)_n$IM mit einer Amingruppe funktionalisiert ist, die an die Carboxylseitenkette des Asp- oder Glu-Rests oder eines Analogons davon konjugiert ist, damit sich Amidbindung ergibt; und/oder wobei zusätzlich zur SEQ-1 das cMBP einen Lys-Rest oder ein Analogon davon innerhalb von 4 Aminosäureresten entweder des C- oder des N- cMBP-Peptidterminus umfasst, und $-(L)_n$IM mit einer Carboxylgruppe funktionalisiert ist, die mit der Epsilon-Amin-Seitenkette des Lys-Rests oder eines Analogons davon konjugiert ist, damit sich eine Amidbindung

ergibt.

3. Lyophilisierte Formulierung nach Anspruch 1 oder Anspruch 2, wobei cMBP die Aminosäuresequenz von entweder SEQ-2 oder SEQ-3 umfasst: Ser-Cys$^a$-Xaa$^1$-CyS$^c$-Xaa$^2$-Gly-Pro-Pro-Xaa$^3$-Phe-Glu-Cys$^d$-Trp- CyS$^b$-Tyr-Xaa$^4$-Xaa$^5$-Xaa$^6$ (SEQ-2);

Ala-Gly-Ser-Cys$^a$-Xaa$^1$-Cys$^c$-Xaa$^2$-Gly-Pro-Pro-Xaa$^3$-Phe-Glu-Cys$^d$- Trp-Cys$^b$-Ty r-Xaa$^4$-Xaa$^5$-Xaa$^6$-Gly-Thr (SEQ-3).

4. Lyophilisierte Formulierung nach einem der vorstehenden Ansprüche, wobei Xaa$^3$ für Arg steht.

5. Lyophilisierte Formulierung nach einem der vorstehenden Ansprüche, wobei zusätzlich zu SEQ-1, SEQ-2 or SEQ-3 cMBP weiter entweder am N- oder am C-Terminus ein Linker-Peptid umfasst, das ausgewählt ist aus -Gly-Gly-Gly-Lys (SEQ-4), -Gly-Ser-Gly-Lys- (SEQ-5) und -Gly-Ser- Gly-Ser-Lys (SEQ-6).

6. Lyophilisierte Formulierung nach einem der vorstehenden Ansprüche, wobei cMBP die Aminosäuresequenz (SEQ-7) umfasst:

Ala-Gly-Ser-Cys$^a$-Tyr-Cys$^c$-Ser-Gly-Pro-Pro-Arg-Phe-Glu-Cys$^d$-Trp- Cys$^b$-Tyr-Glu-Thr-Glu-Gly-Thr-Gly-Gly-Gly-Lys.

7. Lyophilisierte Formulierung nach einem der vorstehenden Ansprüche, wobei sowohl $Z^1$ als auch $Z^2$ unabhängig voneinander $M^{IG}$ sind, wahlweise wobei $Z^1$ für Acetyl steht und $Z^2$ für ein primäres Amid steht.

8. Lyophilisierte Formulierung nach einem der vorstehenden Ansprüche, wobei IM für einen Farbstoff steht, der ein Absorbanzmaximum im Bereich 600 bis 1.000 nm aufweist, wahlweise wobei IM für einen Cyaninfarbstoff steht, wahlweise wobei der Cyaninfarbstoff die Formel III aufweist:

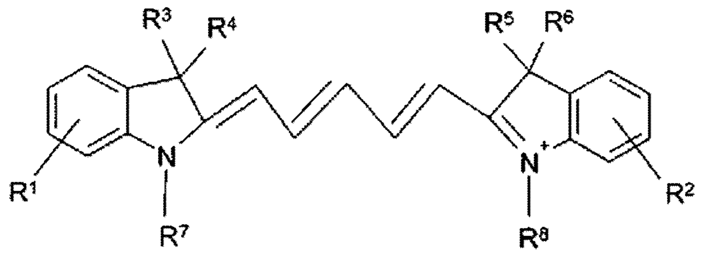

(Formula III)

worin:

$R^1$ und $R^2$ unabhängig für H oder $SO_3M^1$ stehen, und mindestens eines von $R^1$ und $R^2$ für $SO_3M^1$ steht, worin $M^1$ für H oder $B^c$ steht;
$R^3$ und $R^4$ unabhängig für $C_{1-4}$-Alkyl oder $C_{1-6}$-Carboxyalkyl stehen;
$R^5$, $R^6$, $R^7$ und $R^8$unabhängig für $R^a$-Gruppen stehen;
worin $R^a$ für $C_{1-4}$-Alkyl, $C_{1-6}$-Carboxyalkyl oder-$(CH_2)_kSO_3M^1$ steht, worin k für eine ganze Zahl mit dem Wert 3 oder 4 steht;
mit der Maßgabe, dass der Cyaninfarbstoff eine Gesamtheit von 1 bis 4 $SO_3M^1$ - Substituenten in den $R^1$-, $R^2$- und $R^a$-Gruppen aufweist.

9. Lyophilisierte Formulierung nach einem der vorstehenden Ansprüche, wobei die Formulierung im rekonstituierten Zustand einen pH-Wert zwischen etwa pH 6,8 und etwa pH 8,2, wahlweise zwischen etwa pH 6,8 und etwa pH 8,

wahlweise zwischen etwa pH 7 und etwa pH 8 aufweist.

**10.** Lyophilisierte Formulierung nach einem der vorstehenden Ansprüche, wobei das Puffermittel in einer Menge vorliegt zur Bereitstellung, wenn es rekonstituiert ist, einer Lösung mit einem pH-Wert zwischen etwa pH 6,8 und etwa pH 9, wahlweise zwischen etwa pH 6,8 und etwa pH 8,2, wahlweise zwischen etwa pH 6,8 und etwa pH 8, wahlweise zwischen etwa pH 7 und etwa pH 8.

**11.** Lyophilisierte Formulierung nach einem der vorstehenden Ansprüche, wobei die Formulierung etwa 8 bis etwa 10 Gew.-% API, wahlweise etwa 9 Gew.-% API umfasst.

**12.** Lyophilisierte Formulierung nach einem der vorstehenden Ansprüche, wobei die Formulierung etwa 7 bis etwa 35 Gew.-% Puffermittel, wahlweise etwa 12 bis etwa 35 Gew.-% Puffermittel, wahlweise etwa 15 bis etwa 35 Gew.-% Puffermittel, wahlweise etwa 18 bis etwa 35 Gew.-% Puffermittel, wahlweise etwa 25 bis etwa 35 Gew.-% Puffermittel, wahlweise etwa 32 bis etwa 35 Gew.-% Puffermittel, wahlweise etwa 32 Gew.-% Puffermittel umfasst.

**13.** Lyophilisierte Formulierung nach einem der vorstehenden Ansprüche, wobei die Formulierung etwa 56 bis etwa 82 Gew.-% Lyoprotektivum, wahlweise etwa 56 bis etwa 77 Gew.-% Lyoprotektivum, wahlweise etwa 56 bis etwa 75 Gew.-% Lyoprotektivum, wahlweise etwa 56 bis etwa 72 Gew.-% Lyoprotektivum, wahlweise etwa 56 bis etwa 66 Gew.-% Lyoprotektivum, wahlweise etwa 56 bis etwa 58 Gew.-% Lyoprotektivum, wahlweise etwa 58 Gew.-% Lyoprotektivum umfasst.

**14.** Lyophilisierte Formulierung nach einem der vorstehenden Ansprüche, wobei das Puffermittel mindestens eines aus einem Phosphatpuffer und einem Alkanolaminpuffer ist, wahlweise wobei der Phosphatpuffer Hydrogenphosphat und Dihydrogenphosphat umfasst, wahlweise wobei der Phosphatpuffer hydratisiert ist, wahlweise wobei der Alkanolaminpuffer Tris(hydroxymethyl)aminomethan ist.

**15.** Lyophilisierte Formulierung nach einem der vorstehenden Ansprüche, wobei das Lyoprotektivum mindestens eines von Saccharose und Mannitol und Derivaten davon ist, wahlweise wobei das Lyoprotektivum Mannitol oder ein Derivat davon ist.

**16.** Lyophilisierte Formulierung nach einem der vorstehenden Ansprüche, wobei die Formulierung weiter einen Tonusregulator umfasst, wahlweise wobei das Lyoprotektivum auch als Tonusregulator wirkt.

**17.** Verfahren zur Herstellung einer lyophilisierten Formulierung, wobei das Verfahren die folgenden Schritte umfasst:

a) Bereitstellen eines aktiven pharmazeutischen Wirkstoffs (API), eines Puffermittels und eines Lyoprotektivums in einem Lyophilisierungsbehälter;
b) Durchführen eines ersten Wasserentfernungsschritts; und
c) Durchführen eines zweiten Wasserentfernungsschritts;

wobei das Lyoprotektivum und das Puffermittel vor der Durchführung der Lyophilisierung zugegeben werden und der API ein Bildgebungsmittel ist, das mindestens ein cMet-bindendes Peptid umfasst, das zur optischen Bildgebung des Säugerkörpers geeignet ist, wobei das Bildgebungsmittel eine Verbindung der Formel I umfasst:

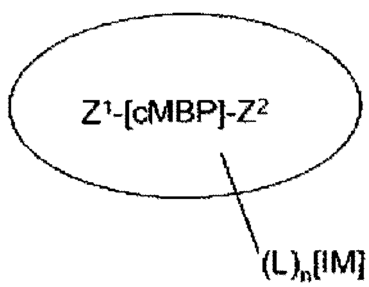

(Formel I)

worin:

$Z^1$ an den N-Terminus von cMBP gebunden ist und für H oder$^{IG}$ steht;
$Z^2$ an den C-Terminus von cMBP gebunden ist und für OH, $NH^2$, $OB^C$ oder $M^{IG}$ steht;
worin $B^c$ für ein biokompatibles Kation steht;
cMBP für ein cMet bindendes cyclisches Peptid mit 17 bis 30 Aminosäuren steht, das die Aminosäuresequenz (SEQ-1) umfasst: $Cys^a$-$Xaa^1$-$Cys^c$-$Xaa^2$-Gly-Pro-Pro-$Xaa^3$-Phe-Glu-$Cys^d$-Trp-$Cys^b$-Tyr-$Xaa^4$-$Xaa^5$-$Xaa^6$;

worin: $Xaa^1$ für Asn, His oder Tyr steht;

$Xaa^2$ für Gly, Ser, Thr oder Asn steht;
$Xaa^3$ für Thr oder Arg steht;
$Xaa^4$ für Ala, Asp, Glu, Gly oder Ser steht;
$Xaa^5$ für Ser oder Thr steht;
$Xaa^6$ für Asp oder Glu steht;

und $Cys^{a-d}$ jeweils für Cysteinreste stehen, so dass Reste a und b sowie c und d cyclisiert sind, um zwei separate Disulfidbindungen zu bilden;

$M^{IG}$ für eine Metabolismus inhibierende Gruppe steht, die eine biokompatible Gruppe ist, die *in vivo* Metabolismus des Peptids hemmt oder unterdrückt;
L für eine synthetische Linker-Gruppe der Formel -(A)m- steht, worin jedes A unabhängig steht für $-CR_2-$, -CR=CR-, -C≡C-, $-CR_2CO_2-$, - $CO_2CR_2-$, -NRCO-, - CONR-, -NR(C=O)NR- -NR(C=S)NR- - $SO_2NR-$, $-NRSO_2-$, $CR_2OCR_2-$, $-CR_2SCR_2-$, $CR_2NRCR_2-$, eine $C_{4-8}$-Cycloheteroalkylengruppe, eine $C_{4-8}$-Cycloalkylengruppe, eine $C_{5-12}$-Arylengruppe, eine $C_{3-12}$-Heteroarylengruppe, eine Aminosäure, einen Zucker oder einen monodispersen Polyethylenglykol-(PEG)-Bildungsblock;
jedes R unabhängig ausgewählt ist aus H, $C_{1-4}$-Alkyl, $C_{2-4}$-Alkenyl, $C_{2-4}$-Alkinyl, $C_{1-4}$-Alkoxyalkyl oder $C_{1-4}$-Hydroxyalkyl; m für eine ganze Zahl mit dem Wert von 1 bis 20 steht;
n für eine ganze Zahl mit dem Wert 0 oder 1 steht;
IM für eine optische Reporterbildgebungseinheit steht, die für die Bildgebung des Säugerkörpers unter Verwendung von Licht mit violetter bis nahinfraroter Wellenlänge (400 bis 1.200 nm) geeignet ist;

wobei die Formulierung etwa 4 bis etwa 12 Gew.-% API, etwa 3 bis etwa 35 Gew.-% Puffermittel und etwa 56 bis etwa 91 Gew.-% Lyoprotektivum umfasst; und wobei die Formulierung, wenn sie rekonstituiert ist, einen pH-Wert zwischen etwa pH 6,8 und etwa pH 9 aufweist.

**18.** Verfahren nach Anspruch 17, wobei der erste Wasserentfernungsschritt bei einer Temperatur von etwa -30 °C oder weniger, wahlweise etwa -35 °C oder weniger, wahlweise etwa -38 °C oder weniger durchgeführt wird.

**19.** Verfahren nach Anspruch 17 oder Anspruch 18, wobei der zweite Wasserentfernungsschritt bei einer Temperatur von etwa 10 °C oder höher, wahlweise von etwa 15 °C oder höher, wahlweise von etwa 20 °C oder höher durchgeführt wird.

**20.** Verfahren nach einem der Ansprüche 17 bis 19, wobei mindestens einer des ersten und zweiten Wasserentfernungsschritts bei einem Druck von 50 pbar oder weniger durchgeführt wird, wahlweise wobei sowohl der erste als auch der zweite Wasserentfernungsschritt bei einem Druck von 50 µbar oder weniger durchgeführt wird.

**21.** Verfahren nach einem der Ansprüche 17 bis 20, wobei die lyophilisierte Formulierung die lyophilisierte Formulierung nach einem der Ansprüche 1 bis 16 ist.

**22.** Pharmazeutische Zusammensetzung, umfassend die Formulierung nach einem der Ansprüche 1 bis 16 und einen biokompatiblen Träger, in einer zur Verabreichung an Säuger geeigneter Form.

**23.** Kit für die Herstellung der pharmazeutischen Zusammensetzung nach Anspruch 22, wobei das Kit die Formulierung nach einem der Ansprüche 1 bis 16 in steriler, fester Form so umfasst, dass nach Rekonstitution mit einer bereitgestellten sterilen Menge des biokompatiblen Trägers nach Anspruch 22, Lösung erfolgt, damit sich die gewünschte pharmazeutische Zusammensetzung ergibt.

**24.** Formulierung nach einem der Ansprüche 1 bis 16 oder die pharmazeutische Zusammensetzung nach Anspruch 22 zur Verwendung als Bildgebungsmittel bei der Bildgebung des Säugerkörpers.

**25.** Formulierung nach einem der Ansprüche 1 bis 16 oder die pharmazeutische Zusammensetzung nach Anspruch 22 zur Verwendung als Arzneimittel.

**26.** Formulierung nach einem der Ansprüche 1 bis 16 oder die pharmazeutische Zusammensetzung nach Anspruch 22 zur Verwendung für Nachweis, Diagnose, Operation, Stadienbestimmung, Behandlung, Überwachung der Behandlung, Überwachung des Krankheitsverlaufs oder Überwachung der Therapie.

**27.** Formulierung nach einem der Ansprüche 1 bis 16 oder die pharmazeutische Zusammensetzung nach Anspruch 22 zur Verwendung für Nachweis, Diagnose, Operation, Stadienbestimmung, Behandlung, Überwachung der Behandlung, Überwachung des Krankheitsverlaufs oder Überwachung der Therapie einer oder mehrerer Krebsvorstufen und Krebs, wahlweise eines oder mehrerer von Darmkrebs, Speiseröhrenkrebs, Brustkrebs, Prostatakrebs, Kopfkrebs, Halskrebs, Eierstockkrebs, Enddarmkrebs, Bauchspeicheldrüsenkrebs, Schilddrüsenkrebs, Magenkrebs und Sarkom.

**28.** Formulierung nach einem der Ansprüche 1 bis 16 oder die pharmazeutische Zusammensetzung nach Anspruch 22 zur Verwendung für Nachweis, Diagnose, Operation, Stadienbestimmung, Behandlung, Überwachung der Behandlung, Überwachung des Krankheitsverlaufs oder Überwachung der Therapie von Stellen der cMet-Überexpression oder -Lokalisierung.

**29.** Formulierung nach einem der Ansprüche 1 bis 16 oder die pharmazeutische Zusammensetzung nach Anspruch 22 zur Verwendung, um ein Bild von Stellen der cMet-Überexpression oder -Lokalisierung, wahlweise *in vivo,* zu erhalten.

## Revendications

**1.** Formulation lyophilisée comprenant :

(i) un ingrédient pharmaceutique actif (API) ;
(ii) un agent tampon ; et
(iii) un lyoprotecteur ;

dans laquelle l'API est un agent d'imagerie comprenant au moins un peptide de liaison à cMet, approprié pour imager optiquement le corps d'un mammifère, l'agent d'imagerie comprenant un composé de Formule I :

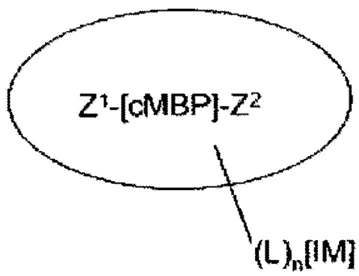

(Formule I)

dans laquelle :

$Z^1$ est attaché à l'extrémité N-terminale de cMBP, et est H ou $M^{IG}$ ;
$Z^2$ est attaché à l'extrémité C-terminale de cMBP, et est OH, $NH^2$, $OB^C$ ou $M^{IG}$ ;
dans laquelle $B^c$ est un cation biocompatible ;
cMBP est un peptide cyclique de liaison à cMet de 17 à 30 acides aminés, qui comprend la séquence d'acides aminés (SEQ-1) :

$Cys^a$-$Xaa^1$-$Cys^c$-$Xaa^2$-Gly-Pro-Pro-$Xaa^3$-Phe-Glu-$Cys^d$-Trp- $Cys^b$-Tyr-$Xaa^4$-$Xaa^5$-$Xaa^6$ ;

dans laquelle : $Xaa^1$ est Asn, His ou Tyr ;

Xaa2 est Gly, Ser, Thr ou Asn ;
$Xaa^3$ est Thr ou Arg ;
$Xaa^4$ est Ala, Asp, Glu, Gly ou Ser ;
$Xaa^5$ est Ser ou Thr ;
$Xaa^6$ est Asp ou Glu ;

et $Cys^{a-d}$ sont chacun des résidus cystéine de sorte que des résidus a et b ainsi que c et d sont cyclisés pour former deux liaisons disulfure séparées ;

$M^{IG}$ est un groupe inhibiteur de métabolisme, qui est un groupe biocompatible qui inhibe ou supprime *in vivo*le métabolisme du peptide ;
L est un groupe de liaison synthétique de formule -(A)m- dans laquelle chaque A est indépendamment $-CR_2-$, -CR=CR-, -C≡C-, $-CR_2CO_2-$, - $CO_2CR_2-$, - NRCO-, -CONR-, -NR(C=O)NR-, -NR(C=S)NR-, $-SO_2NR-$, $-NRSO_2-$, $CR_2OCR_2-$, - $CR_2SCR_2-$, $CR_2NRCR_2-$, un groupe cyclohétéroalkylène en $C_{4-8}$, un groupe cycloalkylène en $C_{4-8}$ , un groupe arylène en $C_{5-12}$, un groupe hétéroarylène en $C_{3-12}$, un acide aminé, un sucre ou un élément de base de polyéthylèneglycol (PEG) monodispersé ;
chaque R est indépendamment choisi parmi H, alkyle en $C_{1-4}$, alcényle en $C_{2-4}$, alcynyle en $C_{2-4}$, alcoxyalkyle en $C_{1-4}$ ou hydroxyalkyle en $C_{1-4}$ ; m est un nombre entier d'une valeur de 1 à 20 ;
n est un nombre entier d'une valeur de 0 ou 1 ;
IM est un fragment d'imagerie reporteur optique adapté à une imagerie du corps d'un mammifère à l'aide d'une lumière de longueur d'onde allant du violet au proche infrarouge (400 à 1200 nm) ;

dans laquelle la formulation comprend d'environ 4 à environ 12 % en poids d'API, d'environ 3 à environ 35 % en poids d'agent tampon et d'environ 56 à environ 91 % en poids de lyoprotecteur ; et dans laquelle la formulation, une fois reconstituée, présente un pH compris entre environ pH 6,8 et environ pH 9.

**2.** Formulation lyophilisée selon la revendication 1, dans laquelle en plus de SEQ-1, le cMBP comprend en outre un résidu Asp ou Glu, ou un analogue de celui-ci, dans les 4 résidus d'acide aminé de l'extrémité peptidique C- ou N- de cMBP, et $-(L)_n$IM est fonctionnalisé avec un groupe aminé, qui est conjugué à la chaîne latérale carboxyle dudit résidu Asp ou Glu, ou un analogue de celui-ci, pour donner une liaison amide ; et/ou dans laquelle en plus de SEQ-1, le cMBP comprend un résidu Lys, ou un analogue de celui-ci, dans les 4 résidus d'acide aminé de l'extrémité peptidique C- ou N- de cMBP, et $-(L)_n$IM est fonctionnalisé avec un groupe carboxyle, qui est conjugué à la chaîne latérale aminé epsilon dudit résidu Lys, ou un analogue de celui-ci, pour donner une liaison amide.

**3.** Formulation lyophilisée selon la revendication 1 ou la revendication 2, dans laquelle cMBP comprend la séquence d'acides aminés de SEQ-2 or SEQ-3 : Ser-$Cys^a$-$Xaa^1$-$Cys^c$-$Xaa^2$-Gly-Pro-Pro-$Xaa^3$-Phe-Glu-$Cys^d$-Trp-$Cys^b$-Tyr-$Xaa^4$-$Xaa^5$-$Xaa^6$ (SEQ-2) ;

Ala-Gly-Ser-$Cys^a$-$Xaa^1$-$Cys^c$-$Xaa^2$-Gly-Pro-Pro-$Xaa^3$-Phe-Glu-$Cys^d$-Trp-$Cys^b$-Tyr-$Xaa^4$-$Xaa^5$-$Xaa^6$-Gly-Thr (SEQ-3).

**4.** Formulation lyophilisée selon l'une quelconque des revendications précédentes, dans laquelle $Xaa^3$ est Arg.

**5.** Formulation lyophilisée selon l'une quelconque des revendications précédentes, dans laquelle en plus de SEQ-1, SEQ-2 ou SEQ-3, cMBP comprend en outre à l'extrémité N- ou C-terminale un peptide de liaison, qui est choisi parmi -Gly-Gly-Gly-Lys (SEQ-4), - Gly-Ser-Gly-Lys- (SEQ-5) et -Gly-Ser-Gly-Ser-Lys (SEQ-6).

**6.** Formulation lyophilisée selon l'une quelconque des revendications précédentes, dans laquelle cMBP comprend la séquence d'acides aminés (SEQ-7) :

Ala-Gly-Ser-Cys$^a$-Tyr-Cys$^c$-Ser-Gly-Pro-Pro-Arg-Phe-Glu-Cys$^d$-Trp-Cys$^b$-Tyr-Glu-Thr-Glu-Gly-Thr-Gly-Gly-Gly-Lys.

**7.** Formulation lyophilisée selon l'une quelconque des revendications précédentes, dans laquelle $Z^1$ et $Z^2$ sont tous deux indépendamment $M^{IG}$, facultativement dans laquelle $Z^1$ est un acétyle et $Z^2$ est un amide primaire.

**8.** Formulation lyophilisée selon l'une quelconque des revendications précédentes, dans laquelle IM est un colorant présentant un maximum d'absorbance dans la plage de 600 à 1 000 nm, facultativement dans laquelle IM est un colorant cyanine, facultativement dans laquelle le colorant cyanine présente la Formule III :

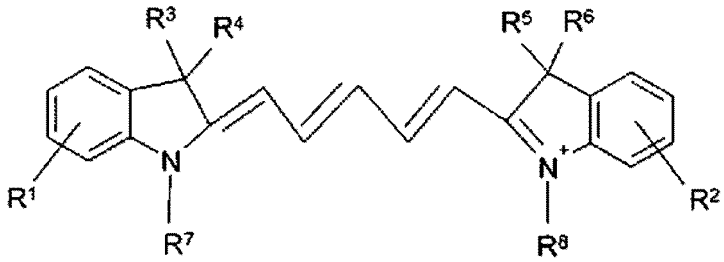

(Formule III)

dans laquelle :

$R^1$ et $R^2$ sont indépendamment H ou $SO_3M^1$, et au moins un de $R^1$ et $R^2$ est $SO_3M^1$, où $M^1$ est H or $B^c$ ;
$R^3$ et $R^4$ sont indépendamment un alkyle en $C_{1-4}$ ou un carboxyalkyle en $C_{1-6}$ ;
$R^5$, $R^6$, $R^7$ et $R^8$ sont indépendamment des groupes $R^a$ ;
dans laquelle $R^a$ est un alkyle en $C_{1-4}$, carboxyalkyle en $C_{1-6}$ ou $-(CH_2)_kSO_3M^1$, où k est un nombre entier d'une valeur de 3 ou 4 ;
à condition que le colorant cyanine ait un total de 1 à 4 substituants $SO_3M^1$ dans les groupes $R^1$, $R^2$ et $R^a$.

**9.** Formulation lyophilisée selon l'une quelconque des revendications précédentes, dans laquelle la formulation, une fois reconstituée, présente un pH compris entre environ pH 6,8 et environ pH 8,2, facultativement entre environ pH 6,8 et environ pH 8, facultativement entre environ pH 7 et environ pH 8.

**10.** Formulation lyophilisée selon l'une quelconque des revendications précédentes, dans laquelle l'agent tampon est présent selon une quantité pour fournir, une fois reconstitué, une solution présentant un pH compris entre environ pH 6,8 et environ pH 9, facultativement entre environ pH 6,8 et environ pH 8,2, facultativement entre environ pH 6,8 et environ pH 8, facultativement entre environ pH 7 et environ pH 8.

**11.** Formulation lyophilisée selon l'une quelconque des revendications précédentes, dans laquelle la formulation comprend d'environ 8 à environ 10 % en poids d'API, facultativement environ 9 % en poids d'API.

**12.** Formulation lyophilisée selon l'une quelconque des revendications précédentes, dans laquelle la formulation comprend d'environ 7 à environ 35 % en poids d'agent tampon, facultativement d'environ 12 à environ 35 % en poids d'agent tampon, facultativement d'environ 15 à environ 35 % en poids d'agent tampon, facultativement d'environ 18 à environ 35 % en poids d'agent tampon, facultativement d'environ 25 à environ 35 % en poids d'agent tampon, facultativement d'environ 32 à environ 35 % en poids d'agent tampon, facultativement environ 32 % en poids d'agent tampon.

**13.** Formulation lyophilisée selon l'une quelconque des revendications précédentes, dans laquelle la formulation comprend d'environ 56 à environ 82 % en poids de lyoprotecteur, facultativement d'environ 56 à environ 77 % en poids de lyoprotecteur, facultativement d'environ 56 à environ 75 % en poids de lyoprotecteur, facultativement d'environ 56 à environ 72 % en poids de lyoprotecteur, facultativement d'environ 56 à environ 66 % en poids de lyoprotecteur,

facultativement d’environ 56 à environ 58 % en poids de lyoprotecteur, facultativement environ 58 % en poids de lyoprotecteur.

**14.** Formulation lyophilisée selon l’une quelconque des revendications précédentes, dans laquelle l’agent tampon est au moins l’un d’un tampon au phosphate et d’un tampon d’alcanolamine, facultativement dans laquelle le tampon au phosphate comprend de l’hydrogénophosphate et du dihydrogénophosphate, facultativement dans laquelle le tampon au phosphate est hydraté, facultativement dans laquelle le tampon d’alcanolamine est du tris(hydroxyméthyl)aminométhane.

**15.** Formulation lyophilisée selon l’une quelconque des revendications précédentes, dans laquelle le lyoprotecteur est au moins l’un du saccharose et du mannitol, et des dérivés de ceux-ci, facultativement dans laquelle le lyoprotecteur est du mannitol, ou un dérivé de celui-ci.

**16.** Formulation lyophilisée selon l’une quelconque des revendications précédentes, dans laquelle la formulation comprend en outre un régulateur de tonicité, facultativement dans laquelle le lyoprotecteur agit également comme un régulateur de tonicité.

**17.** Procédé de préparation d’une formulation lyophilisée, le procédé comprenant les étapes consistant à :

a) fournir un ingrédient pharmaceutique actif (API), un agent tampon, et un lyoprotecteur dans un récipient de lyophilisation ;
b) effectuer une première étape d’élimination d’eau ; et
c) effectuer une seconde étape d’élimination d’eau ;

dans lequel le lyoprotecteur et l’agent tampon sont ajoutés avant la réalisation de la lyophilisation, et l’API est un agent d’imagerie comprenant au moins un peptide de liaison à cMet, approprié pour imager optiquement le corps d’un mammifère, l’agent d’imagerie comprenant un composé de Formule I :

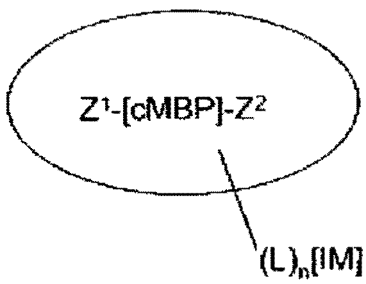

(Formule I)

dans laquelle :

$Z^1$ est attaché à l’extrémité N-terminale de cMBP, et est H ou $M^{IG}$ ;
$Z^2$ est attaché à l’extrémité C-terminale de cMBP, et est OH, $NH^2$, $OB^C$ ou $M^{IG}$ ;
dans laquelle $B^c$ est un cation biocompatible ;
cMBP est un peptide cyclique de liaison à cMet de 17 à 30 acides aminés, qui comprend la séquence d’acides aminés (SEQ-1): $Cys^a$-$Xaa^1$-$Cys^c$-$Xaa^2$-Gly-Pro-Pro-$Xaa^3$-Phe-Glu-$Cys^d$-Trp- $Cys^b$-Tyr-$Xaa^4$-$Xaa^5$-$Xaa^6$;

dans laquelle : $Xaa^1$ est Asn, His ou Tyr ;

$Xaa^2$ est Gly, Ser, Thr ou Asn ;
$Xaa^3$ est Thr ou Arg ;
$Xaa^4$ est Ala, Asp, Glu, Gly ou Ser ;
$Xaa^5$ est Ser ou Thr;
$Xaa^6$ est Asp ou Glu ;

et $Cys^{a-d}$ sont chacun des résidus cystéine de sorte que des résidus a et b ainsi que c et d sont cyclisés

pour former deux liaisons disulfure séparées ;

$M^{IG}$ est un groupe inhibiteur de métabolisme, qui est un groupe biocompatible qui inhibe ou supprime *in vivo*le métabolisme du peptide ;
L est un groupe de liaison synthétique de formule -(A)m- dans laquelle chaque A est indépendamment $-CR_2-$, -CR=CR-, -C≡C-, $-CR_2CO_2-$, $-CO_2CR_2-$, - NRCO-, -CONR-, -NR(C=O)NR-, -NR(C=S)NR-, $-SO_2NR-$, $-NRSO_2-$, $CR_2OCR_2-$, - $CR_2SCR_2-$, CR2NRCR2-, un groupe cyclohétéroalkylène en $C_{4-8}$, un groupe cycloalkylène en $C_{4-8}$, un groupe arylène en $C_{5-12}$, un groupe hétéroarylène en $C_{3-12}$, un acide aminé, un sucre ou un élément de base de polyéthylèneglycol (PEG) monodispersé ;
chaque R est indépendamment choisi parmi H, alkyle en $C_{1-4}$, alcényle en $C_{2-4}$, alcynyle en $C_{2-4}$, alcoxyalkyle en $C_{1-4}$ ou hydroxyalkyle en $C_{1-4}$ ;
m est un nombre entier d'une valeur de 1 à 20 ;
n est un nombre entier d'une valeur de 0 ou 1 ;
IM est un fragment d'imagerie reporteur optique adapté à une imagerie du corps d'un mammifère à l'aide d'une lumière de longueur d'onde allant du violet au proche infrarouge (400 à 1 200 nm) ;

dans laquelle la formulation comprend d'environ 4 à environ 12 % en poids d'API, d'environ 3 à environ 35 % en poids d'agent tampon et d'environ 56 à environ 91 % en poids de lyoprotecteur ; et dans laquelle la formulation, une fois reconstituée, présente un pH compris entre environ pH 6,8 et environ pH 9.

**18.** Procédé selon la revendication 17, dans lequel la première étape d'élimination d'eau est effectuée à une température d'environ -30 °C ou moins, facultativement d'environ -35 °C ou moins, facultativement d'environ -38 °C ou moins.

**19.** Procédé selon la revendication 17 ou la revendication 18, dans lequel la seconde étape d'élimination d'eau est effectuée à une température d'environ 10 °C ou plus, facultativement d'environ 15 °C ou plus, facultativement d'environ 20 °C ou plus.

**20.** Procédé selon l'une quelconque des revendications 17 à 19, dans lequel au moins l'une des première et seconde étapes d'élimination d'eau est effectuée à une pression de 50 μbar ou moins, facultativement dans lequel les première et seconde étapes d'élimination d'eau sont toutes deux effectuées à une pression de 50 μbar ou moins.

**21.** Procédé selon l'une quelconque des revendications 17 à 20, dans lequel la formulation lyophilisée est la formulation lyophilisée selon l'une quelconque des revendications 1 à 16.

**22.** Composition pharmaceutique comprenant la formulation selon l'une quelconque des revendications 1 à 16 et un support biocompatible, sous une forme appropriée pour une administration à un mammifère.

**23.** Kit pour la préparation de la composition pharmaceutique selon la revendication 22, le kit comprenant la formulation selon l'une quelconque des revendications 1 à 16 sous une forme solide stérile de telle sorte que lors de la reconstitution avec une alimentation stérile du support biocompatible selon la revendication 22, une dissolution se produit pour donner la composition pharmaceutique voulue.

**24.** Formulation selon l'une quelconque des revendications 1 à 16, ou composition pharmaceutique selon la revendication 22, à utiliser comme agent d'imagerie lors d'une imagerie du corps d'un mammifère.

**25.** Formulation selon l'une quelconque des revendications 1 à 16, ou composition pharmaceutique selon la revendication 22, à utiliser comme médicament.

**26.** Formulation selon l'une quelconque des revendications 1 à 16, ou composition pharmaceutique selon la revendication 22, à utiliser dans la détection, le diagnostic, la chirurgie, l'échelonnement, le traitement, la surveillance d'un traitement, la surveillance d'une progression de maladie ou la surveillance d'une thérapie.

**27.** Formulation selon l'une quelconque des revendications 1 à 16, ou composition pharmaceutique selon la revendication 22, à utiliser dans la détection, le diagnostic, la chirurgie, l'échelonnement, le traitement, la surveillance d'un traitement, la surveillance d'une progression de maladie ou la surveillance d'une thérapie d'un ou plusieurs parmi un état précancéreux et cancer, facultativement un ou plusieurs parmi un cancer colorectal, cancer de l'œsophage, cancer du sein, cancer de la prostate, cancer de la tête, cancer du cou, cancer des ovaires, cancer rectal, cancer du pancréas, cancer de la thyroïde, cancer gastrique et sarcome.

**28.** Formulation selon l'une quelconque des revendications 1 à 16, ou composition pharmaceutique selon la revendication 22, à utiliser dans la détection, le diagnostic, la chirurgie, l'échelonnement, le traitement, la surveillance d'un traitement, la surveillance d'une progression de maladie ou la surveillance d'une thérapie de sites de sur-expression ou de localisation de cMet.

**29.** Formulation selon l'une quelconque des revendications 1 à 16, ou composition pharmaceutique selon la revendication 22, à utiliser pour obtenir une image de sites de sur-expression ou de localisation de cMet, facultativement *in vivo.*

Figure 1

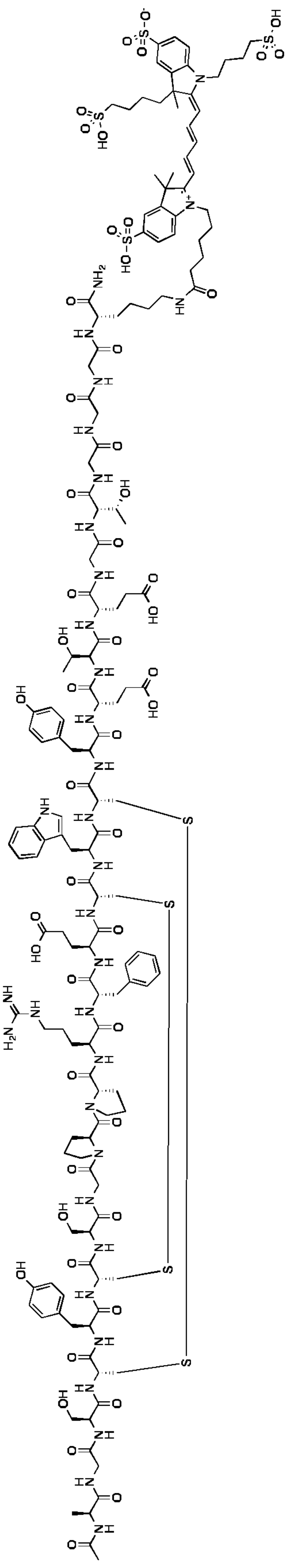

(EMI-137)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008139207 A
- WO 2004078778 A
- GB 1804835 A


### Non-patent literature cited in the description


- *Topics Curr.Chem.,* 2002, vol. 222, 1-29
- *Adv.Drug Deliv.Rev.,* 2005, vol. 57, 1087-1108
- **THEORODORA W. GREENE ; PETER G. M. WUTS.** Protective Groups in Organic Synthesis. John Wiley & Sons, 1991
- **P. LLOYD-WILLIAMS ; F. ALBERICIO ; E. GIRALD.** Chemical Approaches to the Synthesis of Peptides and Proteins. CRC Press, 1997
- **FLANAGAN et al.** *Bioconj.Chem.,* 1997, vol. 8, 751-756
- **LIN et al.** *ibid,* 2002, vol. 13, 605-610
- **ZAHEER.** *Mol.Imaging,* 2002, vol. 1 (4), 354-364
- Biomedical Photonics Handbook. CRC Press, 2003
- Handbook of Biomedical Fluorescence. Marcel Dekker, Inc, 2003
- An Introduction to Biomedical Optics. CRC Press, 2007
- **SEVICK-MURACA et al.** *Curr.Opin.Chem.Biol.,* 2002, vol. 6, 642-650
- **ZENG et al.** *Clin. Exp. Metastasis,* 2004, vol. 21, 409-417
- **FLATMARK et al.** *Eur. J. Cancer,* 2004, vol. 40, 1593-1598